# EUROPEAN PATENT SPECIFICATION

(11) **EP 2 200 982 B1**
(45) Date of publication and mention of the grant of the patent: **14.03.2012**
(21) Application number: 08803620.7
(22) Date of filing: 04.09.2008
(51) Int. Cl.: C07D 211/58, C07D 401/04, A61K 31/4468, A61P 25/18

(54) **PIPERIDINE DERIVATIVES AS NK3 RECEPTOR ANTAGONISTS**
PIPERIDINDERIVATE ALS NK3-REZEPTORANTAGONISTEN
DÉRIVÉS DE PIPÉRIDINE EN TANT QU'ANTAGONISTES DU RÉCEPTEUR NK3

(30) Priority: 14.09.2007 EP 07116441
(43) Date of publication of application: 30.06.2010
(73) Proprietor: F. Hoffmann-La Roche AG, 4070 Basel (CH)
(72) Inventor: KNUST, Henner, 79618 Rheinfelden (DE); NETTEKOVEN, Matthias, 79639 Grenzach-Wyhlen (DE); RATNI, Hasane, F-68440 Habsheim (FR); VIFIAN, Walter, CH-4600 Olten (CH); WU, Xihan, Shanghai 201204 (CN)
(74) Representative: Poppe, Regina
(86) International application number: PCT/EP2008/061649
(87) International publication number: WO 2009/033995

(56) References cited:
- WO-A-95/28931
- KAMALI F: "Osanetant Sanofi-Synthélabo" CURRENT OPINION IN INVESTIGATIONAL DRUGS, CURRENT DRUGS, LONDON, GB, vol. 2, no. 7, 1 January 2001 (2001-01-01), pages 950-956, XP008060165 ISSN: 0967-8298 cited in the application

## Description

The invention relates to a compound of general formula 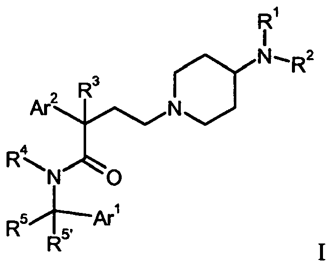 wherein
- Ar¹/Ar²: are independently from each other phenyl or pyridinyl, which are optionally substituted by one or two substituents, selected from the group consisting of halogen, lower alkyl, lower alkoxy, lower alkyl substituted by halogen, lower alkoxy substituted by halogen, lower alkyl substituted by alkoxy, lower alkyl substituted by cyano, lower di-alkyl amino, pyridinyl or cyano;
- R¹: is hydrogen, lower alkyl, -(CH₂)₂O-lower alkyl or cycloalkyl:
- R²: is -S(O)₂-lower alkyl or -C(O)-lower alkyl;
- or R¹ and R²: form together with the N-atom to which they are attached a pyrrolidin-2-one or a piperidin-2-one group;
- R³: is hydrogen, halogen or lower alkyl;
- R⁴: is hydrogen or lower alkyl;
- R⁵/R^{5'}: are independently from each other hydrogen, lower alkyl, lower alkyl substituted by halogen, lower alkyl substituted by hydroxy or cycloalkyl;
- or R⁵ and R^{5'}: form together with the carbon-atom to which they are attached a cycloalkyl group;
or to a pharmaceutically active salt thereof.

The invention includes all stereoisomeric forms, including individual diastereoisomers and enantiomers of the compound of formula (I) as well as racemic and non-racemic mixtures thereof.

The compounds of formula I differ from the relevant prior art as disclosed in WO 95/28931 essentially in the piperidine residue which are substituted in the 4-position by a phenyl group whereas this position for present compounds of formula I is un-substituted.

It has been found that the present compounds are high potential NK-3 receptor antagonists for the treatment of depression, pain, psychosis, Parkinson's disease, schizophrenia, anxiety and attention deficit hyperactivity disorder (ADHD).

The three main mammalian tachykinins, substance P (SP), neurokinin A (NKA) and neurokinin B (NKB) belong to the family of neuropeptides sharing the common COOH-terminal pentapeptide sequence of Phe-X-Gly-Leu-Met-NH₂. As neurotransmitters, these peptides exert their biological activity via three distinct neurokinin (NK) receptors termed as NK-1, NK-2 and NK-3. SP binds preferentially to the NK-1 receptor, NKA to the NK-2 and NKB to the NK-3 receptor.

The NK-3 receptor is characterized by a predominant expression in CNS and its involvement in the modulation of the central monoaminergic system has been shown. These properties make the NK-3 receptor a potential target for central nervous system disorders such as anxiety, depression, bipolar disorders, Parkinson's disease, schizophrenia and pain (Neurosci. Letters, 2000, 283, 185 -188*;* Exp. Opin. Ther. Patents 2000, 10, 939-960*;* Neuroscience, 1996, 74, 403-414*;* Neuropeptides, 1998, 32, 481-488).

Schizophrenia is one of the major neuropsychiatric disorders, characterized by severe and chronic mental impairment. This devastating disease affects about 1 % of the world's population. Symptoms begin in early adulthood and are followed by a period of interpersonal and social dysfunction. Schizophrenia manifests as auditory and visual hallucinations, paranoia, delusions (positive symptoms), blunted affect, depression, anhedonia, poverty of speech, memory and attention deficits as well as social withdrawal (negative symptoms).

For decades scientists and clinicians have made efforts with the aim of discovering an ideal agent for the pharmacological treatment of schizophrenia. However, the complexity of the disorders, due to a wide array of symptoms, has hampered those efforts. There are no specific focal characteristics for the diagnosis of schizophrenia and no single symptom is consistently present in all patients. Consequently, the diagnosis of schizophrenia as a single disorder or as a variety of different disorders has been discussed but not yet resolved. The major difficulty in the development of a new drug for schizophrenia is the lack of knowledge about the cause and nature of this disease. Some neurochemical hypotheses have been proposed on the basis of pharmacological studies to rationalize the development of a corresponding therapy: the dopamine, the serotonin and the glutamate hypotheses. But taking into account the complexity of schizophrenia, an appropriate multireceptor affinity profile might be required for efficacy against positive and negative signs and symptoms. Furthermore, an ideal drug against schizophrenia would preferably have a low dosage allowing once-per-day dosage, due to the low adherence of schizophrenic patients.

In recent years clinical studies with selective NK1 and NK2 receptor antagonists appeared in the literature showing results for the treatment of emesis, depression, anxiety, pain and migraine (NK1) and asthma (NK2 and NK1). The most exciting data were produced in the treatment of chemotherapy-induced emesis, nausea and depression with NK1 and in asthma with NK2- receptor antagonists. In contrast, no clinical data on NK3 receptor antagonists have appeared in the literature until 2000. Osanetant (SR 142,801) from Sanofi-Synthelabo was the first identified potent and selective non-peptide antagonist described for the NK3 tachykinin receptor for the potential treatment of schizophrenia, which was reported in the literature (Current Opinion in Investigational Drugs, 2001,2(7), 950-956 *and* Psychiatric Disorders Study 4, Schizophrenia, June 2003, Decision Recources, Inc., Waltham, Massachusetts). The proposed drug SR 142,801 has been shown in a phase II trial as active on positive symptoms of schizophrenia, such as altered behaviour, delusion, hallucinations, extreme emotions, excited motor activity and incoherent speech, but inactive in the treatment of negative symptoms, which are depression, anhedonia, social isolation or memory and attention deficits.

The neurokinin-3 receptor antagonists have been described as useful in pain or inflammation, as well as in schizophrenia, Exp. Opinion.Ther. Patents (2000), 10(6), 939-960 *and* Current Opinion in Investigational Drugs, 2001, 2(7), 950-956 956 *and* Psychiatric Disorders Study 4, Schizophrenia, June 2003, Decision Recources, Inc., Waltham, Massachusetts*)*.

Objects of the present invention are novel compounds of formula I, their manufacture, medicaments based on a compound in accordance with the invention and their production as well as the use of compounds of formula I in the control or prevention of illnesses such as depression, pain, bipolar disorders, psychosis, Parkinson's disease, schizophrenia, anxiety and attention deficit hyperactivity disorder (ADHD).

The preferred indications using the compounds of the present invention are depression, psychosis, Parkinson's disease, schizophrenia, anxiety and attention deficit hyperactivity disorder (ADHD).

The following definitions of the general terms used in the present description apply irrespective of whether the terms in question appear alone or in combination.

As used herein, the term "lower alkyl" denotes a straight- or branched-chain alkyl group containing from 1-8 carbon atoms, for example, methyl, ethyl, propyl, isopropyl, n-butyl, i-butyl, t-butyl and the like. Preferred lower alkyl groups are groups with 1-4 carbon atoms.

The term "lower alkyl substituted by halogen" denotes an alkyl group as defined above, wherein at least one hydrogen atom is replaced by halogen, for example -CF₃, -CHF₂, -CH₂F, -CH₂CF₃, -CH₂CH₂CF₃, -CH₂CF₂CF₃ and the like. Preferred lower alkyl substituted by halogen groups are groups having 1-4 carbon atoms.

The term "lower alkyl substituted by hydroxy" denotes an alkyl group as defined above, wherein at least one hydrogen atom is replaced by a hydroxy, for example -CH₂OH, -CH₂CH₂OH, and the like.

The term "lower alkoxy" denotes a group wherein the alkyl residue is as defined above and which is attached via an oxygen atom, for example, methoxy, ethoxy, propoxy, isopropoxy, n-butoxy, i-butoxy, 2-butoxy, t-butoxy and the like. Preferred alkoxy groups are groups with 1-4 carbon atoms.

The term "lower alkoxy substituted by halogen" denotes a group wherein the alkyl residue is as defined above "lower alkyl substituted by halogen" and which is attached via an oxygen atom. Preferred lower alkoxy substituted by halogen groups are groups having 1-4 carbon atoms.

The term "halogen" denotes chlorine, iodine, fluorine and bromine.

The term "pharmaceutically acceptable acid addition salt" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

The following groups of compounds of formula I are preferred:
Preferred are compounds of formula I, wherein Ar¹ and Ar² are both phenyl.
Especially preferred from this group are compounds, wherein
- R¹ is methyl and R² is S(O)₂CH₃, for example
2-(3,4-dichloro-phenyl)-N-(3-fluoro-4-trifluoromethyl-benzyl)-4-[4-(methanesulfonyl-methyl-amino)-piperidin-1-yl]--2,N-dimethyl-butyramide
N-(4-chloro-3-trifluoromethyl-benzyl)-2-(3,4-dichloro-phenyl)-4-[4-(methanesulfonyl-methyl-amino) -piperidin-1-yl] -2,N-dimethyl-butyramide
2-(3,4-dichloro-phenyl)-N-(4-fluoro-3-trifluoromethyl-benzyl)-4- [4-(methanesulfonyl-methyl-amino)-piperidin-1-yl] -2,N-dimethyl-butyramide
N-(3,4-dichloro-benzyl)-2-(3,4-dichloro-phenyl)-4-[4-(methanesulfonyl-methyl-amino)-piperidin-1-yl]-2,N-dimethyl-butyramide or
2-(3,4-dichloro-phenyl)-N-[1-(3,4-dichloro-phenyl)-2-hydroxy-ethyl]-4-[4-(methanesulfonyl-methyl-amino)-piperidin-1-yl]-2-methyl-butyramide; diastereoisomer 1.
- R¹ is cyclopropyl and R² is S(O)₂CH₃, for example
4-[4-(cyclopropyl-methanesulfonyl-amino)-piperidin-1-yl]-2-(3,4-dichloro-phenyl)-N-(3-fluoro-4-trifluoromethyl-benzyl)-2,N-dimethyl-butyramide
N-(4-chloro-3-trifluoromethyl-benzyl)-4- [4-(cyclopropyl-methanesulfonyl-amino)-piperidin-1-yl]-2-(3,4-dichloro-phenyl)-2,N-dimethyl-butyramide
4- [4-(cyclopropyl-methanesulfonyl-amino)-piperidin-1-yl] -2-(3,4-dichloro-phenyl)-N-(4-fluoro-3-trifluoromethyl-benzyl)-2,N-dimethyl-butyramide or
4-[4-(cyclopropyl-methanesulfonyl-amino)-piperidin-1-yl]-N-(3,4-dichloro-benzyl)-2-(3,4-dichloro-phenyl)-2,N-dimethyl-butyramide.
- R¹ is ethyl and R² is S(O)₂CH₃, for example
2-(3,4-dichloro-phenyl)-4-[4-(ethyl-methanesulfonyl-amino)-piperidin-1-yl]-N-(3-fluoro-4-trifluoromethyl-benzyl)-2,N-dimethyl-butyramide
N-(3,4-dichloro-benzyl)-2-(3,4-dichloro-phenyl)-4-[4-(ethyl-methanesulfonyl-amino)-piperidin-1-yl] -2,N-dimethyl-butyramide
N-(3,4-dichloro-benzyl)-2-(3,4-dichloro-phenyl)-4-[4-(ethyl-methanesulfonyl-amino)-piperidin-1-yl] -2-methyl-butyramide
N-(4-chloro-benzyl)-2-(3,4-dichloro-phenyl)-4-[4-(ethyl-methanesulfonyl-amino)-piperidin-1-yl]-2,N-dimethyl-butyramide
N-(4-chloro-3-trifluoromethyl-benzyl)-2-(3,4-dichloro-phenyl)-4-[4-(ethyl-methanesulfonyl-amino) -piperidin-1 -yl] -2,N-dimethyl-butyramide
2-(3,4-dichloro-phenyl)-4- [4-(ethyl-methanesulfonyl-amino)-piperidin-1-yl] -N-(4-fluoro-3-trifluoromethyl-benzyl)-2,N-dimethyl-butyramide
N-(3-chloro-benzyl)-2-(3,4-dichloro-phenyl)-4-[4-(ethyl-methanesulfonyl-amino)-piperidin-1-yl] -2,N-dimethyl-butyramide
2-(3,4-dichloro-phenyl)-4-[4-(ethyl-methanesulfonyl-amino)-piperidin-1-yl]-2,N-dimethyl-N-(4-trifluoromethyl-benzyl)-butyramide
2-(3,4-dichloro-phenyl)-4-[4-(ethyl-methanesulfonyl-amino)-piperidin-1-yl]-2,N-dimethyl-N-(4-methyl-benzyl)-butyramide
N-(2-chloro-benzyl)-2-(3,4-dichloro-phenyl)-4-[4-(ethyl-methanesulfonyl-amino)-piperidin-1-yl]-2,N-dimethyl-butyramide or
2-(3,4-dichloro-phenyl)-N-[1-(3,4-dichloro-phenyl)-2-hydroxy-ethyl] -4- [4-(ethyl-methanesulfonyl-amino)-piperidin-1-yl]-2-methyl-butyramide; diastereoisomer 1.
- R¹ and R² form together with the N-atom to which they are attached a pyrrolidin-2-one group, for example
2-(3,4-dichloro-phenyl)-N-(3-fluoro-4-trifluoromethyl-benzyl)-2,N-dimethyl-4-[4-(2-oxo-pyrrolidin-1-yl)-piperidin-1-yl] -butyramide
N-(3,4-dichloro-benzyl)-2-(3,4-dichloro-phenyl)-2,N-dimethyl-4-[4-(2-oxo-pyrrolidin-1-yl)-piperidin-1-yl] -butyramide
N-(3,4-dichloro-benzyl)-2-(3,4-dichloro-phenyl)-2-methyl-4-[4-(2-oxo-pyrrolidin-1-yl)-piperidin-1-yl] -butyramide
N-(4-chloro-benzyl)-2-(3,4-dichloro-phenyl)-2,N-dimethyl-4-[4-(2-oxo-pyrrolidin-1-yl)-piperidin-1-yl] -butyramide
N-(4-chloro-3-trifluoromethyl-benzyl)-2-(3,4-dichloro-phenyl)-2,N-dimethyl-4-[4-(2-oxo-pyrrolidin-1-yl) -piperidin-1-yl] -butyramide
2-(3,4-dichloro-phenyl)-N-(4-fluoro-3-trifluoromethyl-benzyl)-2,N-dimethyl-4-[4-(2-oxo-pyrrolidin-1-yl) -piperidin-1-yl]-butyramide
N-(3-chloro-benzyl)-2-(3,4-dichloro-phenyl)-2,N-dimethyl-4-[4-(2-oxo-pyrrolidin-1-yl)-piperidin-1-yl] -butyramide
2-(3,4-dichloro-phenyl)-2,N-dimethyl-4- [4-(2-oxo-pyrrolidin-1-yl)-piperidin-1-yl]-N-(4-trifluoromethyl-benzyl)-butyramide
2-(3,4-dichloro-phenyl)-2,N-dimethyl-N-(4-methyl-benzyl)-4-[4-(2-oxo-pyrrolidin-1-yl)-piperidin-1-yl] -butyramide
N-(2-chloro-benzyl)-2-(3,4-dichloro-phenyl)-2,N-dimethyl-4-[4-(2-oxo-pyrrolidin-1-yl)-piperidin-1-yl] -butyramide
2-(3,4-dichloro-phenyl)-N-[-1-(3,4-dichloro-phenyl)-2-hydroxy-ethyl]-2-methyl-4-[4-(2-oxo-pyrrolidin-1-yl)-piperidin-1-yl]-butyramide; diastereoisomer 1
2-(3,4-dichloro-phenyl)-2-methyl-4-[4-(2-oxo-pyrrolidin-1-yl)-piperidin-1-yl]-N-(2,2,2-trifluoro-1-phenyl-ethyl)-butyramide
2-(3,4-dichloro-phenyl)-2-methyl-4-[4-(2-oxo-pyrrolidin-l-yl)-piperidin-l-yl]-N-[2,2,2-trifluoro-1-(4-fluoro-phenyl)-ethyl] -butyramide
N-(4-chloro-benzyl)-2-(3,4-dichloro-phenyl)-N-methyl-4-[4-(2-oxo-pyrrolidin-1-yl)-piperidin-1-yl] -butyramide
(R or S)-2-(3,4-dichloro-phenyl)-N-(4-fluoro-3-trifluoromethyl-benzyl)-2,N-dimethyl-4-[4-(2-oxo-pyrrolidin-1-yl)-piperidin-1-yl]-butyramide
(R or S)-2-(3,4-dichloro-phenyl)-N-[(Sor R)-1-(3,4-dichloro-phenyl)-2-hydroxy-ethyl]-2-methyl-4- [4-(2-oxo-pyrrolidin-1-yl)-piperidin-1-yl]-butyramide
2-(3,4-dichloro-phenyl)-2-methyl-4-[4-(2-oxo-pyrrolidin-1-yl)-piperidin-1-yl]-N-((S)-1-phenyl-propyl)-butyramide
2-(3,4-dichloro-phenyl)-N-[(R)-1-(4-fluoro-phenyl)-2-hydroxy-ethyl]-2-(Ror S) methyl-4-[4-(2-oxo-pyrrolidin-1-yl)-piperidin-1-yl]-butyramide or
2-(3,4-dichloro-phenyl)-N-(4-fluoro-3-trifluoromethyl-benzyl)-2-methyl-4-[4-(2-oxopyrrolidin-1-yl)-piperidin-1-yl] -butyramide.
- R¹ is lower alkyl and R² is C(O)CH₃, for example
4- [4-(acetyl-methyl-amino)-piperidin-1-yl] -2-(3,4-dichloro-phenyl)-N-(3-fluoro-4-trifluoromethyl-benzyl)-2-methyl-butyramide
4-[4-(acetyl-methyl-amino)-piperidin-1-yl]-N-(3,4-dichloro-benzyl)-2-(3,4-dichloro-phenyl)-2,N-dimethyl-butyramide
4- [4-(acetyl-methyl-amino)-piperidin-1-yl] -N-(3,4-dichloro-benzyl)-2-(3,4-dichloro-phenyl)-2-methyl-butyramide
4-[4-(acetyl-methyl-amino)-piperidin-1-yl]-N-(4-chloro-benzyl)-2-(3,4-dichloro-phenyl)-2,N-dimethyl-butyramide
4- [4-(acetyl-methyl-amino)-piperidin-1-yl]-N-(4-chloro-3-trifluoromethyl-benzyl)-2-(3,4-dichloro-phenyl)-2,N-dimethyl-butyramide
4- [4-(acetyl-methyl-amino)-piperidin-1-yl]-2-(3,4-dichloro-phenyl)-N-(4-fluoro-3-trifluoromethyl-benzyl)-2,N-dimethyl-butyramide
4- [4-(acetyl-methyl-amino)-piperidin-1-yl]-N-(3-chloro-benzyl)-2-(3,4-dichloro-phenyl)-2,N-dimethyl-butyramide
4- [4-(acetyl-methyl-amino)-piperidin-1-yl]-2-(3,4-dichloro-phenyl)-2,N-dimethyl-N-(4-trifluoromethyl-benzyl)-butyramide
4- [4-(acetyl-methyl-amino)-piperidin-1-yl]-2-(3,4-dichloro-phenyl)-N- [1-(3,4-dichloro-phenyl)-2-hydroxy-ethyl]-2-methyl-butyramide; diastereoisomer 1
(R or S)-4-[4-(acetyl-methyl-amino)-piperidin-1-yl]-2-(3,4-dichloro-phenyl)-N-(4-fluoro-3-trifluoromethyl-benzyl)-2,N-dimethyl-butyramide
4-[4-(acetyl-methyl-amino)-piperidin-1-yl]-*N*-[1-(4-chloro-phenyl)-2-hydroxy-ethyl]-2-(3,4-dichloro-phenyl)-2-methyl-butyramide (diastereosisomer 1)
4-[4-(acetyl-propyl-amino)-piperidin-1-yl]-2-(3,4-dichloro-phenyl)-*N*-[(R)-1-(4-fluorophenyl)-2-hydroxy-ethyl]-2-methyl-butyramide (epimer 1)
4-[4-(acetyl-propyl-amino)-piperidin-1-yl]-2-(3,4-dichloro-phenyl)-*N*-[(R)-1-(4-fluorophenyl)-2-hydroxy-ethyl]-2-methyl-butyramide (epimer 2)
4- [4-(acetyl-propyl-amino)-piperidin-1-yl] -2-(3,4-dichloro-phenyl)-2-methyl-*N*-((S)-1-phenyl-propyl)-butyramide
4-[4-(acetyl-ethyl-amino)-piperidin-1-yl]-2-(3,4-dichloro-phenyl)-*N*-[(R)-1-(4-fluorophenyl)-2-hydroxy-ethyl]-2-methyl-butyramide (epimer 1) or
4-[4-(acetyl-ethyl-amino)-piperidin-1-yl]-2-(3,4-dichloro-phenyl)-2-methyl-*N*-((S)-1-phenyl-propyl)-butyramide.
- R¹ is -(CH₂)₂OCH₃ and R² is C(O)CH₃, for example
4-{4-[acetyl-(2-methoxy-ethyl)-amino]-piperidin-1-yl}-2-(3,4-dichloro-phenyl)-*N*-[1-(4-fluoro-phenyl)-2-hydroxy-ethyl]-2-methyl-butyramide (diastereoisomer 1)
4-{4-[acetyl-(2-methoxy-ethyl)-amino]-piperidin-1-yl}-2-(3,4-dichloro-phenyl)-*N*-[[1-(3,4-dichloro-phenyl)-2-hydroxy-ethyl]-2-methyl-butyramide (diastreoisomer 1)
4-{4-[acetyl-(2-methoxy-ethyl)-amino]-piperidin-1-yl}-2-(3,4-dichloro-phenyl)-*N*-[(R)-1-(4-fluoro-phenyl)-2-hydroxy-ethyl]-2-(R or S) methyl-butyramide or
4-{4-[acetyl-(2-methoxy-ethyl)-amino]-piperidin-1-yl}-2-(3,4-dichloro-phenyl)-2-methyl-*N*-((S)-1-phenyl-propyl)-butyramide.

The preparation of compounds of formula I of the present invention may be carried out in sequential or convergent synthetic routes. Syntheses of the compounds of the invention are shown in the following scheme 1. The skills required for carrying out the reaction and purification of the resulting products are known to those skilled in the art. The substituents and indices used in the following description of the processes have the significance given herein before unless indicated to the contrary.

In more detail, the compounds of formula I can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The present compounds of formula I and their pharmaceutically acceptable salts can be prepared by processes described below, which process comprises
a) cleaving off an O-protecting group under aqueous basic conditions from a compound of formula IV 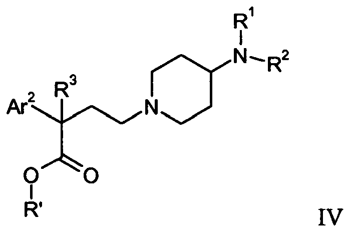 and reacting a compound of formula IV under coupling conditions with an amine of formula 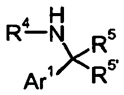 to a compound of formula 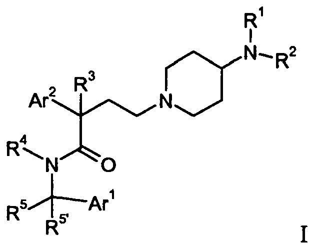 wherein the definitions have same meanings as described above, and
   if desired, converting the compounds obtained into pharmaceutically acceptable acid addition salts.

The process is described in scheme 1 in more detail.

### General experimental part:

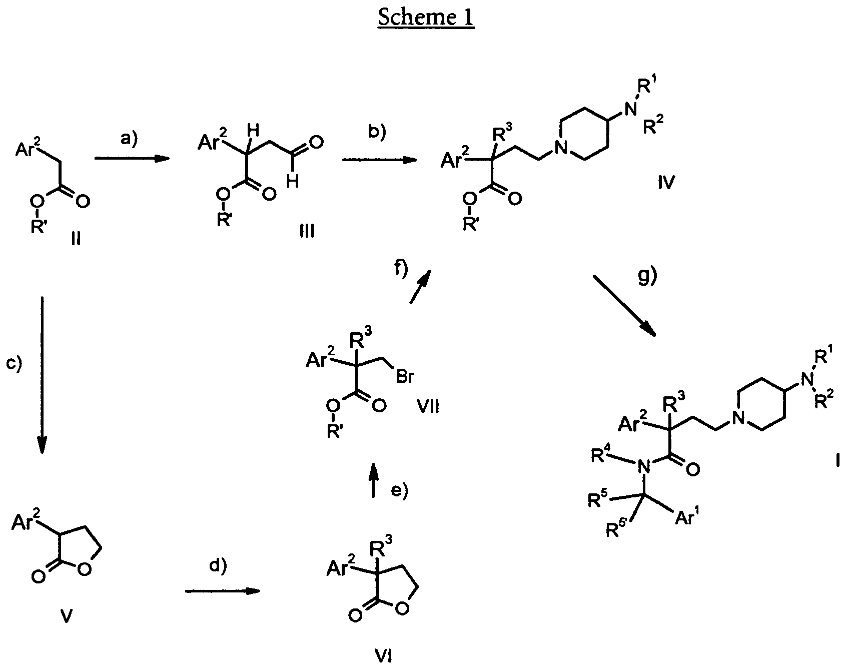

R¹, R², R³, R⁴, R⁵, R^{5'}, Ar¹ and Ar² are as described above and R' is an O-protecting group, such as benzyl.

### Step a)

Phenylacetic acid ester derivatives **II** are commercially available or can be accessed by methods described in literature. Reaction of ester derivatives **II** with protected bromo alkyl aldehydes (either commercially available or synthetically accessible by methods known in the art) under basic conditions lead to aldehyde derivatives **III** as described analogously in literature (for reaction conditions described in literature affecting such reactions see for example: Comprehensive Organic Transformations: A Guide to Functional Group Preparations, 2nd Edition, Richard C. Larock. John Wiley & Sons, New York, NY. 1999). However, it is convenient to react ester derivative **II** with the respective protected bromo alkyl aldehyde (commercially available or accessible by methods known) in the presence of a base and a solvent. There is no particular restriction on the nature of the solvent to be employed, provided that it has no adverse effect on the reaction or the reagents involved and that it can dissolve the reagents, at least to some extent. Examples for suitable solvents include dimethylformamide (DMF), tetrahydrofuran (THF) and the like. There is no particular restriction on the nature of the base used in this stage, and any base commonly used in this type of reaction may equally be employed here. Examples of such bases include NaH and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield the aldehyde protected intermediate which can be subjected to acidic cleavage of the protecting group in the presence of a solvent. There is no particular restriction on the nature of the solvent to be employed, provided that it has no adverse effect on the reaction or the reagents involved and that it can dissolve the reagents, at least to some extent. Examples for suitable solvents include tetrahydrofuran (THF) and the like. There is no particular restriction on the nature of the acid used in this stage, and any acid commonly used in this type of reaction may equally be employed here. Examples of such acid include HCl and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield aldehyde derivatives **III.**

### Step b)

Reductive aminations are widely described in literature (for reaction conditions described in literature affecting such reactions see for example: Comprehensive Organic Transformations: A Guide to Functional Group Preparations, 2nd Edition, Richard C. Larock. John Wiley & Sons, New York, NY. 1999). However, we find it convenient to transform aldehyde derivative **III** with piperidine derivatives (Journal of Medicinal Chemistry (2006), 49(16), 4801-4804) under reductive conditions in the presence of a solvent to afford ester derivatives **IV.** There is no particular restriction on the nature of the solvent to be employed, provided that it has no adverse effect on the reaction or the reagents involved and that it can dissolve the reagents, at least to some extent. Examples for suitable solvents include tetrahydrofuran (THF) and the like. There is no particular restriction on the nature of the reducing agent used in this stage, and any reducing agent commonly used in this type of reaction may equally be employed here. Examples of such reducing agents include sodium triacetoxyborohydride and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield ester derivatives **IV.**

### Step c)

Reaction of ester derivatives **II** with hydroxy-protected alkyl halides (either commercially available or synthetically accessible by methods known in the art) under basic conditions lead upon cleavage of the hydroxyl protecting group to lactones V as described in literature (for reaction conditions described in literature affecting such reactions see for example: Comprehensive Organic Transformations: A Guide to Functional Group Preparations, 2nd Edition, Richard C. Larock. John Wiley & Sons, New York, NY. 1999). However, it is convenient to react ester derivative **II** with 2-(2-bromoethoxy) tetrahydro-2-H-pyrane (commercially available in the presence of a base and a solvent. There is no particular restriction on the nature of the solvent to be employed, provided that it has no adverse effect on the reaction or the reagents involved and that it can dissolve the reagents, at least to some extent. Examples for suitable solvents include dimethylformamide (DMF), tetrahydrofuran (THF) and the like. There is no particular restriction on the nature of the base used in this stage, and any base commonly used in this type of reaction may equally be employed here. Examples of such bases include NaH and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield the hydroxy protected intermediate which can be subjected to acidic cleavage of the protecting group in the presence of a solvent. There is no particular restriction on the nature of the solvent to be employed, provided that it has no adverse effect on the reaction or the reagents involved and that it can dissolve the reagents, at least to some extent. Examples for suitable solvents include dimethylformamide (DMF), tetrahydrofuran (THF) and the like. There is no particular restriction on the nature of the acid used in this stage, and any acid commonly used in this type of reaction may equally be employed here. Examples of such acid include HCl and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield lactone derivatives V.

### Step d)

Lactone derivatives V can conveniently be transferred into the respective substituted lactone derivatives VI by reaction of lactone V with an electrophile in the presence of a base in the presence of a solvent. There is no particular restriction on the nature of the solvent to be employed, provided that it has no adverse effect on the reaction or the reagents involved and that it can dissolve the reagents, at least to some extent. Examples for suitable solvents include dimethylformamide (DMF), tetrahydrofuran (THF) and the like. There is no particular restriction on the nature of the base used in this stage, and any base commonly used in this type of reaction may equally be employed here. Examples of such bases include NaH and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield lactone derivatives **VI.**

### Step e)

Lactone derivative **VI** can conveniently be transferred into the respective ester derivative **VII** by a two step reaction sequence. Any commonly used synthetic sequence is applicable however, we find it convenient to open the lactone derivative **VII** with HBr in the presence of an acid. Any commonly used acid which in combination with HBr affects such a reaction can be used. Examples of such acids include acetic acid and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield the intermediately built acid derivative which is subjected to esterification conditions. Common procedures are described in literature, however, we find it convenient to transform the intermediately built acid into the respective ester derivative **VII** by reaction with SOCl₂ in methanol. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield ester derivative **VII.**

### Step f)

Transformation of ester derivatives **VII** with piperidine derivatives to access piperidine derivatives **IV** can be done by any commonly used procedure. However, we find it convenient to react ester derivative **VII** with piperidine derivatives in the presence of a solvent and a base. There is no particular restriction on the nature of the solvent to be employed, provided that it has no adverse effect on the reaction or the reagents involved and that it can dissolve the reagents, at least to some extent. Examples for suitable solvents include dimethylformamide (DMF), tetrahydrofuran (THF) and the like. There is no particular restriction on the nature of the base used in this stage, and any base commonly used in this type of reaction may equally be employed here. Examples of such bases include DIPEA, NEt₃ and the like. The reaction can take place over a wide range of temperatures, and the precise reaction temperature is not critical to the invention. It is convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield piperidine derivatives **IV**.

### Step g)

Transformation of piperidine derivatives **IV** into the final amide derivatives can be done according to procedures described in literature. However, we find it convenient to employ a two step reaction sequence in which the ester functionality in **IV** is cleaved under aqueous basic conditions and the liberated acid functionality converted with the respective amines under coupling conditions and to the piperidine derivatives I. There is no particular restriction on the nature of the aqueous base to be employed, provided that it has no adverse effect on the reaction or the reagents involved and that it can dissolve the reagents, at least to some extent. Examples for suitable aqueous bases include NaOH, LiOH and the like. Any commonly used co-solvent can be employed. Examples include THF and the like. The coupling of carboxylic acids with amines is widely described in literature and the procedures are known to those in the art (For reaction conditions described in literature affecting such reactions see for example: Comprehensive Organic Transformations: A Guide to Functional Group Preparations, 2nd Edition, Richard C. Larock. John Wiley & Sons, New York, NY. 1999). The intermediately built acid can conveniently be transformed to the respective amide through coupling with an amine (either commercially available or accessible by methods described in references or by methods known in the art; as appropriate) by employing the usage of coupling reagents. For example coupling reagents like N,N'-carbonyldiimidazole (CDI), N,N'-dicyclohexylcarbodiimide (DCC), 1-(3-dimethylaminopropyl)-3-ethylcarbodiimide hydrochloride (EDCI), 1-[bis(dimethylamino)methylene]-1H-1,2,3-triazolo[4,5-b]pyridinium-3-oxide hexafluorophosphate (HATU), 1-hydroxy-1,2,3-benzotriazole (HOBT), O-benzotriazol-1-yl-N,N,N',N'-tetramethyluronium tetrafluoroborate (TBTU) and the like can equally well be employed to affect such transformation. We find it convenient to carry out the reaction in a solvent like dimethylformamide (DMF) and in the presence of a base. There is no particular restriction on the nature of the solvent to be employed, provided that it has no adverse effect on the reaction or the reagents involved and that it can dissolve the reagents, at least to some extent. Examples for suitable solvents include: DMF, dichloromethane (DCM), dioxane, THF, and the like. There is no particular restriction on the nature of the base used in this stage, and any base commonly used in this type of reaction may equally be employed here. Examples of such bases include triethylamine and diisopropylethylamine, and the like. The reaction can take place over a wide range of temperatures, and the precise reaction temperature is not critical to the invention. We find it convenient to carry out the reaction with heating from ambient temperature to reflux. The time required for the reaction may also vary widely, depending on many factors, notably the reaction temperature and the nature of the reagents. However, a period of from 0.5 h to several days will usually suffice to yield piperidine derivatives **I**.

### Examples

### Intermediate 1

### 2-(3,4-dichloro-phenyl)-4-(4-methanesulfonylamino-piperidin-1-yl)-2-methyl-butyric acid

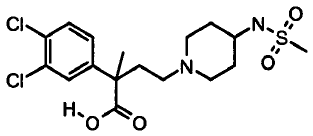

### a step 1:

### 3-(3,4-Dichloro-phenyl)-dihydro-furan-2-one (commercially available)

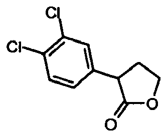

A mixture of 30.00 g (137 mmol) (3,4-dichloro-phenyl)-acetic acid methyl ester (commercially available), 6.47 g (151 mmol) NaH (55%) and 35.80 g (171 mmol) 2-(2-bromo-ethoxy)-tetrahydro-pyran in 100 mL DMF was stirred at room temperature for 17h. The mixture was evaporated to dryness and partitioned between water and ethyl acetate. The combined organic phases were washed with NaCl aq., dried with Na₂SO₄ and evaporated. The residue was treated with 400 mL 4N HCl in dioxane and stirred for 16 h at room temperature. The mixture was evaporated to dryness and subjected to column chromatography on silica eluting with a gradient formed from ethyl acetate and heptane. The combined product fractions were evaporated to yield 18.5 g (58%) of the title compound as yellow oil.

### b step 2:

### 3-(3,4-Dichloro-phenyl)-3-methyl-dihydro-furan-2-one

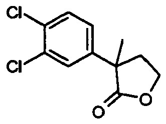

A mixture of 18.50 g (80 mmol) 3-(3,4-dichloro-phenyl)-dihydro-furan-2-one, 3.84 g (88 mmol) NaH (55% suspension) and 14.20 g (100 mmol) iodomethane in 300 mL THF was stirred for 64 h at room temperature. NH₄Cl aq. sat. was added and the mixture was extracted with ethyl acetate. The organic phases were washed with NaCl aq. sat. dried with Na₂SO₄, filtered and evaporated to dryness. The residue was purified by flash column chromatography on silica eluting with a gradient formed from ethyl acetate and heptane. The product containing fractions were evaporated to yield 16 g (82 %) of the title compound as yellow oil. MS(m/e): 246.0 (MH⁺).

### c step 3:

### 4-Bromo-2-(3,4-dichloro-phenyl)-2-methyl-butyric acid methyl ester

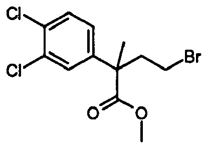

To a mixture of 3.30 g (13.5 mmol) 3-(3,4-dichloro-phenyl)-3-methyl-dihydro-furan-2-one in 15 mL acetic acid was added 48 mL HBr (33%) in acetic acid and after 63 h 20 mL HBr (33%) in acetic acid was added again and stirred for another 21 h at room temperature. The mixture was pored onto ice-water and extracted with ethyl ether. The combined organic phases were washed with NaCl aq. sat., dried with Na₂SO₄ filtered and evaporated to dryness, The residue was taken up in 150 mL toluene and 6.50 mL (89.0 mmol) thionylchloride were added. The mixture was heated to 75 °C for 4 h, cooled to 0 °C, treated with 20 mL methanol and allowed to stand for 16 h at room temperature. The mixture was evaporated to dryness and subjected to column chromatography on silica eluting with a gradient formed from ethyl acetate and heptane. The product containing fractions were evaporated to yield 4.32 g (94 %) of the title compound as light yellow oil. MS(m/e): 341.9 (MH⁺).

### d) step 4:

### 2-(3,4-dichloro-phenyl)-4-(4-methanesulfonylamino-piperidin-1-yl)-2-methyl-butyric acid methyl ester

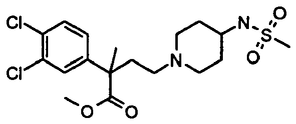

A mixture of 0.7 0g (2.05 mmol) 4-bromo-2-(3,4-dichloro-phenyl)-2-methyl-butyric acid methyl ester and 1.83 g (10.30 mmol) N-piperidin-4-yl-methanesulfonamide (commercially available) in 30 mL N,N-dimethylacetamide was stirred for 89 h at 60 °C. The mixture was evaporated to dryness and methanol, DCM and isolute was added and evaporated to dryness. The residue was subjected to column chromatography on silica eluting with a gradient formed from DCM, methanol and NH₃ aq.. The product containing fractions were evaporated to yield 0.76 g (84 %) of the title compound as light brown waxy solid. MS(m/e): 437.3 (MH⁺).

### e) step 5:

A mixture of 0.76 g (1.7 mmol) 2-(3,4-dichloro-phenyl)-4-(4-methanesulfonylamino-piperidin-1-yl)-2-methyl-butyric acid methyl ester and 0.23 g (5.4 mmol) LiOH·H₂O in 15 mL water and 15 mL THF was stirred at room temperature for 18 h. The mixture was evaporated and treated with 4N HCl aq. and evaporated to dryness. The residue was used without further purification in the subsequent step. MS(m/e): 423.1 (MH⁺).

### Intermediate 2

### 2-(3,4-Dichloro-phenyl)-4-(4-methanesulfonylamino-piperidin-1-yl)-butyric acid

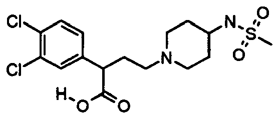

### a) step 1:

### 2-(3,4-Dichloro-phenyl)-4-oxo-butyric acid methyl ester

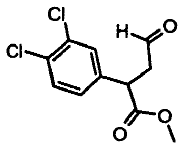

A mixture of 25.0 g (114 mmol) (3,4-dichloro-phenyl)-acetic acid methyl ester (commercially available), 5.7 g (131 mmol) NaH (55%) and 23.1 g (137 mmol) bromoacetaldehyde dimethylacetal in 80 mL DMF was stirred at room temperature for 3 h. The mixture was poured onto ice / water and extracted with ethyl acetate. The combined organic phases were washed with NaCl aq., dried with Na₂SO₄ and evaporated to dryness. The residue was dissolved in 250 mL THF and treated with 300 mL 1N HCl at room temperature for 20 h. Water was added and the mixture was extracted with ethyl acetate. The combined organic phases were washed with NaCl aq., dried with Na₂SO₄, evaporated to dryness and subjected to column chromatography on silica eluting with a gradient formed from heptane and ethyl acetate. The product containing fractions were evaporated to yield 9.7 g (32 %) of the title compound as light yellow oil. MS(m/e): 260.1 / 262.2 (MH⁺)

### b) step 2:

### 2-(3,4-Dichloro-phenyl)-4-(4-methanesulfonylamino-piperidin-1-yl)-butyric acid methyl ester

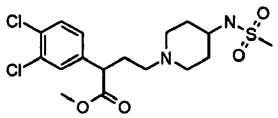

A mixture of 0.89 g (3.4 mmol) 2-(3,4-dichloro-phenyl)-4-oxo-butyric acid methyl ester, 0.67 g (3.7 mmol) N-Piperidin-4-yl-methanesulfonamide (commercially available), 1.08 g (5.0 mmol) sodium triacteoxyborohydride and 0.30 g (5.0 mmol) acetic acid in 40 mL THF was stirred at room temperature for 64 h. Water and Na₂CO₃ aq. was added and the mixture was extracted with ethyl acetate. The combined organic fractions were washed with NaCl sat. aq. dried with Na₂SO₄, filtered and evaporated to dryness. The residue was purified by flash column chromatography on silica eluting with a gradient formed from DCM, methanol and NH₃ aq.. The product containing fractions were evaporated to yield 1.21 g (84 %) of the title compound as off-white solid. MS(m/e): 423.1 (MH⁺).

### c) step 3:

A mixture of 1.16 g (2.7 mmol) 2-(3,4-dichloro-phenyl)-4-(4-methanesulfonylamino-piperidin-1-yl)-butyric acid methyl ester and 0.35 g (8.2 mmol) LiOH·H₂O in 20 mL water and 20 mL THF was stirred for 69 h at room temperature and evaporated. The residue was treated with 4N HCl aq. and evaporated to dryness and dried in vacuo. The residue was used without further purification in the subsequent step. MS(m/e): 409.4 (MH⁺).

### Intermediate 3

### 2-(3,4-Dichloro-phenyl)-4-[4-(methanesulfonyl-methyl-amino)-piperidin-1-yl]-2-methyl-butyric acid

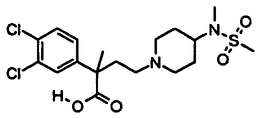

### a) step 1:

### 2-(3,4-Dichloro-phenyl)-4-[4-(methanesulfonyl-methyl-amino)-piperidin-1-yl]-2-methyl-butyric acid methyl ester

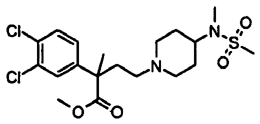

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-methyl-butyric acid methyl ester and N-methyl-N-piperidin-4-yl-methanesulfonamide (DE 2824064) as light yellow viscous oil. MS(m/e): 451.2 (MH⁺).

### b) step 2:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 2-(3,4-dichloro-phenyl)-4-[4-(methanesulfonyl-methyl-amino)-piperidin-1-yl]-2-methyl-butyric acid methyl ester by saponification with LiOH·H₂O as off-white foam. MS(m/e): 437.1 (MH⁺).

### Intermediate 4

### 2-(3,4-Dichloro-phenyl)-2-fluoro-4-[4-(methanesulfonyl-methyl-amino)-piperidin-1-yl]-butyric acid

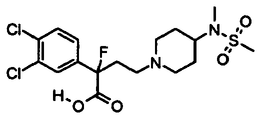

### a) step 1:

### 3-(3,4-Dichloro-phenyl)-3-fluoro-dihydro-furan-2-one

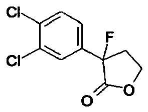

A mixture of 4.00 g (18.7 mmol) 3-(3,4-dichloro-phenyl)-dihydro-furan-2-one (commercially available), 0.83 g (19.0 mmol) NaH (55% suspension in oil) and 6.00 g (19.0 mmol) N-fluorobenzenesulfonimide (commercially available) in 100 mL THF stirred at room temperature for 1 h. The mixture was filtered and the filtrate evaporated to dryness. DCM and isolute was added and the mixture was evaporated to dryness. The residues was subjected to column chromatography on silica eluting with a gradient formed from heptane and toluene. The product containing fraction were evaporated to yield 3.8 g (88 %) of the title compound as colourless oil. MS(m/e): 248.1/ 250.1 /251.1 (MH⁺).

### b) step 2:

### 4-Bromo-2-(3,4-dichloro-phenyl)-2-fluoro-butyric acid methyl ester

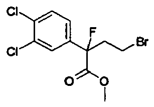

In analogy to the procedure described for the preparation of 4-bromo-2-(3,4-dichloro-phenyl)-2-methyl-butyric acid methyl ester (intermediate 1, step 3) the title compound was synthesized from 3-(3,4-dichloro-phenyl)-3-fluoro-dihydro-furan-2-one by acidic bromination with acetic acid and HBr and subsequent esterification with SOCl₂ and methanol. The title compound was obtained as colourless oil. MS(m/e): 342/346/348 (MH⁺).

### c) step 3:

### 2-(3,4-Dichloro-phenyl)-2-fluoro-4-[4-(methanesulfonyl-methyl-amino)-piperidin-1-yl]-butyric acid methyl ester

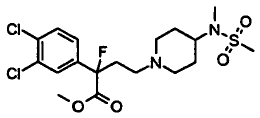

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-fluoro-butyric acid methyl ester and N-methyl-N-piperidin-4-yl-methanesulfonamide (DE 2824064) as light brown waxy solid. MS(m/e): 455.1 (MH⁺).

### d) step 4:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 2-(3,4-dichloro-phenyl)-2-fluoro-4-[4-(methanesulfonyl-methyl-amino)-piperidin-1-yl]-butyric acid methyl ester by saponification with LiOH·H₂O as off-white solid. MS(m/e): 441.1 (MH⁺).

### Intermediate 5

### N-Cyclopropyl-N-piperidin-4-yl-methanesulfonamide

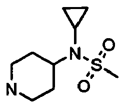

### a) step 1:

### N-(1-Benzyl-piperidin-4-yl)-N-cyclopropyl-methanesulfonamide

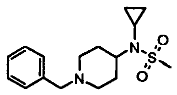

A mixture of 9.40 g (40.8 mmol) 1-benzyl-cyclopropylpiperidine-4-amine (commercially available), 5.14 g (44.8 mmol) methanesulfonyl chloride and 4.95 g (49.0 mmol) NEt₃ in 150 mL DCM was stirred for 17 h at room temperature. The mixture was evaporated to dryness and subjected to column chromatography on silica eluting with a gradient formed from DCM, methanol and NH₃ aq.. The product containing fractions were evaporated to yield 11.40 g (90 %) of the title compound as light yellow solid. MS(m/e): 309.3 (MH⁺).

### b) step 2:

A mixture of 11.3 g (36.6 mmol) N-(1-benzyl-piperidin-4-yl)-N-cyclopropyl methane sulfonamide and 5.1 g Pd/C (10 %) in 150 mL THF, 300 mL methanol and 31.7 mL formic acid was hydrogenated at room temperature. The mixture was filtered and evaporated to dryness. Water and Na₂CO₃ aq. was added and the mixture was extracted with THF/ethyl acetate. The combined organic phases were washed with NaCl aq. sat., dried with Na₂SO₄, filtered and evaporated to dryness. The residue was taken up in DCM and subjected to flash column chromatography on silica eluting with a gradient formed from DCM, methanol and NH₃ aq.. The product containing fractions were evaporated to yield 5 g (62 %) of the title compound as white solid. MS(m/e): 219.1 (MH⁺).

### Intermediate 6

### 4-[4-(Cyclopropyl-methanesulfonyl-amino)-piperidin-1-yl]-2-(3,4-dichloro-phenyl)-2-methyl-butyric acid

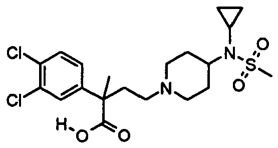

### a) step 1:

### 4-[4-(Cyclopropyl-methanesulfonyl-amino)-piperidin-1-yl]-2-(3,4-dichloro-phenyl)-2-methyl-butyric acid methyl ester

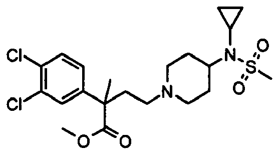

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-methyl-butyric acid methyl ester and N-cyclopropyl-N-piperidin-4-yl-methanesulfonamide as viscous colourless oil. MS(m/e): 476.9 (MH⁺).

### b) step 2:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 4-[4-cyclopropyl-methanesulfonyl-amino)-piperidin-1-yl]-2-(3,4-dichloro-phenyl)-2-methyl-butyric acid methyl ester by saponification with LiOH·H₂O as white foam. MS(m/e): 446.1 (MH⁺).

### Intermediate 7

### 2-(3,4-Dichloro-phenyl)-2-fluoro-4-(4-methanesulfonylamino-piperidin-1-yl)-butyric acid

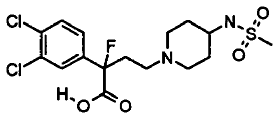

### a) step 1:

### 2-(3,4-Dichloro-phenyl)-2-fluoro-4-(4-methanesulfonylamino-piperidin-1-yl)-butyric acid methyl ester

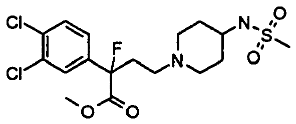

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-fluoro-butyric acid methyl ester and N-piperidin-4-yl-methanesulfonamide (commercially available) as light brown waxy solid. MS(m/e): 441.1 (MH⁺).

### b) step 2:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 2-(3,4-dichloro-phenyl)-2-fluoro-4-(4-methanesulfonylamino-piperidin-1-yl)-butyric acid methyl ester by saponification with LiOH·H₂O and conversion with HCl into the respective salt as off-white solid. MS(m/e): 427.1 (MH⁺).

### Intermediate 8

### N-Ethyl-N-piperidin-4-yl-methanesulfonamide

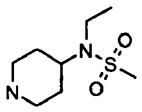

### a) step 1:

### 4-Ethylamino-piperidine-1-carboxylic acid benzyl ester

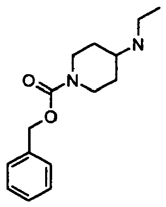

A mixture of 28.93 g (124 mmol) 1-(benzyloxycarbonyl)-4-piperidinone (commercially available, 8.30 g (186 mmol) ethylamine (2N in THF), 39.42 g (186 mmol) sodium triacetoxyborohydride and 11.10 g (186 mmol) acetic acid in 300 mL THF was stirred for 68 h at room temperature. Water and Na₂CO₃ aq. was added and extracted with ethyl acetate. The organic phases were washed with NaCl aq. sat., dried with Na₂SO₄, filtered and evaporated to dryness. The crude title compound was used in the consecutive step. MS(m/e): 263.0 (MH⁺).

### b) step 2:

### 4-(Ethyl-methanesulfonyl-amino)-piperidine-1-carboxylic acid benzyl ester

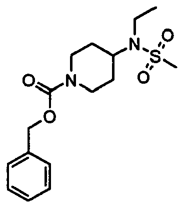

A mixture of 32.68 g (125 mmol) 4-ethylamino-piperidine-1-carboxylic acid benzyl ester, 16.00 g (140 mmol) methanesulfonyl chloride and 19.30 (149 mmol) DIPEA in 300 mL DCM was stirred for 80 min at room temperature. The mixture was concentrated, isolute was added and evaporated to dryness. The residue was subjected to flash column chromatography on silica eluting with a gradient formed from DCM, methanol and NH₃ aq.. The product containing fractions were evaporated and the residue was triturated with a mixture formed from heptane and diethyl ether. The precipitate was filtered and dried to yield 32.4 g (76 %) of the title compound as white solid. MS(m/e): 341.1 (MH⁺).

### c) step 3:

A solution of 32.4 g (95 mmol) 4-(ethyl-methanesulfonyl-amino)-piperidine-1-carboxylic acid benzyl ester in 400 mL THF and 300 mL methanol was hydrogenated at room temperature over 3.5 g Pd/ C (10%). The mixture was filtered and evaporated to dryness to yield the crude title compound which was used without further purification in the consecutive step. MS(m/e): 207.1 (MH⁺).

### Intermediate 9

### 2-(3,4-Dichloro-phenyl)-4-[4-(ethyl-methanesulfonyl-amino)-piperidin-1-yl]-2-methyl-butyric acid

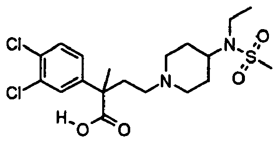

### a) step 1:

### 2-(3,4-Dichloro-phenyl)-4-[4-(ethyl-methanesulfonyl-amino)-piperidin-1-yl]-2-methyl-butyric acid methyl ester

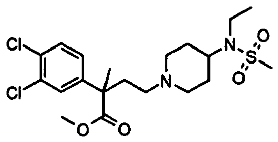

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-methyl-butyric acid methyl ester and N-ethyl-N-piperidin-4-yl-methanesulfonamide (intermediate 8) as light yellow viscous oil. MS(m/e): 467.0 (MH⁺).

### b) step 2:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 2-(3,4-dichloro-phenyl)-4-[4-(ethyl-methanesulfonyl-amino)-piperidin-1-yl]-2-methyl-butyric acid methyl ester by saponification with LiOH·H₂O and conversion with HCl into the respective salt as white foam. MS(m/e): 451.0 (MH⁺).

### Intermediate 10

### 2-(3,4-Dichloro-phenyl)-2-methyl-4-[4-(2-oxo-pyrrolidin-1-yl)-piperidin-1-yl]-butyric acid

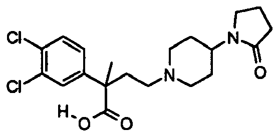

### a) step 1:

### 2-(3,4-Dichloro-phenyl)-2-methyl-4-[4-(2-oxo-pyrrolidin-1-yl)-piperidin-1-yl]-butyric acid methyl ester

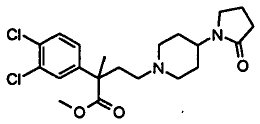

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-methyl-butyric acid methyl ester and 1-piperidin-4-yl-pyrrolidin-2-one (commercially available) as light yellow viscous oil. MS(m/e): 427.1 (MH⁺).

### b) step 2:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 2-(3,4-dichloro-phenyl)-2-methyl-4-[4-(2-oxopyrrolidin-1-yl)-piperidin-1-yl]-butyric acid methyl ester by saponification with LiOH·H₂O and conversion with HCl into the respective salt as white foam. MS(m/e): 415.2 (MH⁺).

### Intermediate 11

### 4-[4-(Acetyl-methyl-amino)-piperidin-1-yl]-2-(3,4-dichloro-phenyl)-2-methyl-butyric acid

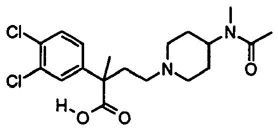

### a) step 1:

### 4-[4-(Acetyl-methyl-amino)-piperidin-1-yl]-2-(3,4-dichloro-phenyl)-2-methyl-butyric acid methyl ester

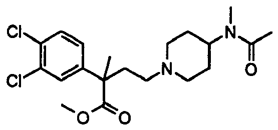

In analogy to the procedure described for the synthesis of 2-(3,4-dichloro-phenyl)-4-(4-methanesulfonylamino-piperidin-1-yl)-2-methyl-butyric acid methyl ester (intermediate 1, step 4) the title compound was prepared from 4-bromo-2-(3,4-dichloro-phenyl)-2-methyl-butyric acid methyl ester and N-methyl-N-piperidin-4-yl-acetamide (WO2005019194) as yellow viscous oil. MS(m/e): 415.3 (MH⁺).

### b) step 2:

In analogy to the procedure described for the synthesis of 2-(3,4-dichloro-phenyl)-4-(4-methanesulfonylamino-piperidin-1-yl)-2-methyl-butyric acid (intermediate 1, step 5) the title compound was prepared from 4-[4-(acetyl-methyl-amino)-piperidin-1-yl]-2-(3,4-dichloro-phenyl)-2-methyl-butyric acid methyl ester by saponification with LiOH·H₂O and conversion with HCl into the respective salt as white foam. MS(m/e): 401.1 (MH⁺).

### Intermediate 12

### (3-Fluoro-4-trifluoromethyl-benzyl)-methyl-amine, hydrochloride

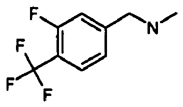

A mixture of 7.65 g (39.8 mmol) 3-fluoro-4-trifluoro-benzaldehyde, 24.39 mL (49.0 mmol) methylamine (2M in THF), 3.60 mL acetic acid and 12.46 g (59.0 mmol) sodium triacetoxyborohydride in 200 mL THF was stirred for 22 h at room temperature. Water and Na₂CO₃ aq. was added and the mixture was extracted with ethyl acetate. The organic phases were washed with NaCl aq., dried with Na₂SO₄, filtered and evaporated to dryness. The residue was purified by flash column chromatography on silica eluting with a gradient formed from DCM, methanol and NH₃ aq.. The combined product containing fractions were evaporated and treated with HCl in diethyl ether. The precipitate was filtered, washed with diethyl ether and dried to yield 4.00 g (42 %) of the title compound as white solid. MS(m/e): 208.1 (MH⁺).

### Intermediate 13

### (4-Fluoro-3-trifluoromethyl-benzyl)-methyl-amine, hyrdochloride

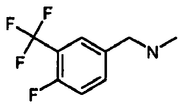

In analogy to the procedure described for the synthesis of (3-fluoro-4-trifluoromethylbenzyl)-methyl-amine, hydrochloride (intermediate 12) the title compound was prepared from 4-fluoro-3-trifluoro-benzaldehyde and methylamine under reductive conditions as white solid. MS(m/e): 208.3 (MH⁺).

### Intermediate 14

### 4-[4-(Acetyl-cyclopropyl-amino)-piperidin-1-yl]-2-(3,4-dichloro-phenyl)-2-methyl-butyric acid

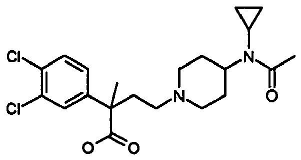

In analogy to the procedure described for the synthesis of 2-(3,4-dichloro-phenyl)-4-(4-methanesulfonylamino-piperidin-1-yl)-butyric acid (intermediate 2) the title compound was prepared from 2-(3,4-dichloro-phenyl)-4-oxo-butyric acid methyl ester, N-Cyclopropyl-N-piperidin-4-yl-acetamide (intermediate 22) and subsequent saponification as exemplified in the synthesis of intermediate 2, c) step 3. MS(m/e): 427.2 (MH⁺).

### Intermediate 15

### 4-{4-[Acetyl-(2-methoxy-ethyl)-amino]-piperidin-1-yl}-2-(3,4-dichloro-phenyl)-2-methyl-butyric acid

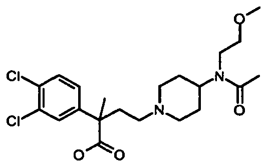

In analogy to the procedure described for the synthesis of 2-(3,4-dichloro-phenyl)-4-(4-methanesulfonylamino-piperidin-1-yl)-butyric acid (intermediate 2) the title compound was prepared from 2-(3,4-dichloro-phenyl)-4-oxo-butyric acid methyl ester, N-(2-Methoxy-ethyl)-N-piperidin-4-yl-acetamide, hydrochloride (intermediate 23) and subsequent saponification as exemplified in the synthesis of intermediate 2, c) step 3. MS(m/e): 445.1 (MH⁺).

### Intermediate 16

### 4-(4-Acetylamino-piperidin-1-yl)-2-(3,4-dichloro-phenyl)-2-methylbutyric acid

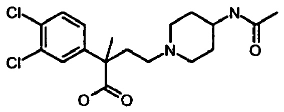

In analogy to the procedure described for the synthesis of 2-(3,4-Dichloro-phenyl)-4-(4-methanesulfonylamino-piperidin-1-yl)-butyric acid (intermediate 2) the title compound was prepared from 2-(3,4-Dichloro-phenyl)-4-oxo-butyric acid methyl ester, 4-Acetylamino-piperidine (commercially available) and subsequent saponification as exemplified in the synthesis of intermediate 2, c) step 3. MS(m/e): 387.2 (MH⁺).

### Intermediate 17

### 4-[4-(Acetyl-propyl-amino)-piperidin-1-yl]-2-(3,4-dichloro-phenyl)-2-methyl-butyric acid

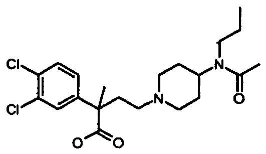

In analogy to the procedure described for the synthesis of 2-(3,4-Dichloro-phenyl)-4-(4-methanesulfonylamino-piperidin-1-yl)-butyric acid (intermediate 2) the title compound was prepared from 2-(3,4-Dichloro-phenyl)-4-oxo-butyric acid methyl ester, N-Piperidin-4-yl-N-propyl-acetamide (WO 9410146) and subsequent saponification as exemplified in the synthesis of intermediate 2, c) step 3. MS(m/e): 429.2 (MH⁺).

### Intermediate 18

### 4-[4-(Acetyl-ethyl-amino)-piperidin-1-yl]-2-(3,4-dichloro-phenyl)-2-methyl-butyric acid

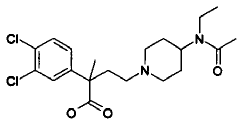

In analogy to the procedure described for the synthesis of 2-(3,4-Dichloro-phenyl)-4-(4-methanesulfonylamino-piperidin-1-yl)-butyric acid (intermediate 2) the title compound was prepared from 2-(3,4-Dichloro-phenyl)-4-oxo-butyric acid methyl ester, N-Ethyl-N-piperidin-4-yl-acetamide (EP 457686) and subsequent saponification as exemplified in the synthesis of intermediate 2, c) step 3. MS(m/e): 415.2 (MH⁺).

### Intermediate 19

### 1-(4-Fluoro-3-trifluoromethyl-phenyl)-ethylamine; hydrochloride

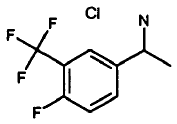

A mixture of 10g (49 mmol) 4-Fluoro-3-(trifluoromethyl) acetopheneone (commercially available), 27.5 g (97 mmol) titanium tetraisopropoxide and 34 mL (243 mmol) 7 N ammonia in MeOH at 0 °C was stirred over the weekend at 20 °C. Subsequently, 5.5 g (146 mmol) sodium borohydride was added and the mixture allowed to come from 0 °C to room temperature over night. Water and ammonia was added and the mixture was filtered through decalit and washed with ethyl acetate. The aqueous phase was separated washed with ethyl acetate and the combined organic phases washed with NaCL sat. aq., dried with Na₂SO₄ and evaporated to dryness. The residue was purified by column chromatography eluting with a gradient formed from DCM, methanol and ammonia. The product containing fractions were evaporated to dryness and transformed with HCl in diethyl ether to the respective hydrochloride salt. Yield: 4.4 g (37 %). MS(m/e): 207.9 (MH⁺).

### Intermediate 20

### 1-(4-Fluoro-3-trifluoromethyl-phenyl)-ethyl-methyl amine; hydrochloride

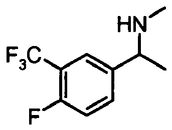

In analogy to the procedure described for the synthesis of Intermediate 20, 1-(4-Fluoro-3-trifluoromethyl-phenyl)-ethylamine; hydrochloride the title compound was prepared from 4-fluoro-3-(trifluoromethyl) acetopheneone (commercially available) and methylamine. MS(m/e): 191.2/222.1 (MH⁺).

### Intermediate 21

### N-Cyclopropyl-N-piperidin-4-yl-acetamide

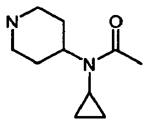

A mixture of 6.25 g (27 mmol) 1-(benzyloxycarbonyl)-4-piperidinone (commercially available, 1.83 g (32 mmol) cyclopropyl amine, 2.3 mL acetic acid and 8.51 g (40 mmol) sodium triacetoxyborohydride was stirred from 0 °C to room temperature over night. Water and Na₂CO₃ aq. was added and the mixture extracted with ethyl acetate. The organic phase was washed with NaCl sat. aq. , dried with Na₂SO₄ and evaporated to dryness. The residue was taken up in 100 mL DCM and 4.48 mL (32 mmol) NEt₃ and 2.09 mL acetyl chloride was added and allowed to stirr at 0 °C for 2 h. The mixture was evaporated and isolute was added and the residue was purified by column chromatography on silica eluting with a gradient formed from DCM, methanol and ammonia. The product containing fractions were evaporated and the residue taken up in methanol and hydrogenated over Pd/C with H₂. Filtration and evaporation yielded 4.5 g (92 %) of the title compound as light yellow solid. MS(m/e): 183.2 (MH⁺).

### Intermediate 22

### N-(2-Methoxy-ethyl)-N-piperidin-4-yl-acetamide, hydrochloride

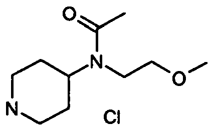

In analogy to the procedure described for the synthesis of N-cyclopropyl-N-piperidin-4-yl-acetamide (intermediate 22) the title compound was prepared from 1-(benzyloxycarbonyl)-4-piperidinone (commercially available and 2-methoxyethyloamine.

The title compound was obtained as hydrochloride salt by treatment of the free base with HCl. MS(m/e): 201.1 (MH⁺).

### Example 1

### 2-(3,4-Dichloro-phenyl)-N-(3-fluoro-4-methoxy-benzyl)-4-(4-methanesulfonylamino-piperidin-1-yl)-2,N-dimethyl-butyramide

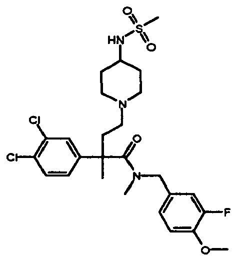

A mixture of 30 mg crude 2-(3,4-dichloro-phenyl)-4-(4-methanesulfonylamino-piperidin-1-yl)-2-methyl-butyric acid, 14 mg (0.068 mmol) (3-fluoro-4-methoxybenzyl)-methyl-amine, hydrochloride, 0.33 mL TBTU/DMF (0.2N) and 50 uL DIPEA in 1.2 mL DMF was stirred at room temperature for 16 h. The mixture was subjected to purification by preparative HPLC on reversed phase eluting with a gradient formed from acetonitrile, water and NEt₃. The product containing fractions were evaporated to yield 6.5 mg of the title compound. MS(m/e): 574.3 (MH⁺).

In analogy to the procedure described for the synthesis of 2-(3,4-dichloro-phenyl)-N-(3-fluoro-4-methoxy-benzyl)-4-(4-methanesulfonylamino-piperidin-1-yl)-2,N-dimethyl-butyramide (example 1) further piperidine derivatives have been prepared from the starting materials listed in table 1. Optionally diastereoisomeric mixture have been separated by column chromatography. Table 1 comprises example 2- example 110.

**Table 1:**

| No | structure | MW | Systematic Name | starting materials | MW found (MH+) | hNK3 Ki (uM) |
|---|---|---|---|---|---|---|
| 1 | 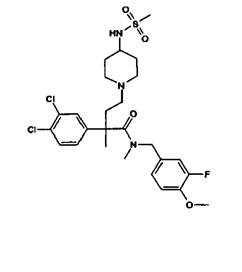 | 574.5 | 2-(3,4-Dichloro-phenyl)-N-(3-fluoro-4-methoxy-benzyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (3-Fluoro-4-methoxy-benzyl)-methyl-amine (commercially available) | 574.3 | 0.1872 |
| 2 | 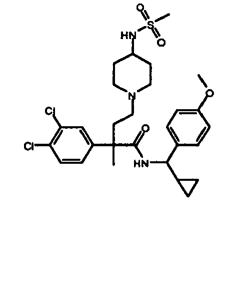 | 582.6 | N-[Cyclopropyl-(4-methoxy-phenyl)-methyl]-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and C-Cyclopropyl-C-(4-methoxy-phenyl)-methylamine (commercially available) | 584.3 | 0.7035 |
| 3 | 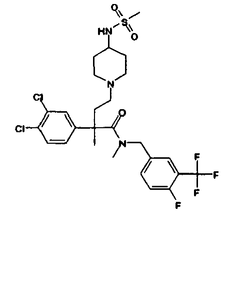 | 612.5 | 2-(3,4-Dichloro-phenyl)-N-(4-fluoro-3-trifluoromethyl-benzyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (4-Fluoro-3-trifluoromethyl-benzyl)-methyl-amine, hyrdochloride (intermediate 13) | 612.1 | 0.0232 |
| 4 | 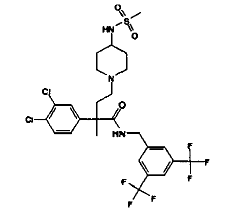 | 648.5 | N-(3,5-Bis-trifluoromethyl-benzyl)-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 3,5-Bis-trifluoromethyl-benzylamine (commercially available) | 648.3 | 0.1396 |
| 5 | 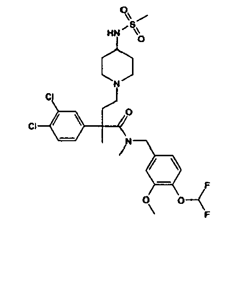 | 622.6 | 2-(3,4-Dichloro-phenyl)-N-(4-difluoromethoxy-3-methoxy-benzyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (4-Difluoromethoxy-3-methoxy-benzyl)-methyl-amine (commercially available) | 622.2 | 0.2716 |
| 6 | 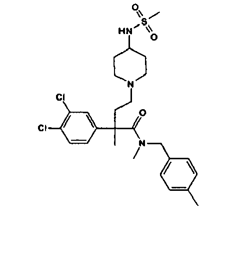 | 540.6 | 2-(3,4-Dichloro-phenyl)-4-(4-methanesulfonylami no-piperidin-1-yl)-2,N-dimethyl-N-(4-methyl-benzyl)-butyramide | 2-(3,4-Dichloro-phenyl)-4-(4-methanesulfonylam ino-piperidin-1-yl)-2-methyl-butyric acid (intermediate 1) and Methyl-(4-methyl-benzyl)-amine (commercially available) | 540.4 | 0.0774 |
| 7 | 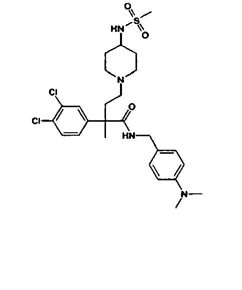 | 555.6 | 2-(3,4-Dichloro-phenyl)-N-(4-dimethylamino-benzyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and (4-Aminomethyl-phenyl)-dimethyl-amine (commercially available) | 555.3 | 0.6205 |
| 8 | 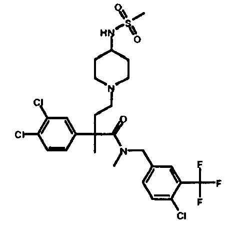 | 629.0 | N-(4-Chloro-3-trifluoromethyl-benzyl)-2-(3,4-dichloro-phenyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (4-Chloro-3-trifluoromethyl-benzyl)-methyl-amine (commercially available) | 630.3 | 0.0226 |
| 9 | 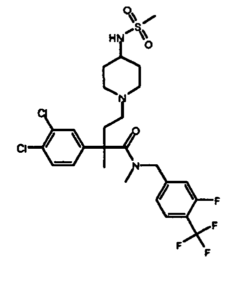 | 612.5 | 2-(3,4-Dichloro-phenyl)-N-(3-fluoro-4-trifluoromethyl-benzyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (3-Fluoro-4-trifluoromethyl-benzyl)-methyl-amine, hydrochloride (intermediate 12) | 612.3 | 0.03 |
| 10 | 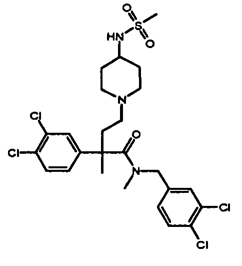 | 595.4 | N-(3,4-Dichloro-benzyl)-2-(3,4-dichloro-phenyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (3,4-Dichloro-benzyl)-methyl-amine (commercially available) | 596.1 | 0.024 |
| 11 | 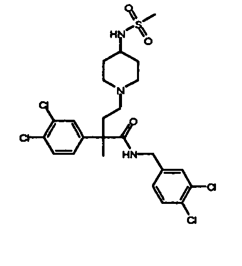 | 581.4 | N-(3,4-Dichloro-benzyl)-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 3,4-Dichloro-benzylamine (commercially available) | 582 | 0.0442 |
| 12 | 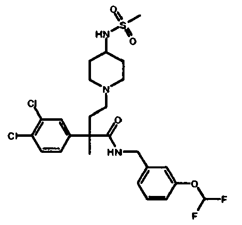 | 578.5 | 2-(3,4-Dichloro-phenyl)-N-(3-difluoromethoxy-benzyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 3-Difluoromethoxy-benzylamine (commercially available) | 578.1 | 0.3094 |
| 13 | 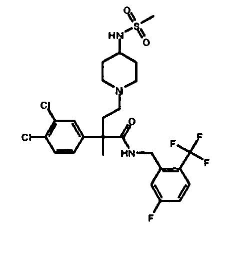 | 598.5 | 2-(3,4-Dichloro-phenyl)-N-(5-fluoro-2-trifluoromethyl-benzyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 5-Fluoro-2-trifluoromethyl-benzylamine (commercially available) | 598.1 | 0.5804 |
| 14 | 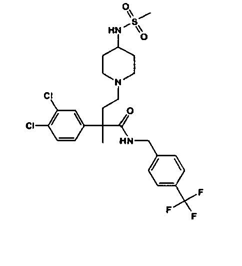 | 580.5 | 2-(3,4-Dichloro-phenyl)-4-(4-methanesulfonylami no-piperidin-1-yl)-2-methyl-N-(4-trifluoromethyl-benzyl)-butyramide | 2-(3,4-Dichloro-phenyl)-4-(4-methanesulfonylam ino-piperidin-1-yl)-2-methyl-butyric acid (intermediate 1) and 4-Trifluoromethyl-benzylamine (commercially available) | 580 | 0.058 |
| 15 | 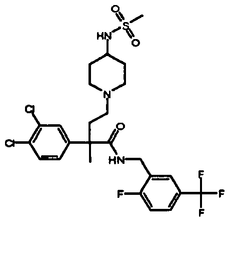 | 598.5 | 2-(3,4-Dichloro-phenyl)-N-(2-fluoro-5-trifluoromethyl-benzyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 2-Fluoro-5-trifluoromethyl-benzylamine (commercially available) | 598.3 | 0.1661 |
| 16 | 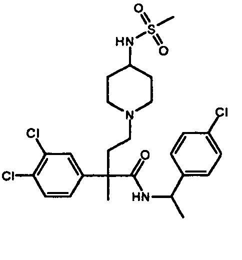 | 561.0 | N-[1-(4-Chloro-phenyl)-ethyl]-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 1-(4-Chloro-phenyl)-ethylamine (commercially available) | 562.5 | 0.1735 |
| 17 | 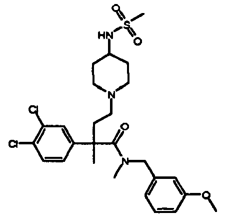 | 556.6 | 2-(3,4-Dichloro-phenyl)-4-(4methanesulfonylami no-piperidin-1-yl)-N-(3-methoxy-benzyl)-2,N-dimethyl-butyramide | 2-(3,4-Dichloro-phenyl)-4-(4-methanesulfonylam ino-piperidin-1-yl)-2-methyl-butyric acid (intermediate 1) and (3-Methoxy-benzyl)-methyl-amine (commercially available) | 556.1 | 0.2127 |
| 18 | 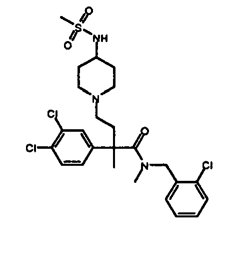 | 561.0 | N-(2-Chloro-benzyl)-2-(3,4-dichloro-phenyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (2-Chloro-benzyl)-methyl-amine (commercially available) | 560.2 | 0.0333 |
| 19 | 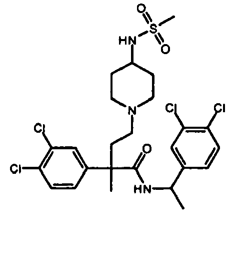 | 595.4 | 2-(3,4-Dichloro-phenyl)-N-[1-(3,4-dichloro-phenyl)-ethyl]-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 1-(3,4-Dichloro-phenyl)-ethylamine (commercially available) | 596.3 | 0.0599 |
| 20 | 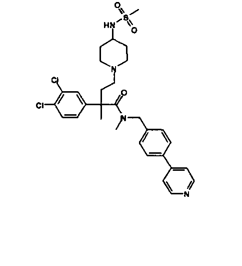 | 603.6 | 2-(3,4-Dichloro-phenyl)-4-(4-methanesulfonylami no-piperidin-1-yl)-2,N-dimethyl-N-(4-pyridin-4-yl-benzyl)-butyramide | 2-(3,4-Dichloro-phenyl)-4-(4-methanesulfonylam ino-piperidin-1-yl)-2-methyl-butyric acid (intermediate 1) and Methyl-(4-pyridin-4-yl-benzyl)-amine (commercially available) | 603.3 | 0.2032 |
| 21 | 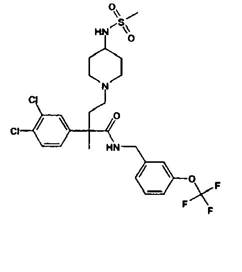 | 596.5 | 2-(3,4-Dichloro-phenyl)-4-(4-methanesulfonylami no-piperidin-1-yl)-2-methyl-N-(3-trifluoromethoxy-benzyl)-butyramide | 2-(3,4-Dichloro-phenyl)-4-(4-methanesulfonylam ino-piperidin-1-yl)-2-methyl-butyric acid (intermediate 1) and 3-Trifluoromethoxy-benzylamine (commercially available) | 596.2 | 0.1468 |
| 22 | 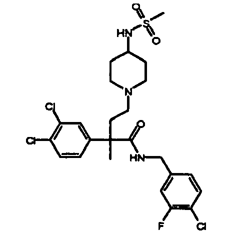 | 564.9 | N-(4-Chloro-3-fluoro-benzyl)-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 4-Chloro-3-fluoro-benzylamine (commercially available) | 566.3 | 0.0708 |
| 23 | 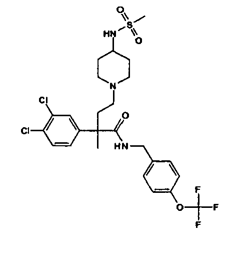 | 596.5 | 2-(3,4-Dichloro-phenyl)-4-(4-methanesulfonylami no-piperidin-1-yl) -2-methyl-N-(4-trifluoromethoxy-benzyl)-butyramide | 2-(3,4-Dichloro-phenyl)-4-(4-methanesulfonylam ino-piperidin-1-yl)-2-methyl-butyric acid (intermediate 1) and 4-Trifluoromethoxy-benzylamine (commercially available) | 596.2 | 0.1403 |
| 24 | 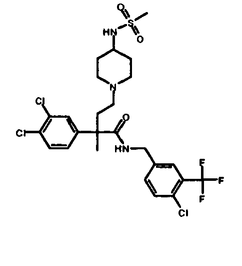 | 614.9 | N-(4-Chloro-3-trifluoromethyl-benzyl)-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and4-Chloro-3-trifluoromethyl-benzylamine (commercially available) | 616.3 | 0.0714 |
| 25 | 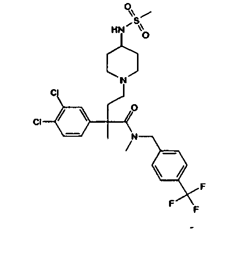 | 594.5 | 2-(3,4-Dichloro-phenyl)-4-(4-methanesulfonylami no-piperidin-1-yl)-2,N-dimethyl-N-(4-trifluoromethyl-benzyl)-butyramide | 2-(3,4-Dichloro-phenyl)-4-(4-methanesulfonylam ino-piperidin-1-yl)-2-methyl-butyric acid (intermediate 1) andMethyl-(4-trifluoromethyl-benzyl)-amine (commercially available) | 594.2 | 0.0287 |
| 26 | 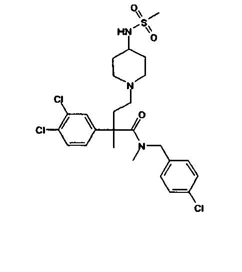 | 561.0 | N-(4-Chloro-benzyl)-2-(3,4-dichloro-phenyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (4-Chloro-benzyl)-methyl-amine (commercially available) | 560.3 | 0.0265 |
| 27 | 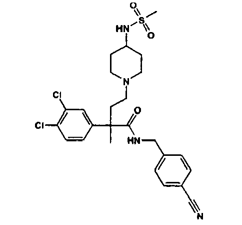 | 537.5 | N-(4-Cyano-benzyl)-2-(3,4-dichloro-phenyl)-4-(4-methanesulfonylami no-piperidin-1-yl)-2-methyl-butyramide | 2-(3,4-Dichloro-phenyl)-4-(4-methanesulfonylam ino-piperidin-1-yl)-2-methyl-butyric acid (intermediate 1) and 4-Aminomethyl-benzonitrile (commercially available) | 537.3 | 0.2633 |
| 28 | 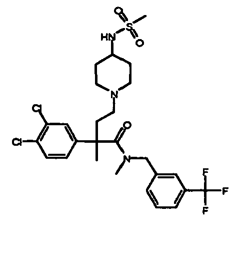 | 594.5 | 2-(3,4-Dichloro-phenyl)-4-(4-methanesulfonylami no-piperidin-1-yl)-2,N-dimethyl-N-(3-trifluoromethyl-benzyl)-butyramide | 2-(3,4-Dichloro-phenyl)-4-(4-methanesulfonylam ino-piperidin-1-yl)-2-methyl-butyric acid (intermediate 1) andMethyl-(3-trifluoromethyl-benzyl)-amine (commercially available) | 594.2 | 0.0217 |
| 29 | 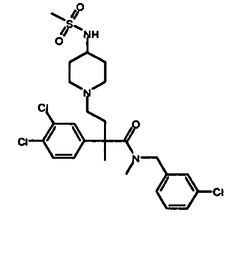 | 561.0 | N-(3-Chloro-benzyl)-2-(3,4-dichloro-phenyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (3-Chloro-benzyl)-methyl-amine (commercially available) | 560.5 | 0.0485 |
| 30 | 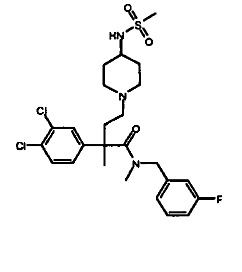 | 544.5 | 2-(3,4-Dichloro-phenyl)-N-(3-fluoro-benzyl)-4-(4-methanesulfonylami no-piperidin-1-yl)-2,N-dimethyl-butyramide | 2-(3,4-Dichloro-phenyl)-4-(4-methanesulfonylam ino-piperidin-1-yl)-2-methyl-butyric acid (intermediate 1) and (3-Fluoro-benzyl)-methyl-amine (commercially available) | 544.2 | 0.1233 |
| 31 | 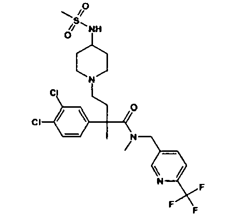 | 595.5 | 2-(3,4-Dichloro-phenyl)-4-(4-methanesulfonylami no-piperidin-1-yl)-2,N-dimethyl-N-(6-trifluoromethyl-pyridin-3-ylmethyl)-butyramide | 2-(3,4-Dichloro-phenyl)-4-(4-methanesulfonylam ino-piperidin-1-yl)-2-methyl-butyric acid (intermediate 1) and Methyl-(6-trifluoromethyl-pyridin-3-ylmethyl)-amine (commercially available) | 595.2 | 0.5004 |
| 32 | 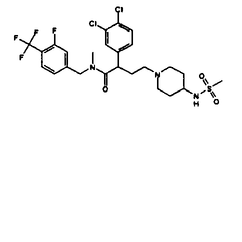 | 598.5 | 2-(3,4-Dichloro-phenyl)-N-(3-fluoro-4-trifluoromethyl-benzyl)-4-(4-methanesulfonylami no-piperidin-1-yl)-N-methyl-butyramide | 2-(3,4-Dichloro-phenyl)-4-(4-methanesulfonylam ino-piperidin-1-yl)-butyric acid (intermediate 2) and (3-Fluoro-4-trifluoromethyl-benzyl)-methyl-amine, hydrochloride (intermediate 12) | 598.3 | 0.0617 |
| 33 | 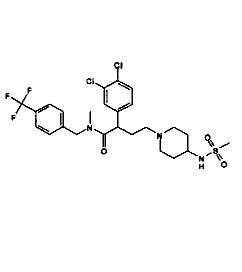 | 580.5 | 2-(3,4-Dichloro-phenyl)-4-(4-methanesulfonylami no-piperidin-1-yl)-N-methyl-N-(4-trifluoromethyl-benzyl)-butyramide | 2-(3,4-Dichloro-phenyl)-4-(4-methanesulfonylam ino-piperidin-1-yl)-butyric acid (intermediate 2) and Methyl-(4trifluoromethyl-benzyl)-amine (commercially available) | 580.3 | 0.0816 |
| 34 | 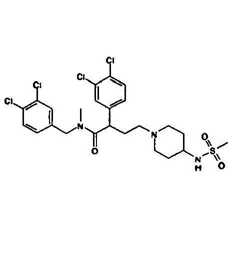 | 581.4 | N-(3,4-Dichloro-benzyl)-2-(3,4-dichloro-phenyl)-4-(4-methanesulfonylami no-piperidin-1-yl)-N-methyl-butyramide | 2-(3,4-Dichlorophenyl)-4-(4-methanesulfonylam ino-piperidin-1-yl)-butyric acid (intermediate 2) and (3,4-Dichloro-benzyl)-methyl-amine (commercially available) | 582.1 | 0.0775 |
| 35 | 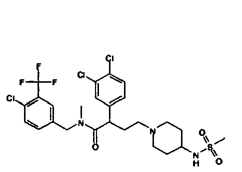 | 614.9 | N-(4-Chloro-3-trifluoromethyl-benzyl)-2-(3,4-dichloro-phenyl)-4-(4-methanesulfonylami no-piperidin-1-yl)-N-methyl-butyramide | 2-(3,4-Dichloro-phenyl)-4-(4-methanesulfonylam ino-piperidin-1-yl)-butyric acid (intermediate 2) and (4-Chloro-3-trifluoromethyl-benzyl) -methyl-amine (commercially available) | 616.1 | 0.1494 |
| 36 | 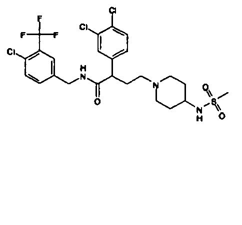 | 600.9 | N-(4-Chloro-3-trifluoromethyl-benzyl)-2-(3,4-dichloro-phenyl)-4-(4-methanesulfonylami no-piperidin-1-yl)-butyramide | 2-(3,4-Dichloro-phenyl)-4-(4-methanesulfonylam ino-piperidin-1-yl)-butyric acid (intermediate 2) and 4-Chloro-3-trifluoromethyl-benzylamine (commercially available) | 600.2 | 0.3541 |
| 37 | 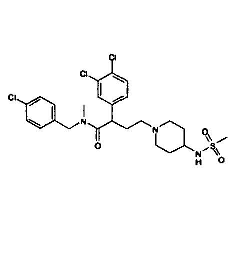 | 546.9 | N-(4-Chloro-benzyl)-2-(3,4-dichloro-phenyl)-4-(4-methanesulfonylami no-piperidin-1-yl)-N-methyl-butyramide | 2-(3,4-Dichloro-phenyl)-4-(4-methanesulfonylam ino-piperidin-1-yl)-butyric acid (intermediate 2) and (4-Chloro-benzyl)-methyl-amine (commercially available) | 546.2 | 0.0787 |
| 38 | 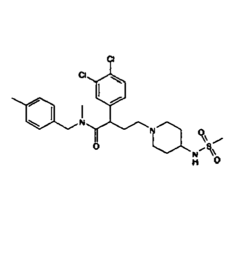 | 526.5 | 2-(3,4-Dichloro-phenyl)-4-(4-methanesulfonylami no-piperidin-1-yl)-N-methyl-N-(4-methyl-benzyl)-butyramide | 2-(3,4-Dichloro-phenyl)-4-(4-methanesulfonylam ino-piperidin-1-yl)-butyric acid (intermediate 2) and Methyl-(4-methyl-benzyl)-amine (commercially available) | 526.4 | 0.0929 |
| 39 | 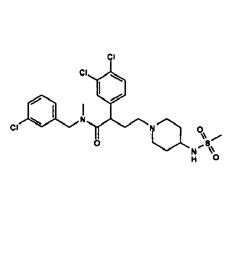 | 546.9 | N-(3-Chloro-benzyl)-2-(3,4-dichloro-phenyl)-4-(4-methanesulfonylami no-piperidin-1-yl)N-methyl-butyramide | 2-(3,4-Dichloro-phenyl)-4-(4-methanesulfonylam ino-piperidin-1-yl)-butyric acid (intermediate 2) and (3-Chloro-benzyl)-methyl-amine (commercially available) | 546.2 | 0.2126 |
| 40 | 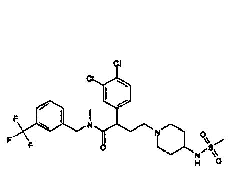 | 580.5 | 2-(3,4-Dichloro-phenyl)-4-(4-methanesulfonylami no-piperidin-1-yl)-N-methyl-N-(3-trifluoromethyl-benzyl)-butyramide | 2-(3,4-Dichloro-phenyl)-4-(4-methanesulfonylam ino-piperidin-1-yl)butyric acid (intermediate 2) and Methyl-(3-trifluoromethyl-benzyl)-amine (commercially available) | 580.3 | 0.2801 |
| 41 | 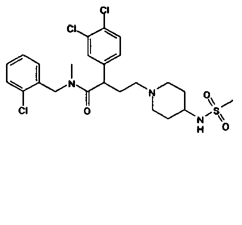 | 546.9 | N-(2-Chloro-benzyl)-2-(3,4-dichloro-phenyl)-4(4-methanesulfonylami no-piperidin-1-yl)-N-methyl-butyramide | 2-(3,4-Dichloro-phenyl)-4-(4-methanesulfonylam ino-piperidin-1-yl)-butyric acid (intermediate 2) and (2-Chloro-benzyl)-methyl-amine (commercially available) | 546.2 | 0.2588 |
| 42 | 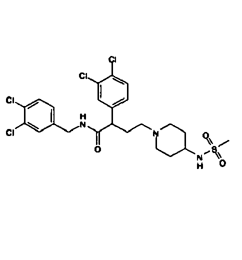 | 567.4 | N-(3,4-Dichloro-benzyl)-2-(3,4-dichloro-phenyl)-4-(4-methanesulfonylami no-piperidin-1-yl)-butyramide | 2-(3,4-Dichloro-phenyl)-4-(4-methanesulfonylam ino-piperidin-1-yl)-butyric acid (intermediate 2) and 3,4-Dichloro-benzylamine (commercially available) | 568.3 | 0.1919 |
| 43 | 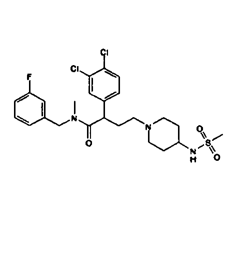 | 530.5 | 2-(3,4-Dichloro-phenyl)-N-(3-fluoro-benzyl)-4-(4-methanesulfonylami no-piperidin-1-yl)-N-methyl-butyramide | 2-(3,4-Dichloro-phenyl)-4-(4-methanesulfonylam ino-piperidin-1-yl)-butyric acid (intermediate 2) and (3-Flulorobenzyl)-methyl-amine (commercially available) | 530.1 | 0.2544 |
| 44 | 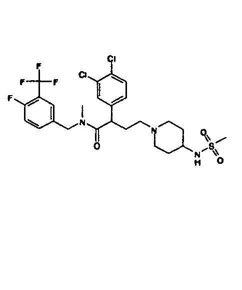 | 598.5 | 2-(3,4-Dichloro-phenyl)-N-(4-fluoro-3-trifluoromethyl-benzyl)-4-(4methanesulfonylami no-piperidin-1-yl)-N-methyl-butyramide | 2-(3,4-Dichloro-phenyl)-4-(4-methanesulfonylam ino-piperidin-1-yl)-butyric acid (intermediate 2) and (4-Fluoro-3-trifluoromethyl-benzyl)-methyl-amine, hyrdochloride (intermediate 13) | 598.3 | 0.1323 |
| 45 | 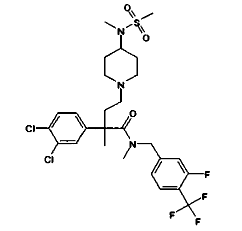 | 626.5 | 2-(3,4-Dichloro-phenyl)-N-(3-fluoro-4-trifluoromethyl-benzyl)-4-[4-(methanesulfonyl-methyl-amino)-piperidin-1-yl]-2,N-dimethyl-butyramide | 2-(3,4-Dichloro-phenyl)-4-[4-(methanesulfonyl-methyl-amino)-piperidin-1-yl]-2-methyl-butyric acid (intermediate 3) and (3-Fluoro-4-trifluoromethyl-benzyl)-methyl-amine, hydrochloride (intermediate 12) | 625.8 | 0.0085 |
| 46 | 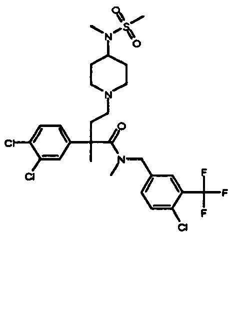 | 643.0 | N-(4-Chloro-3-trifluoromethyl-benzyl)-2-(3,4-dichloro-phenyl)-4-[4-(methanesulfonyl-methyl-amino)-piperidin-1-yl]-2,N-dimethyl-butyramide | 2-(3,4-Dichloro-phenyl)-4-[4-(methanesulfonyl-methyl-amino)-piperidin-l-yl]-2-methyl-butyric acid (intermediate 3) and (4-Chloro-3-trifluoromethyl-benzyl) -methyl-amine (commercially available) | 641.8 | 0.0024 |
| 47 | 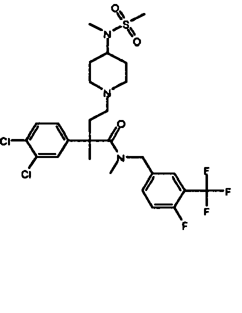 | 626.5 | 2-(3,4-Dichloro-phenyl)-N-(4-fluoro-3-trifluoromethyl-benzyl)-4-[4-(methanesulfonyl-methyl-amino)-piperidin-1-yl]-2,N-dimethyl-butyramide | 2-(3,4-Dichloro-phenyl)-4-[4-(methanesulfonyl-methyl-amino)-piperidin-1-yl]-2-methyl-butyric acid (intermediate 3) and (4-Fluoro-3-trifluoromethyl-benzyl)-methyl-amine, hyrdochloride (intermediate 13) | 625.7 | 0.0021 |
| 48 | 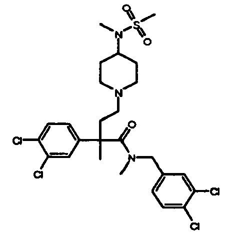 | 609.4 | N-(3,4-Dichloro-benzyl)-2-(3,4-dichloro-phenyl)-4-[4-(methanesulfonyl-methyl-amino)-piperidin-1-yl]-2,N-dimethyl-butyramide | 2-(3,4-Dichloro-phenyl)-4-[4-(methanesulfonyl-methyl-amino)-piperidin-1-yl]-2-methyl-butyric acid (intermediate 3) and (3,4-Dichloro-benzyl)-methyl-amine (commercially available) | 609.5 | 0.0036 |
| 49 | 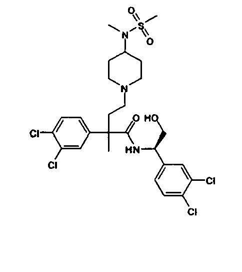 | 625.4 | 2-(3,4-Dichloro-phenyl)-N-[1-(3,4-dichloro-phenyl)-2-hydroxy-ethyl]-4-[4-(methanesulfonyl-methyl-amino)-piperidin-1-yl]-2-methyl-butyramide; diastereoisomer 1 | 2-(3,4-Dichloro-phenyl)-4-[4-(methanesulfonyl-methyl-amino)-piperidin-1-yl]-2-methyl-butyric acid (intermediate 3) and 2-Amino-2-(3,4-dichloro-phenyl)-ethanol (WO 2005058892) | 626.2 | 0.0055 |
| 50 | 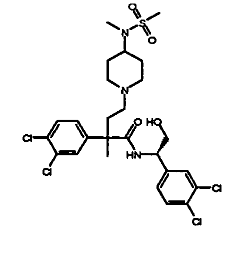 | 625.4 | 2-(3,4-Dichloro-phenyl)-N-[1-(3,4-dichloro-phenyl)-2-hydroxy-ethyl]-4-[4-(methanesulfonyl-methyl-amino)-piperidin-1-yl]-2-methyl-butyramide; diastereoisomer 2 | 2-(3,4-Dichloro-phenyl)-4-[4-(methanesulfonyl-methyl-amino)-piperidin-1-yl]-2-methyl-butyric acid (intermediate 3) and 2-Amino-2-(3,4-dichloro-phenyl)-ethanol (WO 2005058892) | 626.2 | 0.0521 |
| 51 | 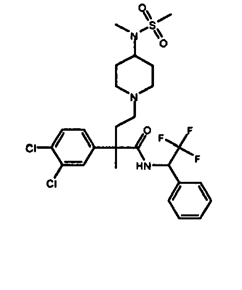 | 594.5 | 2-(3,4-Dichloro-phenyl)-4-[4-(methanesulfonyl-methyl-amino)-piperidin-1-yl]-2-methyl-N-(2,2,2-trifluoro-l-phenyl-ethyl)-butyramide | 2-(3,4-Dichloro-phenyl)-4-[4-(methanesulfonyl-methyl-amino)-piperidin-1-yl]-2-methyl-butyric acid (intermediate 3) and 2,2,2-Trifluoro-1-phenyl-ethylamine (commercially available) | 594.3 | 0.0187 |
| 52 | 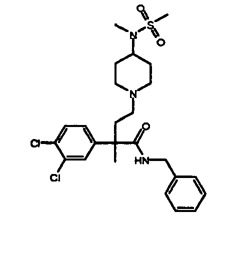 | 526.5 | N-Benzyl-2-(3,4-dichloro-phenyl)-4-[4-(methanesulfonyl-methyl-amino)-piperidin-1-yl]-2-methyl-butyramide | 2-(3,4-Dichloro-phenyl)-4-[4-(methanesulfonyl-methyl-amino)-piperidin-1-yl]-2-methyl-butyric acid (intermediate 3) and benzylamine (commercially available) | 525.8 | 0.0648 |
| 53 | 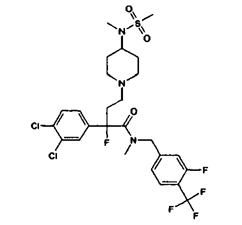 | 630.5 | 2-(3,4-Dichloro-phenyl)-2-fluoro-N-(3-fluoro-4-trifluoromethyl-benzyl)-4-[4-(methanesulfonyl-methyl-amino)-piperidin-1-yl]-N-methyl-butyramide | 2-(3,4-Dichloro-phenyl)-2-fluoro-4-[4-(methanesulfonyl-methyl-amino)-piperidin-1-yl]-butyric acid (intermediate 4) and (3-Fluoro-4-trifluoromethyl-benzyl)-methyl-amine, hydrochloride (intermediate 12) | 629.8 | 0.0183 |
| 54 | 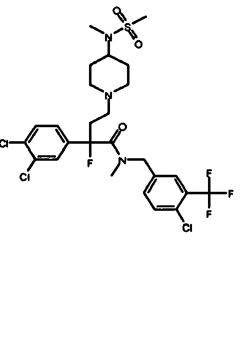 | 647.0 | N-(4-Chloro-3-trifluoromethyl-benzyl)-2-(3,4-dichloro-phenyl)-2-fluoro-4-[4-(methanesulfonyl-methyl-amino)-piperidin-1-yl]-N-methyl-butyramide | 2-(3,4-Dichloro-phenyl)-2-fluoro-4-[4-(methanesulfonyl-methyl-amino)-piperidin-1-yl]-butyric acid (intermediate 4) and (4-Chloro-3-trifluoromethyl-benzyl)-methyl-amine (commercially available) | 645.7 | 0.0245 |
| 55 | 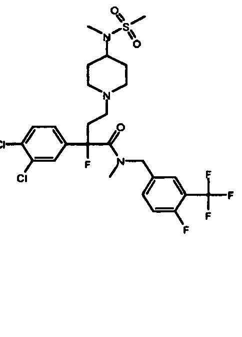 | 630.5 | 2-(3,4-Dichloro-phenyl)-2-fluoro-N-(4-fluoro-3-trifluoromethyl-benzyl)-4-[4-(methanesulfonyl-methyl-amino)-piperidin-1-yl]-N-methyl-butyramide | 2-(3,4-Dichloro-phenyl)-2-fluoro-4-[4-(methanesulfonyl-methyl-amino)-piperidin-1-yl]-butyric acid (intermediate 4) and (4-Fluoro-3-trifluoromethyl-benzyl)-methyl-amine, hyrdochloride (intermediate 13) | 629.8 | 0.0234 |
| 56 | 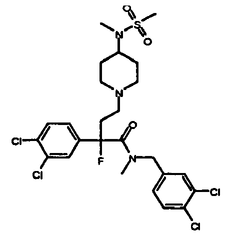 | 613.4 | N-(3,4-Dichloro-benzyl)-2-(3,4-dichloro-phenyl)-2-fluoro-4-[4-(methanesulfonyl-methyl-amino)-piperidin-1-yl]-N-methyl-butyramide | 2-(3,4-Dichloro-phenyl)-2-fluoro-4-[4-(methanesulfonyl-methyl-amino)-piperidin-1-yl]-butyric acid (intermediate 4) and (3,4-Dichloro-benzyl)-methyl-amine (commercially available) | 613.7 | 0.0228 |
| 57 | 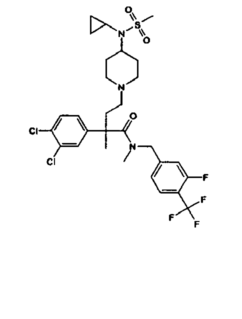 | 652.6 | 4-[4-(Cyclopropyl-methanesu fonyl-amino)-piperidin-1-yl]-2-(3,4-dichloro-phenyl)-N-(3-fluoro-4-trifluoromethyl-benzyl)-2,N-dimethyl-butyramide | 4-[4-(Cyclopropyl-methanesu fonyl-amino)-piperidin-1-yl]-2-(3,4-dichloro-phenyl)-2-methyl-butyric acid (intermediate 6) and (3-Fluoro-4-trifluoromethyl-benzyl)-methyl-amine, hydrochloride (intermediate 12) | 653.8 | 0.0059 |
| 58 | 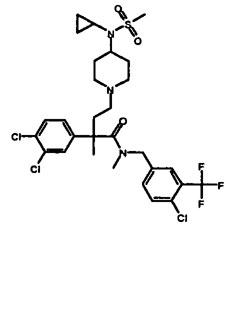 | 669.0 | N-(4-Chloro-3-trifluoromethyl-benzyl)-4-[4-(cyclopropyl-methanesulfonyl-amino)-piperidin-1-yl]-2-(3,4-dichloro-phenyl)-2,N-dimethyl-butyramide | 4-[4-(Cyclopropyl-methanesu fonyl-amino)-piperidin-1-yl]-2-(3,4-dichloro-phenyl)-2-methyl-butyric acid (intermediate 6) and (4-Chloro-3-trifluoromethyl-benzyl)-methyl-amine (commercially available) | 669.8 | 0.0036 |
| 59 | 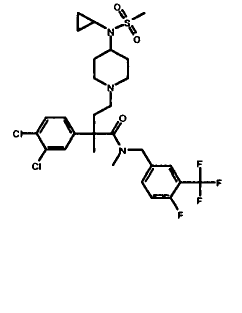 | 652.6 | 4-[4-(Cyclopropyl-methanesulfonyl-amino)-piperidin-1-yl]-2-(3,4-dichloro-phenyl)-N-(4-fluoro-3-trifluoromethyl-benzyl)-2,N-dimethyl-butyramide | 4-[4-(Cyclopropyl-methanesulfonyl-amino)-piperidin-1-yl]-2-(3,4-dichloro-phenyl)-2-methyl-butyric acid (intermediate 6) and (4-Fluoro-3-trifluoromethyl-benzyl)-methyl-amine, hyrdochloride (intermediate 13) | 653.7 | 0.0043 |
| 60 | 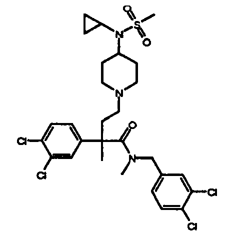 | 635.5 | 4-[4-(Cyclopropyl-methanesu fonyl-amino)-piperidin-1-yl]-N-(3,4-dichloro-benzyl)-2-(3,4-dichloro-phenyl)-2,N-dimethyl-butyramide | 4-[4-(Cyclopropyl-methanesulfonyl-amino)-piperidin-1-yl]-2-(3,4-dichloro-phenyl)-2-methyl-butyric acid (intermediate 6) and (3,4-Dichloro-benzyl)-methyl-amine (commercially available) | 635.5 | 0.0026 |
| 61 | 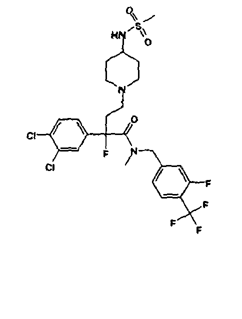 | 616.5 | 2-(3,4-Dichloro-phenyl)-2-fluoro-N-(3-fluoro-4-trifluoromethyl-benzyl)-4-(4-methanesulfonylami no-piperidin-1-yl)-N-methyl-butyramide | 2-(3,4-Dichloro-phenyl)-2-fluoro-4-(4-methanesulfonylam ino-piperidin-1-yl)-butyric acid (intermediate 7) and (3-Fluoro-4-trifluoromethyl-benzyl)-methyl-amine, hydrochloride (intermediate 12) | 616.1 | 0.08 |
| 62 | 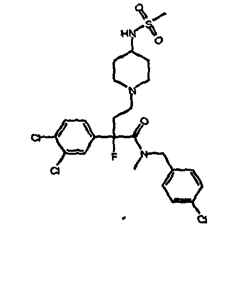 | 564.9 | N-(4-Chlorobenzyl)-2-(3,4-dichloro-phenyl)-2-fluoro-4-(4-methanesulfonylami : no-piperidin-1-yl) -N-methyl-butyramide | 2-(3,4-Dichloro-phenyl)-2-fluoro-4-(4-methanesulfonylam ino-piperidin-1-yl)-butyric acid (intermediate 7) and (4-Chloro-benzyl)-methyl-amine (commercially available) | 564.2 | 0.1052 |
| 63 | 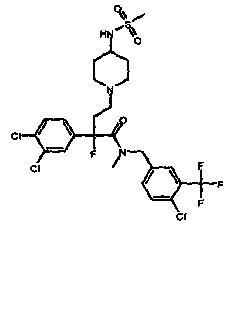 | 632.9 | N-(4-Chloro-3-trifluoromethyl-benzyl)-2-(3,4-dichloro-phenyl)-2-fluoro-4-(4-methanesulfonylami no-piperidin-1-yl)-N-methyl-butyramide | 2-(3,4-Dichloro-phenyl)-2-fluoro-4-(4-methanesulfonylam ino-piperidin-1-yl)-butyric acid (intermediate 7) and (4-Chloro-3-trifluoromethyl-benzyl)-methyl-amine (commercially available) | 632.2 | 0.1169 |
| 64 | 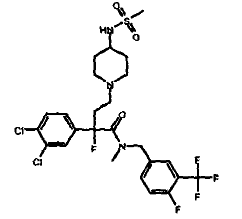 | 616.5 | 2-(3,4-Dichloro-phenyl)-2-fluoro-N-(4-fluoro-3-trifluoromethyl-benzyl)-4-(4-methanesulfonylami no-piperidin-1-yl)-N-methyl-butyramide | 2-(3,4-Dichloro-phenyl)-2-fluoro-4-(4-methanesulfonylam ino-piperidin-1-yl)-butyric acid (intermediate 7) and (4-Fluoro-3-trifluoromethyl-benzyl)-methyl-amine, hyrdochloride (intermediate 13) | 616.1 | 0.1219 |
| 65 | 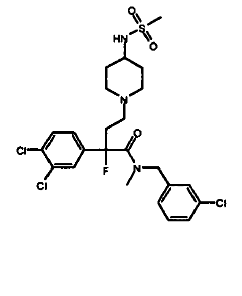 | 564.9 | N-(3-Chloro-benzyl)-2-(3,4-dichloro-phenyl)-2-fluoro-4-(4-methanesulfonylami no-piperidin-1-yl)-N-methyl-butyramide | 2-(3,4-Dichloro-phenyl)-2-fluoro-4-(4-methanesulfonylam ino-piperidin-1-yl)-butyric acid (intermediate 7) and (3-Chloro-benzyl)-methyl-amine (commercially available) | 564.2 | 0.3067 |
| 66 | 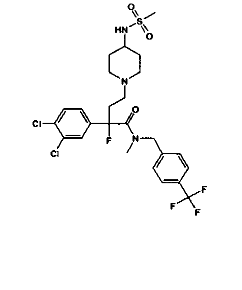 | 598.5 | 2-(3,4-Dichloro-phenyl)-2-fluoro-4-(4-methanesulfonylami no-piperidin-1-yl)-N-methyl-N-(4-trifluoromethyl-benzyl)-butyramide | 2-(3,4-Dichloro-phenyl)-2-fluoro-4-(4-methanesulfonylam ino-piperidin-1-yl)-butyric acid (intermediate 7) and Methyl-(4-trifluoromethyl-benzyl)-amine (commercially available) | 598.3 | 0.0843 |
| 67 | 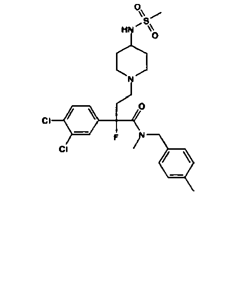 | 544.5 | 2-(3,4-Dichloro-phenyl)-2-fluoro-4-(4-methanesulfonylami no-piperidin-1-yl)-N-methyl-N-(4-methyl-benzyl)-butyramide | 2-(3,4-Dichloro-phenyl)-2-fluoro-4-(4-methanesulfonylam ino-piperidin-1-yl)-butyric acid (intermediate 7) and Methyl-(4-methyl-benzyl)-amine (commercially available) | 546.2 | 0.3227 |
| 68 | 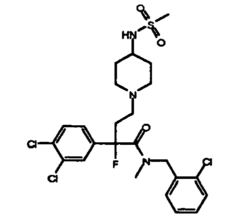 | 564.9 | N-(2-Chloro-benzyl)-2-(3,4-dichloro-phenyl)-2-fluoro-4-(4-methanesulfonylami no-piperidin-1-yl)-N-methyl-butyramide | 2-(3,4-Dichloro-phenyl)-2-fluoro-4-(4-methanesulfonylam ino-piperidin-1-yl)-butyric acid (intermediate 7) and (2-Chloro-benzyl)-methyl-amine (commercially available) | 564.2 | 0.1585 |
| 69 | 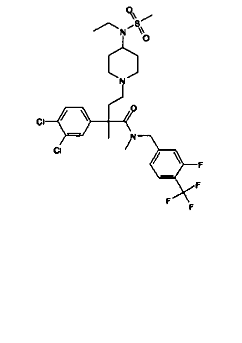 | 640.6 | 2-(3,4-Dichloro-phenyl)-4-[4-(ethyl-methanesulfonyl-amino)-piperidin-1-yl]-N-(3-fluoro-4-trifluoromethyl-benzyl)-2,N-dimethyl-butyramide | 2-(3,4-Dichloro-phenyl)-4-[4-(ethyl-methanesulfonyl-amino)-piperidin-1-yl]-2-methyl-butyric acid (intermediate 9) and (3-Fluoro-4-trifluoromethyl-benzyl)-methyl-amine, hydrochloride (intermediate 12) | 640.3 | 0.0024 |
| 70 | 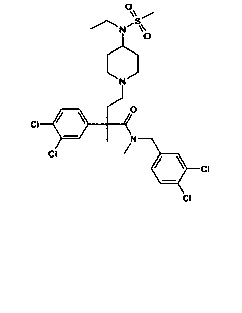 | 623.5 | N-(3,4-Dichloro-benzyl)-2-(3,4-dichloro-phenyl)-4-[4-(ethyl-methanesulfonyl-amino)-piperidin-1-yl]-2,N-dimethyl-butyramide | 2-(3,4-Dichloro-phenyl)-4-[4-(ethyl-methanesulfonyl-amino)-piperidin-1-yl]-2-methyl-butyric acid (intermediate 9) and (3,4-Dichloro-benzyl)-methyl-amine (commercially available) | 624.3 | 0.0012 |
| 71 | 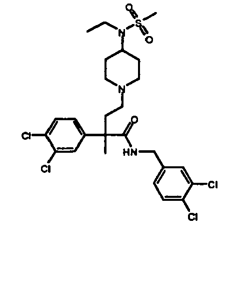 | 609.4 | N-(3,4-Dichloro-benzyl)-2-(3,4-dichloro-phenyl)-4-[4-(ethyl-methanesulfonyl-amino)-piperidin-1-yl]-2-methyl-butyramide | 2-(3,4-Dichloro-phenyl)-4-[4-(ethyl-methanesulfonyl-amino)-piperidin-1-yl]-2-methyl-butyric acid (intermediate 9) and 3,4-Dichloro-benzylamine (commercially available) | 610.1 | 0.0028 |
| 72 | 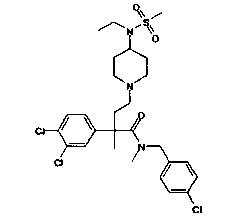 | 589.0 | N-(4-Chloro-benzyl)-2-(3,4-dichloro-phenyl)-4-[4-(ethyl-methanesulfonyl-amino)-piperidin-1-yl]-2,N-dimethyl-butyramide | 2-(3,4-Dichloro-phenyl)-4-[4-(ethyl-methanesulfonyl-amino)-piperidin-1-yl]-2-methyl-butyric acid (intermediate 9) and (4-Chloro-benzyl)-methyl-amine (commercially available) | 590.2 | 0.0022 |
| 73 | 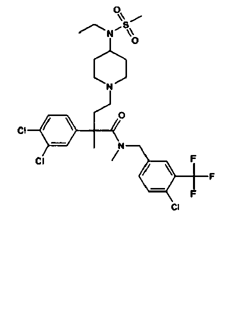 | 657.0 | N-(4-Chloro-3-trifluoromethyl-benzyl)-2-(3,4-dichloro-phenyl)-4-[4-(ethyl-methanesulfonyl-amino)-piperidin-1-yl]-2,N-dimethyl-butyramide | 2-(3,4-Dichloro-phenyl)-4-[4-(ethyl-methanesulfonyl-amino)-piperidin-1-yl]-2-methyl-butyric acid (intermediate 9) and 4-Chloro-3-trifluoromethyl-benzylamine (commercially available) | 658.3 | 0.0015 |
| 74 | 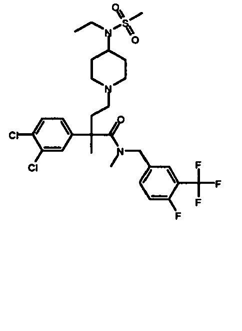 | 640.6 | 2-(3,4-Dichlorophenyl)-4-[4-(ethyl-methanesulfonyl-amino)-piperidin-1-yl]-N-(4-fluoro-3-trifluoromethyl-benzyl)-2,N-dimethyl-butyramide | 2-(3,4-Dichloro-phenyl)-4-[4(ethyl-methanesulfonyl-amino)-piperidin-1-yl]-2-methyl-butyric acid (intermediate 9) and (4-Fluoro-3-trifluoromethyl-benzyl)-methyl-amine, hyrdochloride (intermediate 13) | 640.2 | 0.0013 |
| 75 | 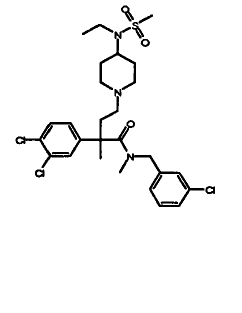 | 589.0 | N-(3-Chloro-benzyl)-2-(3,4-dichloro-phenyl)-4-[4-(ethyl-methanesulfonyl-amino)-piperidin-1-yl]-2,N-dimethyl-butyramide | 2-(3,4-Dichloro-phenyl)-4-[4-(ethyl-methanesulfonyl-amino)-piperidin-1-yl]-2-methyl-butyric acid (intermediate 9) and (3-Chloro-benzyl)-methyl-amine (commercially available) | 590.2 | 0.0039 |
| 76 | 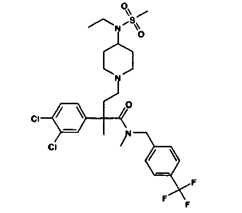 | 622.6 | 2-(3,4-Dichloro-phenyl)-4-[4-(ethyl-methanesulfonyl-amino)-piperidin-1-yl]-2,N-dimethyl-N-(4-trifluoromethyl-benzyl)-butyramide | 2-(3,4-Dichloro-phenyl)-4-[4-(ethyl-methanesulfonyl-amino)-piperidin-1-yl]-2-methyl-butyric acid (intermediate 9) and Methyl-(4-trifluoromethyl-benzyl)-amine (commercially available) | 622.4 | 0.0017 |
| 77 | 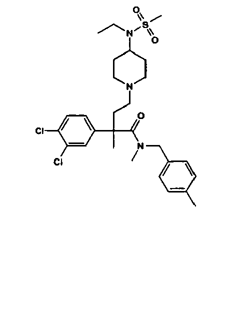 | 568.6 | 2-(3,4-Dichloro-phenyl)-4-[4-(ethyl-methanesulfonyl-amino)-piperidine-1 -yl]-2,N-dimethyl-N-(4-methyl-benzyl)-butyramide | 2-(3,4-Dichloro-phenyl)-4-[4-(ethyl-methanesulfonyl-amino)-piperidin-1-yl]-2-methyl-butyric acid (intermediate 9) and Methyl-(4-methyl-benzyl)-amine (commercially available) | 568.3 | 0.0031 |
| 78 | 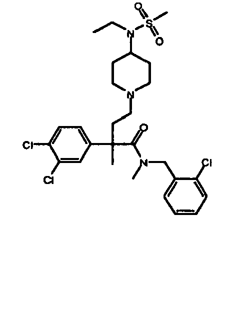 | 589.0 | N-(2-Chloro-benzyl)-2-(3,4-dichloro-phenyl)-4-[4-(ethyl-methanesulfonyl-amino)-piperidin-1-yl]-2,N-dimethyl-butyramide | 2-(3,4-Dichloro-phenyl)-4-[4-(ethyl-methanesulfonyl-amino)-piperidin-1-yl]-2-methyl-butyric acid (intermediate 9) and (2-Chloro-benzyl)-methyl-amine (commercially available) | 590.2 | 0.0036 |
| 79 | 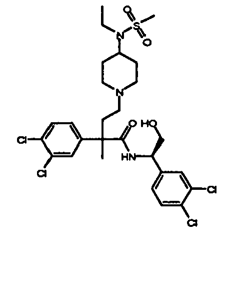 | 639.5 | 2-(3,4-Dichloro-phenyl)-N-[1-(3,4-dichloro-phenyl)-2-hydroxy-ethyl]-4-[4-(ethyl-methanesulfonyl-amino)-piperidin-1-yl]-2-methyl-butyramide; diastereoisomer 1 | 2-(3,4-Dichloro-phenyl)-4-[4-(ethyl-methanesulfonyl-amino)-piperidin-1-yl]-2-methyl-butyric acid (intermediate 9) and 2-Amino-2-(3,4-dichloro-phenyl)-ethanol (WO 2005058892) | 640.3 | 0.0015 |
| 80 | 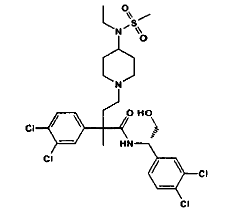 | 639.5 | 2-(3,4-Dichloro-phenyl)-N-[1-(3,4-dichloro-phenyl)-2-hydroxy-ethyl]-4-[4-(ethyl-methanesulfonyl-amino)-piperidin-1-yl]-2-methyl-butyramide; diastereoisomer 2 | 2-(3,4-Dichloro-phenyl)-4-[4-(ethyl-methanesulfonyl-amino)-piperidin-1-yl]-2-methyl-butyric acid (intermediate 9) and 2-Amino-2-(3,4-dichloro-phenyl)-ethanol (WO 2005058892) | 640.2 | 0.0133 |
| 81 | 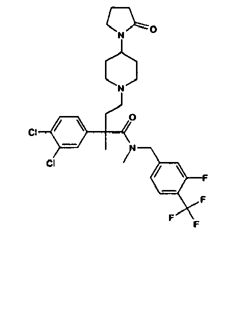 | 602.5 | 2-(3,4-Dichloro-phenyl)-N-(3-fluoro-4-trifluoromethyl-benzyl)-2,N-dimethyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and (3-Fluoro-4-trifluoromethyl-benzyl)-methyl-amine, hydrochloride (intermediate 12) | 601.9 | 0.003 |
| 82 | 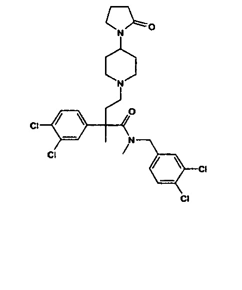 | 585.4 | N-(3,4-Dichloro-benzyl)-2-(3,4-dichloro-phenyl)-2,N-dimethyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and (3,4-Dichloro-benzyl)-methyl-amine (commercially available) | 585.7 | 0.0012 |
| 83 | 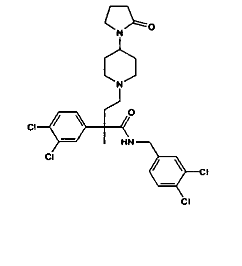 | 571.4 | N-(3,4-Dichloro-benzyl)-2-(3,4-dichloro-phenyl)-2-methyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and 3,4-Dichloro-benzylamine (commercially available) | 569.7 | 0.0023 |
| 84 | 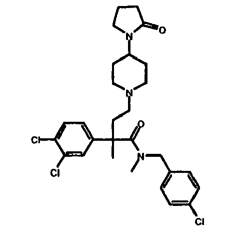 | 551.0 | N-(4-Chloro-benzyl)-2-(3,4-dichloro-phenyl)-2,N-dimethyl-4-[4-(2-oxo-pyrrolidin-1-yl)-piperidin-1-yl]-butyramide | 2-(3,4-Dichloro-phenyl)-2-methyl-4-[4-(2-oxopyrrolidin-1-yl)-piperidin-1-yl]-butyric acid (intermediate 10) and (4-Chloro-benzyl)-methyl-amine (commercially available) | 549.9 | 0.0021 |
| 85 | 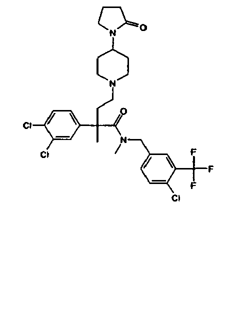 | 619.0 | N-(4-Chloro-3-trifluoromethylbenzyl)-2-(3,4-dichloro-phenyl)-2,N-dimethyl-4-[4-(2-oxo-pyrrolidin-l-yl)-piperidin-1-yl]-butyramide | 2-(3,4-Dichloro-phenyl)-2-methyl-4-[4-(2-oxopyrrolidin-1-yl)-piperidin-1-yl]-butyric acid (intermediate 10) and 4-Chloro-3-trifluoromethyl-benzylamine (commercially available) | 619.7 | 0.0014 |
| 86 | 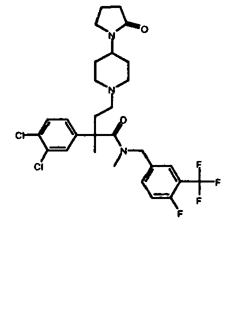 | 602.5 | 2-(3,4-Dichloro-phenyl)-N-(4-fluoro-3-trifluoromethyl-benzyl)-2,N-dimethyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and (4-Fluoro-3-trifluoromethyl-benzyl)-methyl-amine, hyrdochloride (intermediate 13) | 601.9 | 0.0014 |
| 87 | 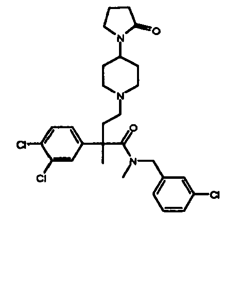 | 551.0 | N-(3-Chloro-benzyl)-2-(3,4-dichloro-phenyl)-2,N-dimethyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and (3-Chloro-benzyl)-methyl-amine (commercially available) | 549.9 | 0.0046 |
| 88 | 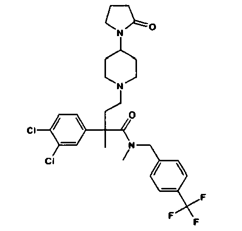 | 584.5 | 2-(3,4-Dichloro-phenyl)-2,N-dimethyl-4-[4-(2-oxo-pyrrolidin-1-yl)-piperidin-1-yl]-N-(4-trifluoromethyl-benzyl)-butyramide | 2-(3,4-Dichloro-phenyl)-2-methyl-4-[4-(2-oxo-pyrrolidin-1-yl)-piperidin-1-yl]-butyric acid (intermediate 10) and Methyl-(4-trifluoromethyl-benzyl)-amine (commercially available) | 583.7 | 0.0018 |
| 89 | 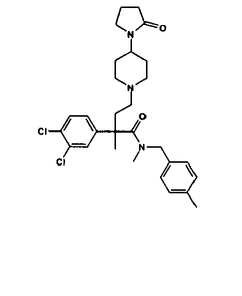 | 530.5 | 2-(3,4-Dichloro-phenyl)-2,N-dimethyl-N-(4-methyl-benzyl)-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and Methyl-(4-methyl-benzyl)-amine (commercially available) | 529.9 | 0.0053 |
| 90 | 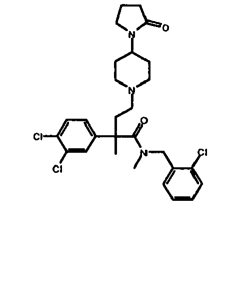 | 551.0 | N-(2-Chloro-benzyl)-2-(3,4-dichloro-phenyl)-2,N-dimethyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and (3-Chloro-benzyl)-methyl-amine (commercially available) | 551.8 | 0.0025 |
| 91 | 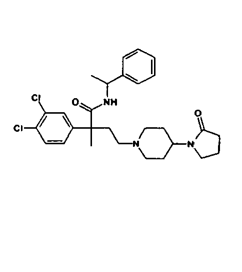 | 516.5 | 2-(3,4-Dichloro-phenyl)-2-methyl-4-[4-(2-oxo-pyrrolidin-1-yl)-piperidin-1-yl]-N-(1-phenyl-ethyl)-butyramide | 2-(3,4-Dichloro-phenyl)-2-methyl-4-[4-(2-oxo-pyrrolidin-1-yl)-piperidin-1-yl]-butyric acid (intermediate 10) and 1-Phenyl-ethylamine (commercially available) | 516.2 | 0.0512 |
| 92 | 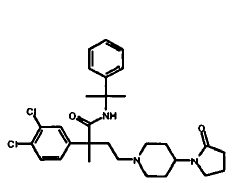 | 530.5 | 2-(3,4-Dichloro-phenyl)-2-methyl-N-(1-methyl-1-phenyl-ethyl)-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and 1-Methyl-1-phenyl-ethylamine (commercially available) | 530.2 | 0.9912 |
| 93 | 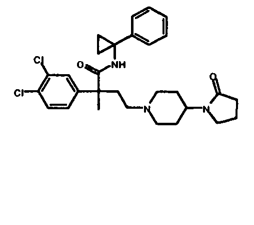 | 528.5 | 2-(3,4-Dichloro-phenyl)-2-methyl-4-[4-(2-oxo-pyrrolidin-1-yl)-piperidin-1-yl]-N-(1-phenyl-cyclopropyl)-butyramide | 2-(3,4-Dichloro-phenyl)-2-methyl-4-[4-(2-oxo-pyrrolidin-1-yl)-piperidin-1-yl]-butyric acid (intermediate 10) and 1-Phenyl-cyclopropylamine (commercially available) | 528.1 | 0.1842 |
| 94 | 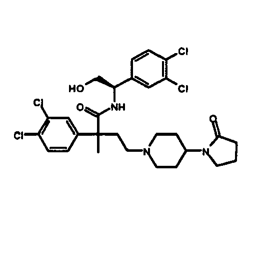 | 601.4 | 2-(3,4-Dichloro-phenyl)-N-[-1-(3,4-dichloro-phenyl)-2-hydroxy-ethyl]-2-methyl-4-[4-(2-oxo-Pyrrolidin-1-yl)-piperidin-1-yl]-butyramide; diastereoisomer 1 | 2-(3,4-Dichloro-phenyl)-2-methyl-4-[4-(2-oxo-pyrrolidin-1-yl)-piperidin-1-yl]-butyric acid (intermediate 10) and 2-Amino-2-(3,4-dichloro-phenyl)-ethanol (WO 2005058892) | 602.1 | 0.003 |
| 95 | 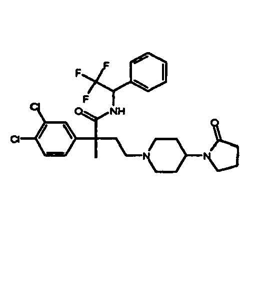 | 570.5 | 2-(3,4-Dichloro-phenyl)-2-methyl-4-[4-(2-oxo-pyrrolidin-1-yl)-piperidin-1-yl]-N-(2,2,2-trifluoro-1-phenyl-ethyl)-butyramide | 2-(3,4-Dichloro-phenyl)-2-methyl-4-[4-(2-oxo-pyrrolidin-1-yl)-piperidin-1-yl]-butyric acid (intermediate 10) and 2,2,2-Trifluoro-1-phenyl-ethylamine (commercially available) | 572.2 | 0.0047 |
| 96 | 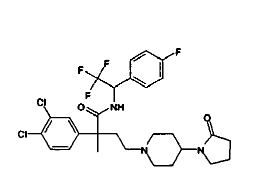 | 588.5 | 2-(3,4-Dichloro-phenyl)-2-methyl-4-[4-(2-oxo-pyrrolidin-1-yl)-piperidin-1-yl]-N-[2,2,2-trifluoro-1-(4-fluoro-phenyl)-ethyl]-butyramide | 2-(3,4-Dichloro-phenyl)-2-methyl-4-[4-(2-oxo-pyrrolidin-1-yl)-piperidin-1-yl]-butyric acid (intermediate 10) and 2,2,2-Trifluoro-1-(4-fluoro-phenyl) -ethylamine (commercially available) | 588.3 | 0.0072 |
| 97 | 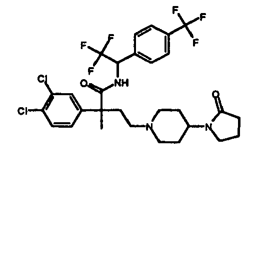 | 638.5 | 2-(3,4-Dichloro-phenyl)-2-methyl-4-[4-(2-oxo-pyrrolidin-1-yl)-piperidin-1-yl]-N-[2,2,2-trifluoro-1-(4-trifluoromethyl-phenyl)-ethyl]-butyramide | 2-(3,4-Dichloro-phenyl)-2-methyl-4-[4-(2-oxo-pyrrolidin-1-yl)-piperidin-1-yl]-butyric acid (intermediate 10) and 2,2,2-Trifluoro-1-(4-trifluoromethyl-phenyl)-ethylamine (commercially available) | 638.2 | 0.0081 |
| 98 | 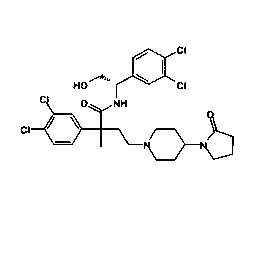 | 601.4 | 2-(3,4-Dichloro-phenyl)-N-[1-(3,4-dichloro-phenyl)-2-hydroxy-ethyl]-2-methyl-4-[4-(2-oxo-pyrrolidin-1-yl)-piperidin-1-yl]-butyramide; diastereoisomer 2 | 2-(3,4-Dichloro-phenyl)-2-methyl-4-[4-(2-oxo-pyrrolidin-1-yl)-piperidin-1-yl]-butyric acid (intermediate 10) and 2-Amino-2-(3,4-dichloro-phenyl)-ethanol (WO 2005058892) | 602.1 | 0.0499 |
| 99 | 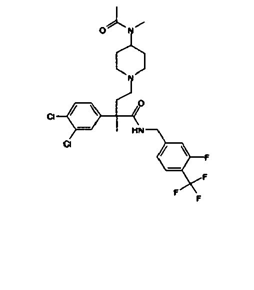 | 590.5 | 4-[4-(Acetyl-methyl-amino)-piperidin-1-yl]-2-(3,4-dichloro-phenyl)-N-(3-fluoro-4-trifluoromethyl-benzyl)-2-methyl-butyramide | 4-[4-(Acetylmethyl-amino)-piperidin-1-yl] -2-(3,4-dichloro-phenyl)-2-methyl-butyric acid (intermediate 11) and 3-Fluoro-4-trifluoromethyl-benzylamine (commercially available) | 590.3 | 0.0058 |
| 100 | 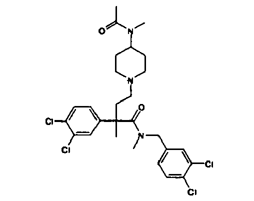 | 573.4 | 4-[4-(Acetyl-methyl-amino)-piperidin-1-yl]-N-(3,4-dichloro-benzyl)-2-(3,4-dichloro-phenyl)-2,N-dimethyl-butyramide | 4-[4-(Acetyl-methyl-amino)-piperidin-1-yl]-2-(3,4-dichloro-phenyl)-2-methyl-butyric acid (intermediate 11) and (3,4-Dichloro-benzyl)-methyl-amine (commercially available) | 574.2 | 0.003 |
| 101 | 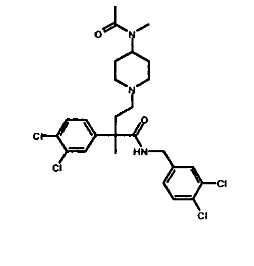 | 559.4 | 4-[4-(Acetyl-methyl-amino)-piperidin-1-yl]-N-(3,4-dichloro-benzyl)-2-(3,4-dichloro-phenyl)-2-methyl-butyramide | 4-[4-(Acetyl-methyl-amino)-piperidin-1-yl]-2-(3,4-dichloro-phenyl)-2-methyl-butyric acid (intermediate 11) and 3,4-Dichloro-benzylamine (commercially available) | 560.2 | 0.0068 |
| 102 | 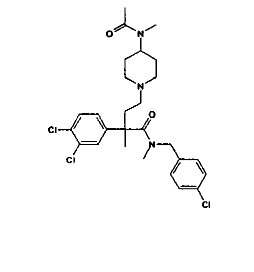 | 539.0 | 4-[4-(Acetyl-methyl-amino)-piperidin-1-yl]-N-(4-chloro-benzyl)-2-(3,4-dichloro-phenyl)2,N-dimethyl-butyramide | 4-[4-(Acetyl-methyl-amino)-piperidin-1-yl]-2-(3,4-dichloro-phenyl)-2-methyl-butyric acid (intermediate 11) and (4-Chloro-benzyl)-methyl-amine (commercially available) | 538.3 | 0.0051 |
| 103 | 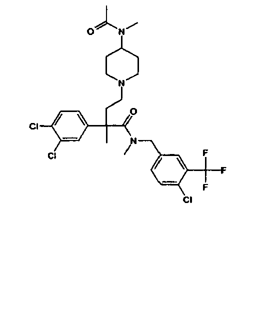 | 606.9 | 4-[4-(Acetyl-methyl-amino)-piperidin-1-yl]-N-(4-chloro-3-trifluoromethyl-benzyl)-2-(3,4-dichloro-phenyl)-2,N-dimethyl-butyramide | 4-[4-(Acetyl-methyl-amino)-piperidin-1-yl]-2-(3,4-dichloro-phenyl)-2-methyl-butyric acid (intermediate 11) and (4-Chloro-3-trifluoromethyl-benzyl) -methyl-amine (commercially available) | 608.2 | 0.0022 |
| 104 | 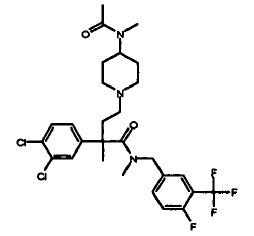 | 590.5 | 4-[4-(Acetyl-methyl-amino)-piperidin-1-yl]-2-(3,4-dichloro-phenyl)-N-(4-fluoro-3-trifluoromethyl-benzyl)-2,N-dimethyl-butyramide | 4-[4-(Acetyl-methyl-amino)-piperidin-1-yl]-2-(3,4-dichloro-phenyl)-2-methyl-butyric acid (intermediate 11) and (4-Fluoro-3-trifluoromethyl-benzyl)-methyl-amine, hyrdochloride (intermediate 13) | 590.3 | 0.0023 |
| 105 | 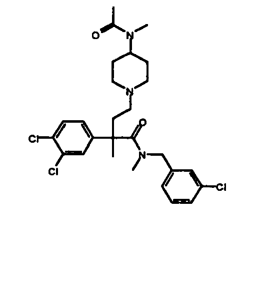 | 539.0 | 4-[4-(Acetyl-methyl-amino)-piperidin-1-yl]-N-(3-chloro-benzyl)-2-(3,4-dichloro-phenyl)-2,N-dimethyl-butyramide | 4-[4-(Acetyl-methyl-amino)-piperidin-1-yl] -2-(3,4-dichloro-phenyl)-2-methyl-butyric acid (intermediate 11) and (3-Chloro-benzyl)-methyl-amine (commercially available) | 538.3 | 0.0087 |
| 106 | 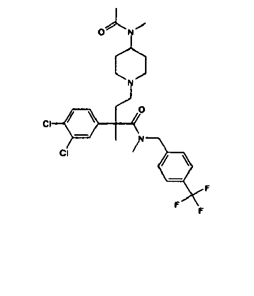 | 572.5 | 4-[4-(Acetyl-methyl-amino)-piperidin-1-yl]-2-(3,4-dicbloro-phenyl)-2,N-dimethyl-N-(4-trifluoromethyl-benzyl)-butyramide | 4-[4-(Acetyl-methyl-amino)-piperidin-1-yl]-2-(3,4-dichloro-phenyl)-2-methyl-butyric acid (intermediate 11) and Methyl-(4-trifluoromethyl-benzyl)-amine (commercially available) | 572.2 | 0.0051 |
| 107 | 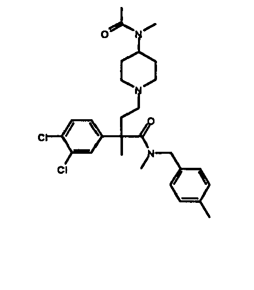 | 518.5 | 4-[4-(Acetyl-methyl-amino)-piperidin-1-yl]-2-(3,4-dichloro-phenyl)-2,N-dimethyl-N-(4-methyl-benzyl)-butyramide | 4-[4-(Acetyl-methyl-amino)-piperidin-1-yl]-2-(3,4-dichloro-phenyl)-2-methyl-butyric acid (intermediate 11) and Methyl-(4-methyl-benzyl)-amine (commercially available) | 518.4 | 0.0106 |
| 108 | 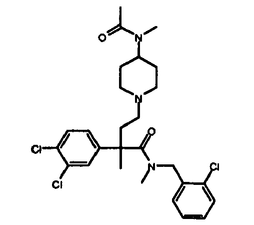 | 539.0 | 4-[4-(Acetyl-methyl-amino)-piperidin-1-yl]-N-(2-chloro-benzyl)-2-(3,4-dichloro-phenyl)-2,N-dimethyl-butyramide | 4-[4-(Acetyl-methyl-amino)-piperidin-1-yl]-2-(3,4-dichloro-phenyl)-2-methyl-butyric acid (intermediate 11) and (2-Chloro-benzyl)-methyl-amine (commercially available) | 538.5 | 0.012 |
| 109 | 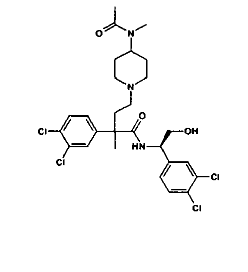 | 589.4 | 4-[4-(Acetyl-methyl-amino)-piperidin-1-yl]-2-(3,4-dichloro-phenyl)-N-[1-(3,4-dichloro-phenyl)-2-hydroxy-ethyl]-2-methyl-butyramide; diastereoisomer 1 | 4-[4-(Acetyl-methyl-amino)-piperidin-1-yl]-2-(3,4-dichloro-phenyl)-2-methyl-butyric acid (intermediate 11) and 2-Amino-2-(3,4-dichloro-phenyl)-ethanol (WO 2005058892) | 590.2 | 0.0056 |
| 110 | 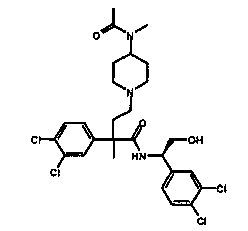 | 589.4 | 4-[4-(Acetyl-methyl-amino)-piperidin-1-yl]-2-(3,4-dichloro-phenyl)-N-[1-(3,4-dichloro-phenyl)-2-hydroxy-ethyl]-2-methyl-butyramide; diastereoisomer 2 | 4-[4-(Acetyl-methyl-amino)-piperidin-1-yl]-2-(3,4-dichloro-phenyl)-2-methyl-butyric acid (intermediate 11) and 2-Amino-2-(3,4-dichloro-phenyl)-ethanol (WO 2005058892) | 590.2 | 0.1103 |

### Example 111

### N-(4-Chloro-benzyl)-2-(4-fluoro-phenyl)-N-methyl-4-[4-(2-oxo-pyrrolidin-1-yl)-piperidin-1-yl]-butyranxide

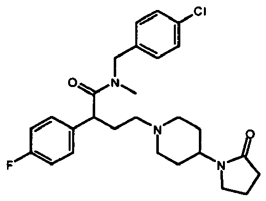

### a) step 1:

### N-(4-Chloro-benzyl)-2-(4-fluoro-phenyl)-N-methyl-acetamide

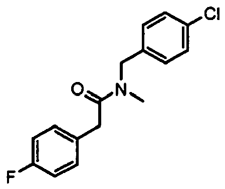

A mixture of 1.09 g (7 mmol) 4-fluorophenylacetic acid, 1.37 g (9 mmol) (4-chlorobenzyl)-methyl-amine, 2.84 g (9 mmol) TBTU and 6 mL (35 mmol) DIPEA in 50 mL DMF was stirred at room temperature for 16 h. After evaporation of all volatiles Na₂CO₃ aq. was added and the mixture was extracted with ethyl acetate. The combined organic phases were washed with NaCl aq., dried with Na₂SO₄, filtered and evaporated to dryness. The residue was purified by column chromatography on silica eluting with a gradient formed from ethyl acetate and heptane. The product containing fractions were evaporated to yield 1.35 g (65 %) of the title compound as yellow oil. MS(m/e): 292.2 (MH⁺).

### b) step 2:

### N-(4-Chloro-benzyl)-2-(4-fluoro-phenyl)-N-methyl-4-oxo-butyramide

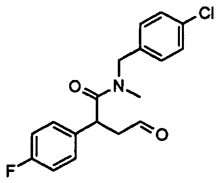

A solution of 1.10 g (3.78 mmol) N-(4-chloro-benzyl)-2-(4-fluoro-phenyl)-N-methylacetamide in 10 mL DMF at 0 °C was treated with 0.18 g (4 mmol) NaH (55% in oil) and allowed to stir at 40-50 °C for 3 h. After cooling to 0 °C the mixture was treated with 0.80 g (5 mmol) bromoaetaldehyde dimethylacetal in 10 mL DMF and the mixture was stirred at room temperature for 16 h. After evaporation of all volatiles the residue was treated with water and THF and 1N HCl aq. was added. The mixture was stirred at room temperature for 2 h and extracted with ethyl acetate. The combined organic phases were washed with NaCl aq. sat., dried with Na₂SO₄, filtered and evaporated. The residue was purified by column chromatography on silica eluting with a gradient formed from tert.-butylmethyl ether and heptane. The product containing fractions were evaporated to yield 0.90 g of the title compound which was used without further purification in the consecutive step. MS(m/e): 332.4 (MH⁺).

### c) step 3:

A mixture of 237 mg (0.71 mmol) N-(4-chloro-benzyl)-2-(4-fluoro-phenyl)-N-methyl-4-oxo-butyramide, 131 mg (0.77 mmol) 1-piperidin-4-yl-pyrrolidin-2-one, 0.226 (10.7 mmol) sodium triacetoxyborohydride and 64 mg acetic acid in 10 mL THF was stirred at room temperature for 17 h. Water and Na₂CO₃ aq. was added and the mixture was extracted with ethyl acetate. The combined organic phases were washed with NaCl aq. sat., dried with Na₂SO₄ filtered and evaporated. The residue was purified by column chromatography on silica eluting with a gradient formed from DCM, methanol and NH₃ aq.. The product containing fractions were evaporated to yield 277 mg (0.57 mmol) of the title compound as white foam. MS(m/e): 486.4 (MH⁺).

In analogy to the procedure described for the synthesis of N-(4-chloro-benzyl)-2-(4-fluoro-phenyl)-N-methyl-4- [4-(2-oxo-pyrrolidin-1-yl)-piperidin-1-yl]-butyramide (example 111) further piperidine derivatives have been synthesized from the starting materials listed in table 2. Table 2 comprises example 112 - 117.

**Table 2:**

| No | structure | MW | Systematic Name | starting materials | MW found (MH+) | hNK3 Ki (uM) |
|---|---|---|---|---|---|---|
| 111 | 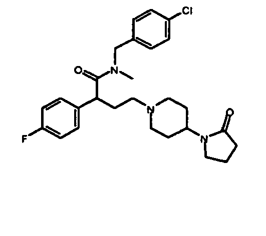 | 486.0 | N-(4-Chloro-benzyl)-2-(4-fluoro-phenyl)-N-methyl-4-[4-(2-oxo-pyrrolidin-1-yl)-piperidin-1-yl]-butyramide | 4-Fluorophenylacetic acid, (4-Chloro-benzyl)-methyl-amine, bromoaetaldehyde dimethylacetal and 1-Piperidin-4-yl-pyrrolidin-2-one | 486.4 | 0.4084 |
| 112 | 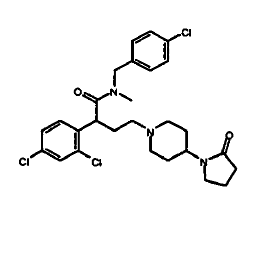 | 536.9 | N-(4-Chloro-benzyl)-2-(2,4-dichloro-phenyl)-N-methyl-4-[4-(2-oxo-pyrrolidin-1-yl)-piperidin-1-yl]-butyramide | 2,4-Dichlorophenylacetic acid, (4-Chloro-benzyl)-methyl-amine, bromoaetaldehyde dimethylacetal and 1-Piperidin-4-yl-pyrrolidin-2-one | 536.2 | 0.1281 |
| 113 | 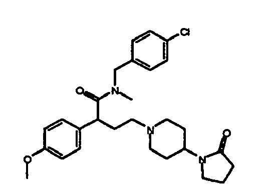 | 498.1 | N-(4-Chloro-benzyl)-2-(4-methoxy-phenyl)-N-methyl-4-[4-(2-oxo-pyrrolidin-1-yl)-piperidin-1-yl]-butyramide | 4-Methoxyphenylacetic acid, (4-Chloro-benzyl)-methyl-amine, bromoaetaldehyde dimethylacetal and 1-Piperidin-4-yl-pyrrolidin-2-one | 498.1 | 0.7748 |
| 114 | 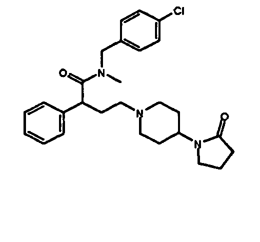 | 468.0 | N-(4-Chloro-benzyl)-N-methyl-4-[4-(2-oxo-pyrrolidin-1-yl)-piperidin-1-yl]-2-phenyl-butyramide | Phenylacetic acid, (4-Chloro-benzyl)-methyl-amine, bromoaetaldehyde dimethylacetal and 1-Piperidin-4-yl-pyrrolidin-2-one | 468.3 | 0.5603 |
| 115 | 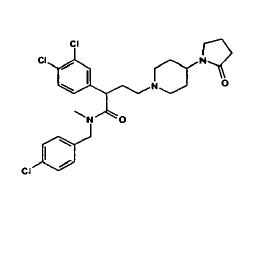 | 536.9 | N-(4-Chloro-benzyl)-2-(3,4-dichloro-phenyl)-N-methyl-4-[4-(2-oxo-pyrrolidin-1-yl)-piperidin-1-yl]-butyramide | 3,4-Dichlorophenylacetic acid, (4-Chloro-benzyl)-methyl-amine, bromoaetaldehyde dimethylacetal and 1-Piperidin-4-yl-pyrrolidin-2-one | 536.1 | 0.0081 |
| 116 | 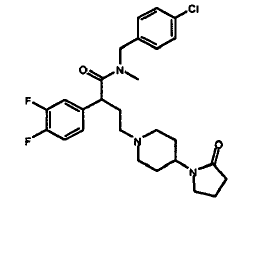 | 504.0 | N-(4-Chloro-benzyl)-2-(3,4-difluoro-phenyl)-N-methyl-4-[4-(2-oxo-pyrrolidin-1-yl)-piperidin-1-yl]-butyramide | 3,4-Difluorophenylacetic acid, (4-Chloro-benzyl)-methyl-amine, bromoaetaldehyde dimethylacetal and 1-Piperidin-4-yl-pyrrolidin-2-one | 504.2 | 0.1977 |
| 117 | 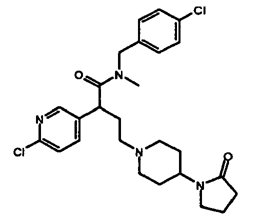 | 503.5 | N-(4-Chloro-benzyl)-2-(6-chloro-pyridin-3-yl)-N-methyl-4-[4-(2-oxo-pyrrolidin-1-yl)-piperidin-1-yl]-butyramide | (6-Chloro-pyridin-3-yl)-acetic acid, (4-Chloro-benzyl)-methyl-amine, bromoaetaldehyde dimethylacetal and 1-Piperidin-4-yl-pyrrolidin-2-one | 503.2 | 0.3872 |

Enantiomers were accessed from their respective starting materials (as listed in table 3) by column chromatography on appropriate chiral phase. Isolated compounds were optionally transferred into their respective salts by treatment with acid. Table 3 comprises example 118-124.

**Table 3.**

| No | structure | MW | Systematic Name | starting materials | MW found (MH+) | hNK3 Ki (uM) |
|---|---|---|---|---|---|---|
| 118 | 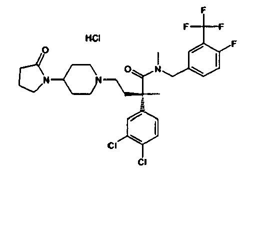 | 639.0 | (S or R)-2-(3,4-Dichloro-phenyl)-N-(4-fluoro-3-trifluoromethyl-benzyl)-2,N-dimethyl-4-[4-(2-oxo-pyrrolidin-1-yl)-piperidin-1-yl]-butyramide; hydrochloride | chiral separation from example 86 | 602.4 | 0.1709 |
| 119 | 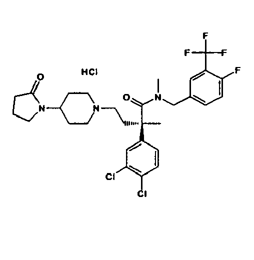 | 639.0 | (R or S)-2-(3,4-Dichloro-phenyl)-N-(4-Eluoro-3-trifluoromethyl-benzyl)-2,N-dimethyl-4-[4-(2-oxo-pyrrolidin-1-yl)-piperidin-1-yl]-butyramide; hydrochloride | chiral separation from example 86 | 602.4 | 0.0008 |
| 120 | 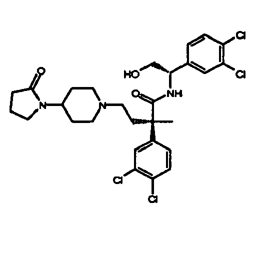 | 601.4 | (R or S)-2-(3,4-Dichloro-phenyl)-N-[(S or R)-1-(3,4-dichloro-phenyl)-2-hydroxy-ethyl] -2-methyl-4-[4-(2-oxo-pyrrolidin-1-yl)-piperidin-1-yl]-butyramide | chiral separation from example 94 | 602.4 | 0.002 |
| 121 | 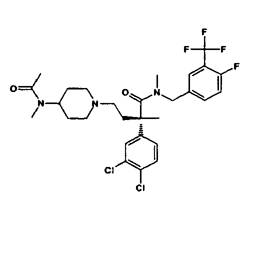 | 590.5 | (S or R)-4-[4-(Acetyl-methyl-amino)-piperidin-1-yl]-2-(3,4-cichloro-phenyl)-N-(4-fluoro-3-trifluoromethyl-benzyl)-2,N-dimethyl-butyramide | chiral separation from example 104 | 590.3 | 0.1735 |
| 122 | 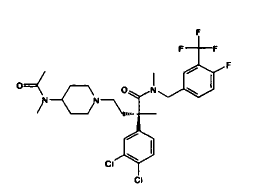 | 590.5 | (R or S)-4-[4-(Acetyl-methyl-amino)-piperidin-1-yl] -2-(3,4-dichloro-phenyl)-N-(4-fluoro-3-trifluoromethyl-benzyl)-2,N-dimethyl-butyramide | chiral separation from example 104 | 590.3 | 0.0015 |
| 123 | 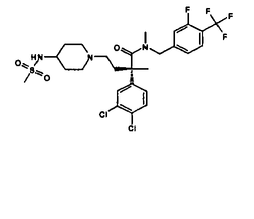 | 612.5 | (SorR)-2-(3,4-Dichloro-phenyl)-N-(3-fluoro-4-trifluoromethyl-benzyl)-4-(4-methanesulfonylamino -piperidin-1-yl)-2,N-dimethyl-butyramide | chiral separation from example 45 | 612.2 | 0.8004 |
| 124 | 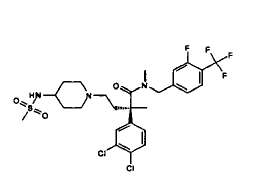 | 612.5 | (R or S)-2-(3,4-Dichloro-phenyl)-N-(3-fluoro-4-trifluoromethyl-benzyl)-4-(4-methanesulfonylamino -piperidin-1-yl)-2,N-dimethyl-butyramide | chiral separation from example 45 | 612.2 | 0.0188 |

### Example 125

### 2-(3,4-Dichloro-phenyl)-N-[1-(4-fluoro-3-trifluoromethyl-phenyl)-ethyl]-2-methyl-4-[4-(2-oxo-pyrrolidin-1-yl)-piperidin-1-yl]-butyramide

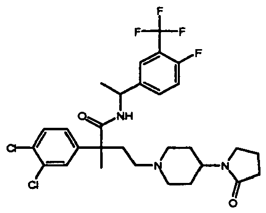

A mixture of 0.1 g (0.22 mmol) 2-(3,4-dichloro-phenyl)-2-methyl-4-[4-(2-oxopyrrolidin-1-yl)-piperidin-1-yl]-butyric acid (intermediate 10), 0.068 g (0.027 mmol) 1-(4-Fluoro-3-trifluoromethyl-phenyl)-ethylamine; hydrochloride (intermediate 20), 0.016 g (0.027 mmol) HATU and 0.23 mL DIPEA in 1.5 mL DMF was stirred at room temperature over night. The mixture was subjected to purification by reversed phase HPLC eluting with a gradient formed from acetonitrile, water and NEt3. the product containing fractions were evaporated to yield 0.079 g (59 %) of the title compound as light yellow viscous oil. MS(m/e): 601.9 (MH⁺).

In analogy to the procedure described for the synthesis of 2-(3,4-dichloro-phenyl)-*N-*[1-(4-fluoro-3-trifluoromethyl-phenyl)-ethyl -2-methyl-4- [4-(2-oxo-pyrrolidin-1-yl)-piperidin-1-yl]-butyramide (example 125) further piperidine derivatives have been prepared from the starting materials listed in table 4. Optionally diastereoisomeric / epimeric mixture have been separated by column chromatography. Table 4 comprises example 125- example 169.

**Table 4:**

| No | structure | MW | Systematic Name | starting materials | MW found (MH+) | hNK3 Ki (uM) |
|---|---|---|---|---|---|---|
| 125 | 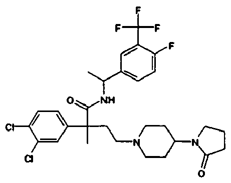 | 602.497 | 2-(3,4-Dichloro-phenyl)-*N*-[1-(4-fluoro-3-trifluoromethyl-phenyl)-ethyl]-2-methyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and 1-(4-Fluoro-3-trifluoromethyl-phenyl)-ethylamine; hydrochloride (intermediate 19) | 601.9 | 0.0254 |
| 126 | 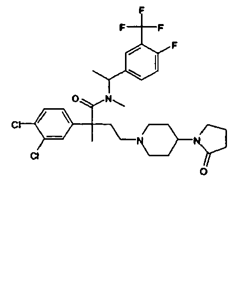 | 616.523 | 2-(3,4-Dichloro-phenyl)-*N*-[1-(4-fluoro-3-trifluoromethyl-phenyl)-ethyl]-2,*N*-dimethyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and 1-(4-Fluoro-3-trifluoromethyl-phenyl)-ethyl-methyl amine; hydrochloride (Intermediate 20) | 615.9 | 0.0303 |
| 127 | 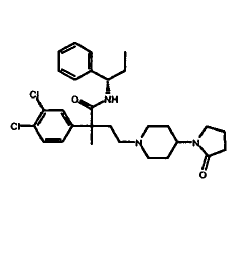 | 530.536 | 2-(3,4-Dichloro-phenyl)-2-methyl-4-[4-(2-oxo-pyrrolidin-1-yl)-piperidin-1-yl]-*N*-((S)-1-phenyl-propyl)-butyramide | 2-(3,4-Dichloro-phenyl)-2-methyl-4-[4-(2-oxo-pyrrolidin-1-yl)-piperidin-1-yl]-butyric acid (intermediate 10) and (S)-(-)-1-phenylpropylamine (commercially available) | 530.2 | 0.0052 |
| 128 | 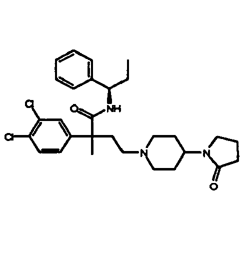 | 530.536 | 2-(3,4-Dichloro-phenyl)-2-methyl-4-[4-(2-oxo-pyrrolidin-1-yl)-piperidin-1-yl]-*N-*((R)-1-phenyl-propyl)-butyramide | 2-(3,4-Dichloro-phenyl)-2-methyl-4-[4-(2-oxo-pyrrolidin-1-yl)-piperidin-1-yl]-butyric acid (intermediate 10)and (R)-(+)-1-phenylpropylamine (commercially available) | 530.2 | 0.0902 |
| 129 | 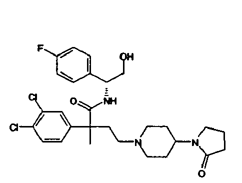 | 550.499 | 2-(3,4-Dichloro-phenyl)-*N*-[(R)-1-(4-fluoro-phenyl)-2-hydroxy-ethyl]-2-(R or S) methyl-4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl]-butyric acid (intermediate 10) and (R)-2-Amino-2-(4-fluoro-phenyl)-ethanol (commercially available) | 550.4 | 0.0082 |
| 130 | 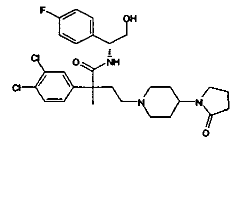 | 550.499 | 2-(3,4-Dichloro-phenyl)-*N*-[(R)-1-(4-fluoro-phenyl)-2-hydroxy-ethyl]-2-(S or R) methyl -4-[4-(2-oxo-pyrrolidin-1-yl)-piperidin-1-yl]-butyramide | 2-(3,4-Dichloro-phenyl)-2-methyl-4-[4-(2-oxo-pyrrolidin-1-yl)-piperidin-1-yl] -butyric acid (intermediate 10) | 550.4 | 0.1007 |
| 131 | 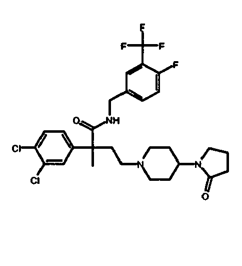 | 588.47 | 2-(3,4-Dichloro-phenyl)-*N*-(4-fluoro-3-trifluoromethyl-benzyl)-2-methyl-4-[4-(2-oxo-pyrrolidin-1-yl)-piperidin-1-yl] -butyramide | 2-(3,4-Dichloro-phenyl)-2-methyl-4-[4-(2-oxo-pyrrolidin-1-yl)-piperidin-1-yl]-butyric acid (intermediate 10) and 4-fluoro-3-(trifluoromethyl)ben zylamine (commercially available | 588.1 | 0.0034 |
| 132 | 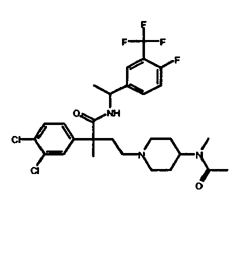 | 590.486 | 4-[4-(Acetyl-methyl-amino)-piperidin-1-yl]-2-(3,4-dichloro-phenyl)-*N*-[1-(4-fluoro-3-trifluoromethyl-phenyl)-ethyl]-2-methyl-butyramide | 4-[4-(Acetyl-methyl-amino)-piperidin-1-yl]-2-(3,4-dichloro-phenyl)-2-methyl-butyric acid (intermediate 11) and 1-(4-Fluoro-3-trifluoromethyl-phenyl)-ethylamine; hydrochloride (intermediate19) | 589.9 | 0.0462 |
| 133 | 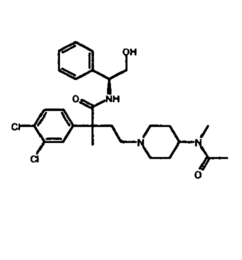 | 520.497 | 4-[4-(Acetyl-methyl-amino)-piperidin-1-yl]-2-(3,4-dichloro-phenyl)-*N*-((S)-2-hydroxy-1-phenyl-ethyl)-2-methyl-butyramide | 4-[4-(Acetyl-methyl-amino)-piperidin-1-yl]-2-(3,4-dichloro-phenyl)-2-methyl-butyric acid (intermediate 11) and (S)-(-)-1-phenylpropylamine (commercially available) | 520.2 | 0.5257 |
| 134 | 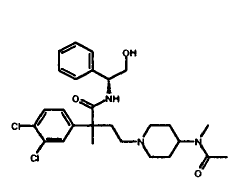 | 520.497 | 4-[4-(Acetyl-methyl-amino)-piperidin-1-yl]-2-(3,4-dichloro-phenyl)-*N*-((R)-2-hydroxy-1-phenyl-ethyl)-2-methyl-butyramide | 4-[4-(Acetyl-methyl-amino)-piperidin-1-yl]-2-(3,4-dichloro-phenyl)-2-methyl-butyric acid (intermediate 11) and (R)-(-)-1-phenylpropylamine (commercially available) | 520.3 | 0.0356 |
| 135 | 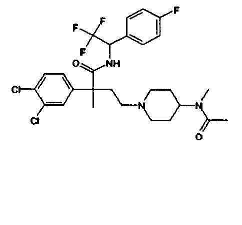 | 576.459 | 4-[4-(Acetyl-methyl-amino)-piperidin-1-yl]-2-(3,4-dichloro-phenyl)-2-methyl-*N*-[2,2,2-trifluoro-1-(4-fluoro-phenyl)-ethyl]-butyramide | 4-[4-(Acetyl-methyl-amino) -piperidin-1-yl]-2-(3,4-dichloro-phenyl)-2-methyl-butyric acid (intermediate 11) and 2,2,2-Trifluoro-1-(4-fluorophenyl)ethylam ine (commercially available) | 575.8 | 0.0126 |
| 136 | 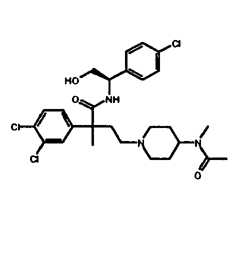 | 554.943 | 4-[4-(Acetyl-methyl-amino)-pipendin-1-yl]-*N*-[1-(4-chloro-phenyl)-2-hydroxy-ethyl]-2-(3,4-dichloro-phenyl)-2-methyl-butyramide (diastereosisomer 1) | 4-[4-(Acetyl-methyl-amino)-piperidin-1-yl]-2-(3,4-dichloro-phenyl)-2-methyl-butyric acid (intermediate 11) and 4-Chlorophenylglycinol (commercially available) | 553.7 | 0.0096 |
| 137 | 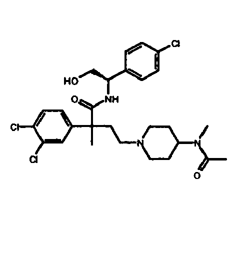 | 554.943 | 4-[4-(Acetyl-methyl-amino)-piperidin-1-yl]-*N*-[1-(4-chloro-phenyl)-2-hydroxy-ethyl]-2-(3,4-dichloro-phenyl)-2-methyl-butyramide (diastereosisomer 2) | 4-[4-(Acetyl-methyl-amino)-piperidin-1-yl]-2-(3,4-dichloro-phenyl)-2-methyl-butyric acid (intermediate 11) and 4-Chlorophenylglycinol (commercially available) | 553.7 | 0.056 |
| 138 | 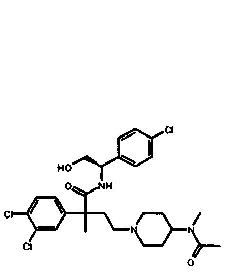 | 538.488 | 4-[4-(Acetyl-methyl-amino)-piperidin-1-yl]-2-(3,4-dichloro-phenyl)-*N*-[1-(4-fluoro-phenyl)-2-hydroxy-ethyl]-2-methyl-butyramide (diastereoisomer 1) | 4-[4-(Acetyl-methyl-amino)-piperidin-1-yl]-2-(3,4-dichloro-phenyl)-2-methyl-butyric acid (intermediate 11) and 4-Fluorophenylglycinol (commercially available) | 538 | 0.0293 |
| 139 | 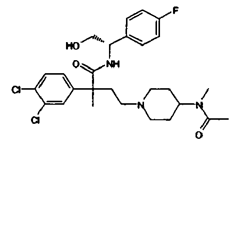 | 538.488 | 4-[4-(Acetyl-methyl-amino)-piperidin-1-yl]-2-(3,4-dichloro-phenyl)-*N*-[1-(4-fluoro-phenyl)-2-hydroxy-ethyl]-2-methyl-butyramide (diastereoisomer 2) | 4-[4-(Acetyl-methyl-amino)-piperidin-1-yl]-2-(3,4-dichloro-phenyl)-2-methyl-butyric acid (intermediate 11) and 4-Fluorophenylglycinol (commercially available) | 538 | 0.1415 |
| 140 | 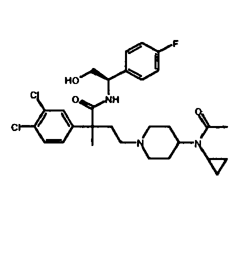 | 564.53 | 4-[4-(Acetyl-cyclopropyl-amino)-piperidin-1-yl]-2-(3,4-dichloro-phenyl)-*N*-[1-(4-fluoro-phenyl)-2-hydroxy-ethyl]-2-methyl-butyramide (diastereoisomer 1) | 4-[4-(Acetyl-cyclopropyl-amino)-piperidin-1-yl]-2-(3,4-dichloro-phenyl)-2-methyl-butyric acid (intermediate 14) and 4-Fluorophenylglycinol (commercially available) | 564.4 | 0.0333 |
| 141 | 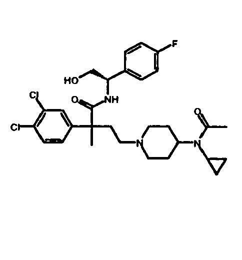 | 564.53 | 4-[4-(Acetyl-cyclopropyl-amino)-piperidin-1-yl]-2-(3,4-dichloro-phenyl)-*N*-[1-(4-fluoro-phenyl)-2-hydroxy-ethyl]-2-methyl-butyramide (diastereoisomer 2) | 4-[4-(Acetyl-cyclopropyl-amino)-piperid n-1-yl]-2-(3,4-dichloro-phenyl)-2-methyl-butyric acid (intermediate 14) and 4-Fluorophenylglycinol (commercially available) | 564.4 | 0.1648 |
| 142 | 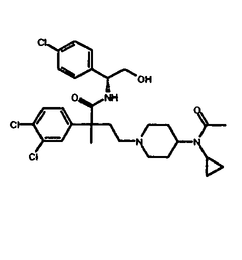 | 580.98 | 4-[4-(Acetyl-cyclopropyl-amino)-piperidin-1-yl]-*N*-[1-(4-chloro-phenyl)-2-hydroxy-ethyl] -2-(3,4-dichloro-phenyl)-2-methyl-butyramide (diastereoisomer 1) | 4-[4-(Acetyl-cycloprooyl-amino)-piperid n-1-yl]-2-(3,4-dichloro-phenyl)-2-methyl-butyric acid (intermediate 14) and 4-Chlorophenylglycinol (commercially available) | 580.4 | 0.0109 |
| 143 | 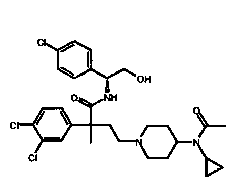 | 580.98 | 4-[4-(Acetyl-cyclopropyl-amino)-piperidin-1-yl]-*N*-[1-(4-chloro-phenyl)-2-hydroxy-ethyl]-2-(3,4-dichloro-phenyl)-2-methyl-butyramide (diastereoisomer 2) | 4-[4-(Acetyl-cyclopropyl-amino)-piperidin-1-yl]-2-(3,4-dichloro-phenyl)-2-methyl-butyric acid (intermediate 14) and 4-Chlorophenylglycinol (commercially available) | 580.4 | 0.0877 |
| 144 | 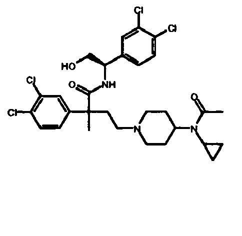 | 615.43 | 4-[4-(Acetyl-cyclopropyl-amino)-piperidin-1-yl]-2-(3,4-dichloro-phenyl)-*N*-[1-(3,4-dichloro-phenyl)-2-hydroxy-ethyl] -2-methyl-butyramide (diastereosiomer 1) | 4-[4-(Acetyl-cyclopropyl-amino)-piperidin-1-yl]-2-(3,4-dichloro-phenyl)-2-methyl-butyric acid (intermediate 14) and 3,4-di-Chlorophenylglycinol (commercially available) | 616.2 | 0.0101 |
| 145 | 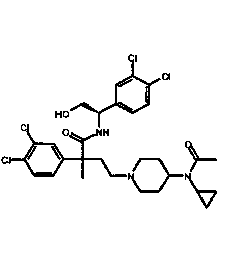 | 615.43 | 4-[4-(Acetyl-cyclopropyl-amino)-piperidin-1-yl]-2-(3,4-dichloro-phenyl)-*N-*[1-(3,4-dichloro-phenyl)-2-hydroxy-ethyl]-2-methyl-butyramide (diastereoisomer 2) | 4-[4-(Acetyl-cyclopropyl-amino) -piperidin-1-yl]-2-(3,4-dichloro-phenyl)-2-methyl-butyric acid (intermediate 14) and 3,4-di-Chlorophenylglycinol (commercially available) | 616.2 | 0.0502 |
| 146 | 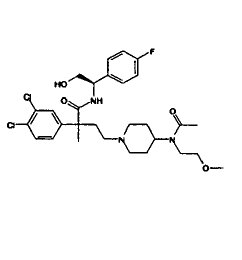 | 582.54 | 4-{4-[Acetyl-(2-methoxy-ethyl)-amino]-piperidin-1-yl}-2-(3,4-dichloro-phenyl)-*N*-[1-(4-fluoro-phenyl)-2-hydroxy-ethyl]-2-methyl-butyramide (diastereoisomer 1) | 4-{4-[Acetyl-(2-methoxy-ethyl)-amino] piperidin-1-yl}-2-(3,4-dichloro-phenyl)-2-methyl-butyric acid (intermediate 15) and 4-Fluorophenylglycinol (commercially available) | 582.2 | 0.0007 |
| 147 | 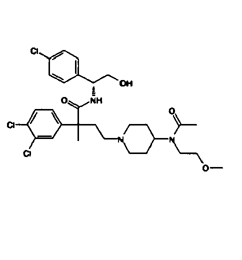 | 598.995 | 4-{4-[Acetyl-(2-methoxy-ethyl)-amino]-piperidin-1-yl}-*N*-[1-(4-chloro-phenyl)-2-hydroxy-ethyl]-2-(3,4-dichloro-phenyl)-2-methyl-butyramide | 4-{4-[Acetyl-(2-methoxy-ethyl)-amino]-piperidin-1-yl}-2-(3,4-dichloro-phenyl)-2-methyl-butyric acid (intermediate 15) and 4-Chlorophenylglycinol (commercially available) | 598.3 | 0.3824 |
| 148 | 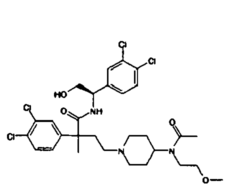 | 633.44 | 4-{4-[Acetyl-(2-methoxy-ethyl)-amino]-piperidin-1-yl}-2-(3,4-dichloro-phenyl)-*N*-[1-(3,4-dichloro-phenyl)-2-hydroxy-ethyl]-2-methyl-butyramide (diastreoisomer 1) | 4-{4-[Acetyl-(2-methoxy-ethyl)-amino] -piperidin-1 -yl}-2-(3,4-dichloro-phenyl)-2-methyl-butyric acid (intermediate 15) and 3,4-di-Chlorophenylglycinol (commercially available) | 634.2 | 0.0038 |
| 149 | 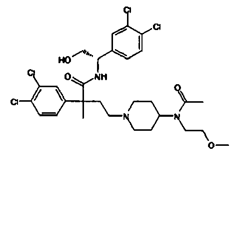 | 633.44 | 4-{4-[Acetyl-(2-methoxy-ethyl)-amino] -piperidin-1-yl}-2-(3,4-dichloro-phenyl)-*N*-[(S)-1-(3,4-dichloro-phenyl)-2-hydroxy-ethyl]-2-methyl-butyramide (diastreoisomer 2) | 4-{4-[Acetyl-(2-methoxy-ethyl)-amino]-piperidin-1-yl}-2-(3,4-dichloro-phenyl)-2-methyl-butyric acid (intermediate 15) and 3,4-di-Chlorophenylglycinol (commercially available) | 634.3 | 0.0194 |
| 150 | 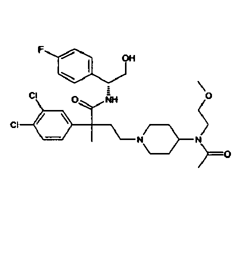 | 582.54 | 4-{4-[Acetyl-(2-methoxy-ethyl)-amino]-piperidin-1-yl}-2-(3,4-dichloro-phenyl)-*N*-[(R)-1-(4-fluoro-phenyl)-2-hydroxy-ethyl]-2-(R or S) methyl-butyramide | 4-{4-[Acetyl-(2-methoxy-ethyl)-amino] -piperidine-1 -yl}-2-(3,4-dichloro-phenyl)-2-methyl-butyric acid (intermediate 15) and (R)-4-Fluorophenylglycinol (commercially available) | 582.2 | 0.0017 |
| 151 | 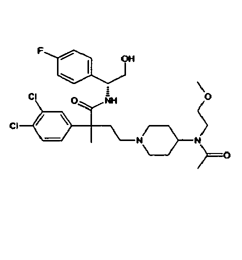 | 582.54 | 4-{4-[Acetyl-(2-methoxy-ethyl)-amino]-piperidin-1-yl}-2-(3,4-dichloro-phenyl)-*N*-[(R)-1-(4-fluoro-phenyl)-2-hydroxy-ethyl]-2-(S or R) methyl-butyramide | 4-{4-[Acetyl-(2-methoxy-ethyl)-amino] -piperidin-1 -yl}-2-(3,4-dichloro-phenyl)-2-methyl-butyric acid (intermediate 15) and (R)-4-Fluorophenylglycinol (commercially available) | 582.2 | 0.0211 |
| 152 | 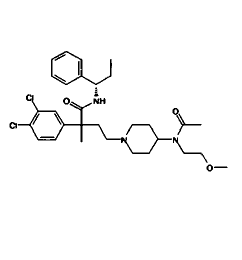 | 562.578 | 4-{4-[Acetyl-(2-methoxy-ethyl)-amino]-piperidin-1-yl}-2-(3,4-dichloro-phenyl)-2-methyl-*N*-((S)-1-phenyl-propyl)-butyramide | 4-{4-[Acetyl-(2-methoxy-ethyl)-amino]-piperidin-1-yl}-2-(3,4-dichloro-phenyl)-2-methyl-butyric acid (intermediate 15) and (S)-(-)-1-phenylpropylamine (commercially available) | 562.2 | 0.0011 |
| 153 | 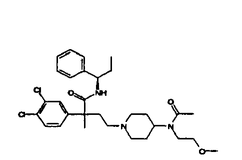 | 562.578 | 4-{4-[Acetyl-(2-methoxy-ethyl)-amino]-piperidin-1-yl}-2-(3,4-dichloro-phenyl)-2-methyl-*N*-((R)-1-phenyl-propyl)-butyramide | 4-{4-[Acetyl-(2-methoxy-ethyl)-amino] -piperidine-1 -yl}-2-(3,4-dichloro-phenyl)-2-methyl-butyric acid (intermediate 15) and (R)-(+)-1-phenylpropylamine (commercially available) | 562.2 | 0.0609 |
| 154 | 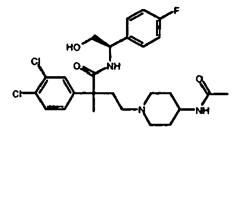 | 524.46 | 4-(4-Acetylamino-piperidin-1-yl)-2-(3,4-dichloro-phenyl)-*N*-[1-(4-fluoro-phenyl)-2-hydroxy-ethyl]-2-methyl-butyramide (diastereoisomer 1) | 4-(4-Acetylamino-piperidin-1-yl)-2-(3,4-dichloro-phenyl)-2-methylbutyric acid (intermediate 16) and 4-Fluorophenylglycinol (commercially available) | 524.3 | 0.1703 |
| 155 | 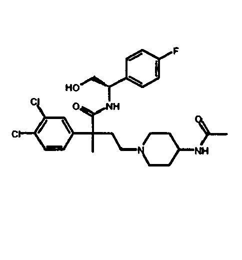 | 524.46 | 4-(4-Acetylamino-piperidin-1-yl)-2-(3,4-dichloro-phenyl)-*N*-[1-(4-fluoro-phenyl)-2-hydroxy-ethyl]-2-methyl-butyramide (diastereoisomer 2) | 4-(4-Acetylamino-piperidin-1-yl)-2-(3,4-dichloro-phenyl)-2-methylbutyric acid (intermediate 16) and 4-Fluorophenylglycinol (commercially available) | 524.3 | 0.0138 |
| 156 | 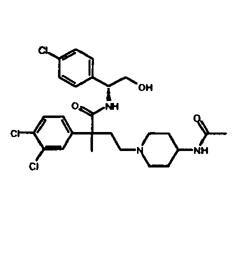 | 540.916 | 4-(4-Acetylamino-piperidin-1-yl)-*N*-[1-(4-chloro-phenyl)-2-hydroxy-ethyl]-2-(3,4-dichloro-phenyl)-2-methyl-butyramide (diastereoisomer 1) | 4-(4-Acetylamino-piperidin-1-yl)-2-(3,4-dichloro-phenyl)-2-methylbutyric acid (intermediate 16) and 4-Chlorophenylglycinol (commercially available) | 540.3 | 0.0505 |
| 157 | 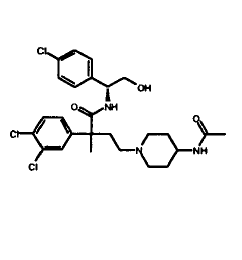 | 540.916 | 4-(4-Acetylamino-piperidin-1-yl)-*N*-[1-(4-chloro-phenyl)-2-hydroxy-ethyl]-2-(3,4-dichloro-phenyl)-2-methyl-butyramide (diastereoisomer 2) | 4-(4-Acetylamino-piperidin-1-yl)-2-(3,4-dichloro-phenyl)-2-methylbutyric acid (intermediate 16) and 4-Chlorophenylglycinol (commercially available) | 540.3 | 0.7727 |
| 158 | 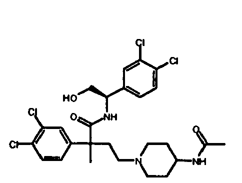 | 575.36 | 4-(4-Acetylamino-piperidin-1-yl)-2-(3,4-dichloro-phenyl)-*N*-[(R)-1-(3,4-dichloro-phenyl)-2-hydroxy-ethyl] -2-methyl-butyramide (diastereoisomer 1) | 4-(4-Acetylamino-piperidin-1-yl)-2-(3,4-dichloro-phenyl)-2-methylbutyric acid (intermediate 16) and 3,4-di-Chlorophenylglycinol (commercially available) | 576.3 | 0.0346 |
| 159 | 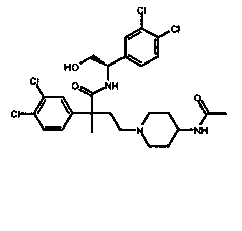 | 575.36 | 4-(4-Acetylamino-piperidin-1-yl)-2-(3,4-dichloro-phenyl)-*N*-[(S)-1-(3,4-dichloro-phenyl)-2-hydroxy-ethyl] -2-methyl-butyramide (diastereoisomer 2) | 4-(4-Acetylamino-piperidin-1-yl)-2-(3,4-dichloro-phenyl)-2-methylbutyric acid (intermediate 16) and 3,4-di-Chlorophenylglycinol (commercially available) | 576.3 | 0.2582 |
| 160 | 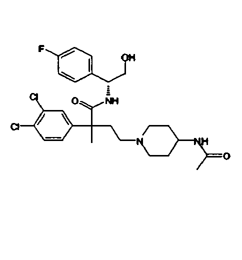 | 524.461 | 4-(4-Acetylamino-piperidin-1-yl)-2-(3,4-dichloro-phenyl)-*N*-[(R)-1-(4-fluoro-phenyl)-2-hydroxy-ethyl] -2-methyl-butyramide | 4-(4-Acetylamino-piperidin-1-yl)-2-(3,4-dichloro-phenyl)-2-methylbutyric acid (intermediate 16) and (R)-4-Fluorophenylglycinol (commercially available) | 524.4 | 0.1998 |
| 161 | 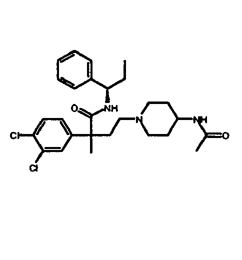 | 504.498 | 4-(4-Acetylamino-piperidin-1-yl)-2-(3,4-dichloro-phenyl)-2-methyl-*N*-((S)-1-phenyl-propyl)-butyramide | 4-(4-Acetylamino-piperidin-1-yl)-2-(3,4-dichloro-phenyl)-2-methylbutyric acid (intermediate 16) and (S)-(-)-1-phenylpropylamine (commercially available) | 504.2 | 0.0442 |
| 162 | 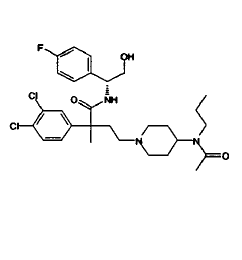 | 566.541 | 4-[4-(Acetyl-propyl-amino)-piperidin-1-yl]-2-(3,4-dichloro-phenyl)-*N*-[(R)-1-(4-fluoro-phenyl)-2-hydroxy-ethyl] -2-methyl-butyramide (epimer 1) | 4-[4-(Acetyl-propyl-amino)-piperidin-1-yl]-2-(3,4-dichloro-phenyl)-2-methyl-butyric acid (intermediate 17) and (R)-4-Fluorophenylglycinol (commercially available) | 566.4 | 0.0005 |
| 163 | 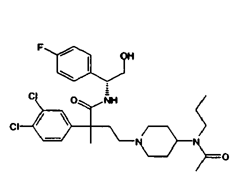 | 566.541 | 4-[4-(Acetyl-propyl-amino)-piperidin-1-yl]-2-(3,4-dichloro-phenyl)-*N*-[(R)-1-(4-fluoro-phenyl)-2-hydroxy-ethyl] -2-methyl-butyramide (epimer 2) | 4-[4-(Acetyl-propyl-amino)-piperidin-1-yl]-2-(3,4-dichloro-phenyl)-2-methyl-butyric acid (intermediate 17) and (R)-4-Fluorophenylglycinol (commercially available) | 566.4 | 0.0056 |
| 164 | 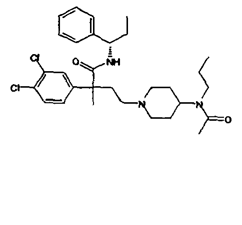 | 546.579 | 4-[4-(Acetyl-propyl-amino)-piperidin-1-yl]-2-(3,4-dichloro-phenyl)-2-methyl-*N*-((S)-1-phenyl-propyl)-butyramide | 4-{4-(Acetyl-propyl-amino)-piperidin-1-yl]-2-(3,4-dichloro-phenyl)-2-methyl-butyric acid (intermediate 17) and (S)-(-)-1-phenylpropylamine (commercially available) | 546.2 | 0.0008 |
| 165 | 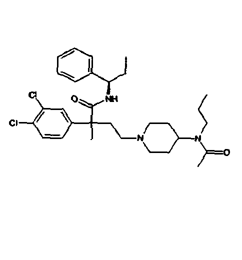 | 546.579 | 4-[4-(Acetyl-propyl-amino)-piperidin-1-yl]-2-(3,4-dichloro-phenyl)-2-methyl-*N*-((R)-1-phenyl-propyl)-butyramide | 4-[4-(Acetyl-propyl-amino)-piperidin-1-yl]-2-(3,4-dichloro-phenyl)-2-methyl-butyric acid (intermediate 17) and (R)-(+)-1-phenylpropylamine (commercially available) | 546.2 | 0.0145 |
| 166 | 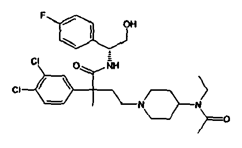 | 552.514 | 4-[4-(Acetyl-ethyl-amino)-piperidin-1-yl]-2-(3,4-dichloro-phenyl)-*N*-[(R)-1-(4-fluoro-phenyl)-2-hydroxy-ethyl]-2-methyl-butyramide (epimer 1) | 4-[4-(Acetyl-ethyl-amino)-piperidin-1-yl]-2-(3,4-dichloro-phenyl)-2-methyl-butyric acid (intermediate 18) and (R)-4-Fluorophenylglycinol . (commercially available) | 552.4 | 0.0022 |
| 167 | 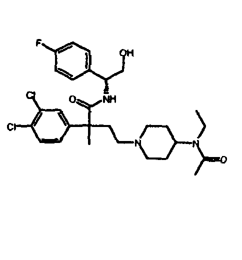 | 552.514 | 4-[4-(Acetyl-ethyl-amino)-piperidin-1-yl]-2-(3,4-dichloro-phenyl)-*N*-[(R)-1-(4-Quoro-phenyl)-2-hydroxy-ethyl]-2-methyl-butyramide (epimer 2) | 4-[4-(Acetyl-ethyl-amino)-piperidin-1-yl]-2-(3,4-dichloro-phenyl)-2-methyl-butyric acid (intermediate 18) and (R)-4-Fluorophenylglycinol (commercially available) | 552.4 | 0.0349 |
| 168 | 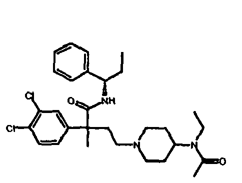 | 532.552 | 4-[4-(Acetyl-ethyl-amino)-piperidin-1-yl]-2-(3,4-dichloro-phenyl)-2-methyl-*N*-((S)-1-phenyl-propyl)-butyramide | 4-[4-(Acetyl-ethyl-amino)-piperidin-1-yl]-2-(3,4-dichloro-phenyl)-2-methyl-butyric acid (intermediate 18) and (S)-(-)-1-phenylpropylamine (commercially available) | 532.2 | 0.003 |
| 169 | 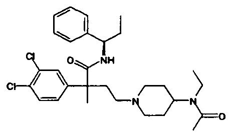 | 532.552 | 4-[4-(Acetyl-ethyl-amino)-piperidin-1-yl]-2-(3,4-dichloro-phenyl)-2-methyl-*N*-((R)-1-phenyl-propyl)-butyramide | 4-[4-(Acetyl-ethyl-amino)-piperidine-1-yl]-2-(3,4-dichloro-phenyl)-2-methyl-butyric acid (intermediate 18) and (R)-(+)-1-phenylpropylamine (commercially available) | 532.3 | 0.0576 |

The salt formation is effected at room temperature in accordance with methods which are known per se and which are familiar to any person skilled in the art. Not only salts with inorganic acids, but also salts with organic acids come into consideration. Hydrochlorides, hydrobromides, sulphates, nitrates, citrates, acetates, maleates, succinates, methan-sulphonates, p-toluenesulphonates and the like are examples of such salts.

As mentioned earlier, the compounds of formula I and their pharmaceutically usable addition salts possess valuable pharmacological properties. It has been found that the compounds of the present invention are antagonists of neurokinin 3 (NK-3) receptors. The compounds were investigated in accordance with the tests given hereinafter.

### [³H]SR142801 competition binding assay

hNK3 receptor binding experiment were performed using [³H]SR142801 (Catalog No. TRK1035, specific activity: 74.0 Ci/mmol, Amersham, GE Healthcare UK limited, Buckinghamshire, UK) and membrane isolated from HEK293 cells transiently expressing recombinant human NK3 receptor. After thawing, the membrane homogenates were centrifuged at 48,000 X g for 10 min at 4 °C, the pellets were resuspended in the 50 mM Tris-HCl, 4 mM MnCl₂, 1 µM phosphoramidon, 0.1 % BSA binding buffer at pH 7.4 to a final assay concentration of 5 µg protein/well. For inhibition experiments, membranes were incubated with [³H]SR142801 at a concentration equal to K_{D} value of radioligand and 10 concentrations of the inhibitory compound (0.0003-10 µM) (in a total reaction volume of 500 µl) for 75 min at room temperature (RT). At the end of the incubation, membranes were filtered onto unitfilter (96-well white microplate with bonded GF/C filter preincubated 1 h in 0.3% PEI + 0.3% BSA, Packard BioScience, Meriden, CT) with a Filtermate 196 harvester (Packard BioScience) and washed 4 times with ice-cold 50 mM Tris-HCl, pH 7.4 buffer. Nonspecific binding was measured in the presence of 10 µM SB222200 for both radioligands. The radioactivity on the filter was counted (5 min) on a Packard Top-count microplate scintillation counter with quenching correction after addition of 45 µl of microscint 40 (Canberra Packard S.A., Zürich, Switzerland) and shaking for 1 h. Inhibition curves were fitted according to the Hill equation: y = 100/(1+(x/IC₅₀)^{nH}), where n_{H} = slope factor using Excel-fit 4 software (Microsoft). IC₅₀ values were derived from the inhibition curve and the affinity constant (Kᵢ) values were calculated using the Cheng-Prussoff equation Kᵢ = IC₅₀/(1+[L]/K_{D}) where [L] is the concentration of radioligand and K_{D} is its dissociation constant at the receptor, derived from the saturation isotherm. All experiments were performed in duplicate and the mean ± standard error (SEM) of the individual Kᵢ values was calculated.

Some results of preferred compounds of the hNK-3 receptor affinity are shown in the following Table:

| **Example No.** | **Kᵢ NK3 h (µM)** | **Example No.** | **Kᵢ NK3 h (µM)** |
|---|---|---|---|
| 45 | 0.0085 | 94 | 0.003 |
| 46 | 0.0024 | 95 | 0.0047 |
| 47 | 0.0021 | 96 | 0.0072 |
| 48 | 0.0036 | 99 | 0.0058 |
| 49 | 0.0055 | 100 | 0.003 |
| 57 | 0.0059 | 101 | 0.0068 |
| 58 | 0.0036 | 102 | 0.0051 |
| 59 | 0.0043 | 103 | 0.0022 |
| 60 | 0.0026 | 104 | 0.0023 |
| 69 | 0.0024 | 105 | 0.0087 |
| 70 | 0.0012 | 106 | 0.0051 |
| 71 | 0.0028 | 109 | 0.0056 |
| 72 | 0.0022 | 115 | 0.0081 |
| 73 | 0.0015 | 119 | 0.0008 |
| 74 | 0.0013 | 120 | 0.002 |
| 75 | 0.0039 | 122 | 0.0015 |
| 76 | 0.0017 | 127 | 0.0052 |
| 77 | 0.0031 | 129 | 0.0082 |
| 78 | 0.0036 | 131 | 0.0034 |
| 79 | 0.0015 | 136 | 0.0096 |
| 81 | 0.003 | 146 | 0.0007 |
| 82 | 0.0012 | 148 | 0.0038 |
| 83 | 0.0023 | 150 | 0.0017 |
| 84 | 0.0021 | 152 | 0.0011 |
| 85 | 0.0014 | 162 | 0.0005 |
| 86 | 0.0014 | 163 | 0.0056 |
| 87 | 0.0046 | 164 | 0.0008 |
| 88 | 0.0018 | 166 | 0.0022 |
| 89 | 0.0053 | 168 | 0.003 |
| 90 | 0.0025 | | |

The compounds of formula I as well as their pharmaceutically usable acid addition salts can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The compounds of formula I and their pharmaceutically usable acid addition salts can be processed with pharmaceutically inert, inorganic or organic excipients for the production of tablets, coated tablets, dragees and hard gelatine capsules. Lactose, corn starch or derivatives thereof, talc, stearic acid or its salts etc can be used as such excipients e.g. for tablets, dragées and hard gelatine capsules.

Suitable excipients for soft gelatine capsules are e.g. vegetable oils, waxes, fats, semisolid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semi-liquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary within wide limits and will, of course, be fitted to the individual requirements in each particular case. In general, in the case of oral administration a daily dosage of about 10 to 1000 mg per person of a compound of general formula I should be appropriate, although the above upper limit can also be exceeded when necessary.

### Example A

Tablets of the following composition are manufactured in the usual manner:

| | mg / tablet |
|---|---|
| Active substance | 5 |
| Lactose | 45 |
| Corn starch | 15 |
| Microcrystalline cellulose | 34 |
| Magnesium stearate | 1 |
| | Tablet weight 100 |

### Example B

Capsules of the following composition are manufactured:

| | mg / capsule |
|---|---|
| Active substance | 10 |
| Lactose | 155 |
| Corn starch | 30 |
| Talc | 5 |
| Capsule fill weight | 200 |

The active substance, lactose and corn starch are firstly mixed in a mixer and then in a comminuting machine. The mixture is returned to the mixer, the talc is added thereto and mixed thoroughly. The mixture is filled by machine into hard gelatine capsules.

### Example C

Suppositories of the following composition are manufactured:

| | mg / supp. |
|---|---|
| Active substance | 15 |
| Suppository mass | 1285 |
| Total | 1300 |

The suppository mass is melted in a glass or steel vessel, mixed thoroughly and cooled to 45 °C. Thereupon, the finely powdered active substance is added thereto and stirred until it has dispersed completely. The mixture is poured into suppository moulds of suitable size, left to cool, the suppositories are then removed from the moulds and packed individually in wax paper or metal foil.

## Claims

1. A compound of formula 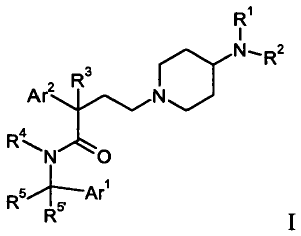 wherein
Ar¹/Ar² are independently from each other phenyl or pyridinyl, which are optionally substituted by one or two substituents, selected from the group consisting of halogen, lower alkyl, lower alkoxy, lower alkyl substituted by halogen, lower alkoxy substituted by halogen, lower alkyl substituted by alkoxy, lower alkyl substituted by cyano, lower di-alkyl amino, pyridinyl or cyano;
R¹ is hydrogen, lower alkyl, -(CH₂)₂O-lower alkyl, or cycloalkyl:
R² is -S(O)₂-lower alkyl or -C(O)-lower alkyl;
or R¹ and R² form together with the N-atom to which they are attached a pyrrolidin-2-one or a piperidin-2-one group;
R³ is hydrogen, halogen or lower alkyl;
R⁴ is hydrogen or lower alkyl;
R⁵/R^{5'} are independently from each other hydrogen, lower alkyl, lower alkyl substituted by halogen, lower alkyl substituted by hydroxy or cycloalkyl;
or R⁵ and R^{5'} form together with the carbon-atom to which they are attached a cycloalkyl group;
or a pharmaceutically active salt thereof.

2. A compound of formula I according to claim 1, wherein Ar¹ and Ar² are both phenyl.

3. A compound of formula I according to claim 2, wherein R¹ is methyl and R² is S(O)₂CH₃.

4. A compound of formula I according to claim 3, which compounds are
2-(3,4-dichloro-phenyl)-N-(3-fluoro-4-trifluoromethyl-benzyl)-4-[4-(methanesulfonyl-methyl-amino)-piperidin-1-yl]-2,N-dimethyl-butyramide
N-(4-chloro-3-trifluoromethyl-benzyl)-2-(3,4-dichloro-phenyl)-4-[4-(methanesulfonyl-methyl-amino)-piperidin-1-yl]-2,N-dimethyl-butyramide
2-(3,4-dichloro-phenyl)-N-(4-fluoro-3-trifluoromethyl-benzyl)-4-[4-(methanesulfonyl-methyl-amino)-piperidin-1-yl]-2,N-dimethyl-butyramide
N-(3,4-dichloro-benzyl)-2-(3,4-dichloro-phenyl)-4-[4-(methanesulfonyl-methyl-amino) -piperidin-1-yl] -2,N-dimethyl-butyramide or
2-(3,4-dichloro-phenyl)-N- [1-(3,4-dichloro-phenyl)-2-hydroxy-ethyl]-4-[4-(methanesulfonyl-methyl-amino)-piperidin-1-yl]-2-methyl-butyramide; diastereoisomer 1.

5. A compound of formula I according to claim 2, wherein R¹ is cyclopropyl and R² is S(O)₂CH₃.

6. A compound of formula I according to claim 5, which compounds are
4-[4-(cyclopropyl-methanesulfonyl-amino)-piperidin-1-yl]-2-(3,4-dichloro-phenyl)-N-(3-fluoro-4-trifluoromethyl-benzyl)-2,N-dimethyl-butyramide
N-(4-chloro-3-trifluoromethyl-benzyl)-4-[4-(cyclopropyl-methanesulfonyl-amino)-piperidin-1-yl]-2-(3,4-dichloro-phenyl)-2,N-dimethyl-butyramide
4-[4-(cyclopropyl-methanesulfonyl-amino)-piperidin-1-yl]-2-(3,4-dichloro-phenyl)-N-(4-fluoro-3-trifluoromethyl-benzyl)-2,N-dimethyl-butyramide or
4-[4-(cyclopropyl-methanesulfonyl-amino)-piperidin-1-yl]-N-(3,4-dichloro-benzyl)-2-(3,4-dichloro-phenyl)-2,N-dimethyl-butyramide.

7. A compound of formula I according to claim 2, wherein R¹ is ethyl and R² is S(O)₂CH₃.

8. A compound of formula I according to claim 7, which compounds are
2-(3,4-dichloro-phenyl)-4-[4-(ethyl-methanesulfonyl-amino)-piperidin-1-yl]-N-(3-fluoro-4-trifluoromethyl-benzyl)-2,N-dimethyl-butyramide
N-(3,4-dichloro-benzyl)-2-(3,4-dichloro-phenyl)-4-[4-(ethyl-methanesulfonyl-amino)-piperidin-1-yl]-2,N-dimethyl-butyramide
N-(3,4-dichloro-benzyl)-2-(3,4-dichloro-phenyl)-4-[4-(ethyl-methanesulfonyl-amino)-piperidin-1-yl-2-methyl-butyramide
N-(4-chloro-benzyl)-2-(3,4-dichloro-phenyl)-4-[4-(ethyl-methanesulfonyl-amino)-piperidin-1-yl]-2,N-dimethyl-butyramide
N-(4-chloro-3-trifluoromethyl-benzyl)-2-(3,4-dichloro-phenyl)-4-[4-(ethyl-methanesulfonyl-amino)-piperidin-1-yl]-2,N-dimethyl-butyramide
2-(3,4-dichloro-phenyl)-4-[4-(ethyl-methanesulfonyl-amino)-piperidin-1-yl]-N-(4-fluoro-3-trifluoromethyl-benzyl)-2,N-dimethyl-butyramide
N-(3-chloro-benzyl)-2-(3,4-dichloro-phenyl)-4-[4-(ethyl-methanesulfonyl-amino)-piperidin-1-yl] -2,N-dimethyl-butyramide
2-(3,4-dichloro-phenyl)-4-[4-(ethyl-methanesulfonyl-amino)-piperidin-1-yl]-2,N-dimethyl-N-(4-trifluoromethyl-benzyl)-butyramide
2-(3,4-dichloro-phenyl)-4-[4-(ethyl-methanesulfonyl-amino)-piperidin-1-yl]-2,N-dimethyl-N-(4-methyl-benzyl)-butyramide
N-(2-chloro-benzyl)-2-(3,4-dichloro-phenyl)-4-[4-(ethyl-methanesulfonyl-amino)-piperidin-1-yl]-2,N-dimethyl-butyramide or
2-(3,4-dichloro-phenyl)-N-[1-(3,4-dichloro-phenyl)-2-hydroxy-ethyl]-4-[4-(ethyl-methanesulfonyl-amino)-piperidin-1-yl]-2-methyl-butyramide; diastereoisomer 1.

9. A compound of formula I according to claim 2, wherein R¹ and R² form together with the N-atom to which they are attached a pyrrolidin-2-one group.

10. A compound of formula I according to claim 9, which compounds are
2-(3,4-dichloro-phenyl)-N-(3-fluoro-4-trifluoromethyl-benzyl)-2,N-dimethyl-4-[4-(2-oxo-pyrrolidin-1-yl)-piperidin-1-yl]-butyramide
N-(3,4-dichloro-benzyl)-2-(3,4-dichloro-phenyl)-2,N-dimethyl-4-(4-(2-oxo-pyrrolidin-1-yl)-piperidin-1-yl]-butyramide
N-(3,4-dichloro-benzyl)-2-(3,4-dichloro-phenyl)-2-methyl-4-[4-(2-oxo-pyrrolidin-1-yl)-piperidin-1-yl]-butyramide
N-(4-chloro-benzyl)-2-(3,4-dichloro-phenyl)-2,N-dimethyl-4-[4-(2-oxo-pyrrolidin-1-yl)-piperidin-1-yl]-butyramide
N-(4-chloro-3-trifluoromethyl-benzyl)-2-(3,4-dichloro-phenyl)-2,N-dimethyl-4-[4-(2-oxo-pyrrolidin-1-yl)-piperidin-1-yl-butyramide
2-(3,4-dichloro-phenyl)-N-(4-fluoro-3-trifluoromethyl-benzyl)-2,N-dimethyl-4-[4-(2-oxo-pyrrolidin-1-yl)-piperidin-1-yl]-butyramide
N-(3-chloro-benzyl)-2-(3,4-dichloro-phenyl)-2,N-dimethyl-4-[4-(2-oxo-pyrrolidin-1-yl)-piperidin-1-yl]-butyramide
2-(3,4-dichloro-phenyl)-2,N-dimethyl-4-[4-(2-oxo-pyrrolidin-1-yl)-piperidin-1-yl]-N-(4-trifluoromethyl-benzyl)-butyramide
2-(3,4-dichloro-phenyl)-2,N-dimethyl-N-(4-methyl-benzyl)-4-[4-(2-oxo-pyrrolidin-1-yl)-piperidin-1-yl]-butyramide
N-(2-chloro-benzyl)-2-(3,4-dichloro-phenyl)-2,N-dimethyl-4-[4-(2-oxo-pyrrolidin-1-yl)-piperidin-1-yl]-butyramide
2-(3,4-dichloro-phenyl)-N-[-1-(3,4-dichloro-phenyl)-2-hydroxy-ethyl]-2-methyl-4-[4-(2-oxo-pyrrolidin-1-yl)-piperidin-1-yl]-butyramide; diastereoisomer 1
2-(3,4-dichloro-phenyl)-2-methyl-4-[4-(2-oxo-pyrrolidin-1-yl)-piperidin-1-yl] -N-(2,2,2-trifluoro-1-phenyl-ethyl)-butyramide
2-(3,4-dichloro-phenyl)-2-methyl-4-[4-(2-oxo-pyrrolidin-1-yl)-piperidin-1-yl]-N-[2,2,2-trifluoro-1-(4-fluoro-phenyl)-ethyl]-butyramide
N-(4-chloro-benzyl)-2-(3,4-dichloro-phenyl)-N-methyl-4-[4-(2-oxo-pyrrolidin-1-yl)-piperidin-1-yl -butyramide
(R or S)-2-(3,4-dichloro-phenyl)-N-(4-fluoro-3-trifluoromethyl-benzyl)-2,N-dimethyl-4- [4-(2-oxo-pyrrolidin-1-yl)-piperidin-1-yl] -butyramide
(R or S)-2-(3,4-dichloro-phenyl)-N-[(S or R)-1-(3,4-dichloro-phenyl)-2-hydroxy-ethyl]-2-methyl-4-[4-(2-oxo-pyrrolidin-1-yl)-piperidin-1-yl]-butyramide
2-(3,4-dichloro-phenyl)-2-methyl-4-[4-(2-oxo-pyrrolidin-1-yl)-piperidin-1-yl]-*N*-((S)-1-phenyl-propyl)-butyramide
2-(3,4-dichloro-phenyl)-*N*-[(R)-1-(4-fluoro-phenyl)-2-hydroxy-ethyl]-2-(R or S) methyl-4-[4-(2-oxo-pyrrolidin-1-yl)-piperidin-1-yl]-butyramide or
2-(3,4-dichloro-phenyl)-*N-*(4-fluoro-3-trifluoromethyl-benzyl)-2-methyl-4-[4-(2-oxopyrrolidin-1-yl)-piperidin-1-yl) -butyramide.

11. A compound of formula I according to claim 2, wherein R¹ is lower alkyl and R² is C(O)CH₃.

12. A compound of formula I according to claim 11, wherein the compounds are
4-[4-(acetyl-methyl-amino)-piperidin-1-yl]-2-(3,4-dichloro-phenyl)-N-(3-fluoro-4-trifluoromethyl-benzyl)-2-methyl-butyramide
4- [4-(acetyl-methyl-amino)-piperidin-1-yl]-N-(3,4-dichloro-benzyl)-2-(3,4-dichloro-phenyl)-2,N-dimethyl-butyramide
4-[4-(acetyl-methyl-amino)-piperidin-l-yl]-N-(3,4-dichloro-benzyl)-2-(3,4-dichloro-phenyl)-2-methyl-butyramide
4-[4-(acetyl-methyl-amino)-piperidin-1-yl]-N-(4-chloro-benzyl)-2-(3,4-dichloro-phenyl)-2,N-dimethyl-butyramide
4-[4-(acetyl-methyl-amino)-piperidin-1-yl]-N-(4-chloro-3-trifluoromethyl-benzyl)-2-(3,4-dichloro-phenyl)-2,N-dimethyl-butyramide
4- [4-(acetyl-methyl-amino)-piperidin-1-yl]-2-(3,4-dichloro-phenyl)-N-(4-fluoro-3-trifluoromethyl-benzyl)-2,N-dimethyl-butyramide
4-[4-(acetyl-methyl-amino)-piperidin-1-yl]-N-(3-chloro-benzyl)-2-(3,4-dichloro-phenyl)-2,N-dimethyl-butyramide
4-[4-(acetyl-methyl-amino)-piperidin-1-yl]-2-(3,4-dichloro-phenyl)-2,N-dimethyl-N-(4-trifluoromethyl-benzyl)-butyramide
4-[4-(acetyl-methyl-amino)-piperidin-1-yl]-2-(3,4-dichloro-phenyl)-N-[1-(3,4-dichloro-phenyl)-2-hydroxy-ethyl]-2-methyl-butyramide; diastereoisomer 1
(R or S)-4-[4-(acetyl-methyl-amino)-piperidin-1-yl]-2-(3,4-dichloro-phenyl)-N-(4-fluoro-3-trifluoromethyl-benzyl)-2,N-dimethyl-butyramide
4-[4-(acetyl-methyl-amino) -piperidin-1-yl]-*N*-[1-(4-chloro-phenyl)-2-hydroxy-ethyl]-2-(3,4-dichloro-phenyl)-2-methyl-butyramide (diastereosisomer 1)
4-[4-(acetyl-propyl-amino)-piperidin-1-yl]-2-(3,4-dichloro-phenyl)-*N*-[(R)-1-(4-fluorophenyl)-2-hydroxy-ethyl]-2-methyl-butyramide (epimer 1)
4-[4-(acetyl-propyl-amino)-piperidin-1-yl]-2-(3,4-dichloro-phenyl)-*N*-[(R)-1-(4-fluorophenyl)-2-hydroxy-ethyl]-2-methyl-butyramide (epimer 2)
4-[4-(acetyl-propyl-amino)-piperidin-1-yl] -2-(3,4-dichloro-phenyl)-2-methyl-*N*-((S)-1-phenyl-propyl)-butyramide
4-[4-(acetyl-ethyl-amino)-piperidin-1-yl]-2-(3,4-dichloro-phenyl)-*N*-[(R)-1-(4-fluorophenyl)-2-hydroxy-ethyl]-2-methyl-butyramide (epimer 1) or
4-[4-(acetyl-ethyl-amino)-piperidin-1-yl]-2-(3,4-dichloro-phenyl)-2-methyl-*N*-((S)-1-phenyl-propyl)-butyramide.

13. A compound of formula I according to claim 2, wherein R¹ is -(CH₂)₂OCH₃ and R² is C(O)CH₃.

14. A compound of formula I according to claim 13, wherein the compounds are
4-{4-[acetyl-(2-methoxy-ethyl)-amino]-piperidin-1-yl}-2-(3,4-dichloro-phenyl)-*N*-[1-(4-fluoro-phenyl)-2-hydroxy-ethyl]-2-methyl-butyramide (diastereoisomer 1)
4-{4-[acetyl-(2-methoxy-ethyl)-amino]-piperidin-1-yl}-2-(3,4-dichloro-phenyl)-*N*-[1-(3,4-dichloro-phenyl)-2-hydroxy-ethyl]-2-methyl-butyramide (diastreoisomer 1)
4-{4-[acetyl-(2-methoxy-ethyl)-amino]-piperidin-1-yl}-2-(3,4-dichloro-phenyl)-*N*-[(R)-1-(4-fluoro-phenyl)-2-hydroxy-ethyl]-2-(R or S) methyl-butyramide or
4-{4-[acetyl-(2-methoxy-ethyl)-amino]-piperidin-1-yl}-2-(3,4-dichloro-phenyl)-2-methyl-*N*-((S)-1-phenyl-propyl)-butyramide.

15. A process for preparation of the compound according to any one of claims 1-14, which process comprises
a) cleaving off an O-protecting group under aqueous basic conditions from a compound of formula IV 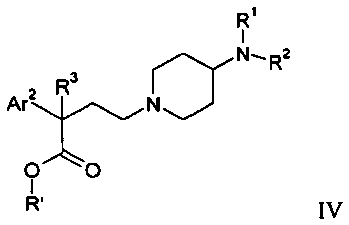
and reacting a compound of formula IV under coupling conditions with an amine of formula 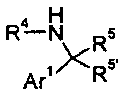 to a compound of formula 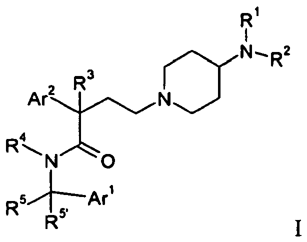 wherein the definitions have same meanings as described in claim 1, and
if desired, converting the compounds obtained into pharmaceutically acceptable acid addition salts.

16. A compound according to claim 1, whenever prepared by a process as claimed in claim 15.

17. A medicament containing one or more compounds as claimed in any one of claims 1-14 and a pharmaceutically acceptable excipient.

18. A medicament according to claim 17 for use in the treatment of depression, pain, psychosis, Parkinson's disease, schizophrenia, anxiety or attention deficit hyperactivity disorder (ADHD).

19. The use of a compound as claimed in any one of claims 1-14 for the manufacture of a medicament for the treatment of depression, pain, psychosis, Parkinson's disease, schizophrenia, anxiety or attention deficit hyperactivity disorder (ADHD).

## Patentansprüche

1. Eine Verbindung der Formel 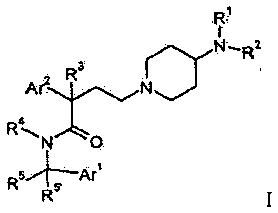 wobei
Ar¹/Ar² unabhängig voneinander Phenyl oder Pyridinyl sind, die gegebenenfalls mit einem oder zwei Substituenten, ausgewählt aus der Gruppe bestehend aus Halogen, Niederalkyl, Niederalkoxy, Niederalkyl, substituiert mit Halogen, Niederalkoxy, substituiert mit Halogen, Niederalkyl, substituiert mit Alkoxy, Niederalkyl, substituiert mit Cyano, Niederdialkylamino, Pyridinyl oder Cyano, substituiert sind;
R¹ Wasserstoff, Niederalkyl, -(CH₂)₂O-Niederalkyl oder Cycloalkyl ist;
R² -S(O)₂-Niederalkyl oder -C(O)-Niederalkyl ist;
oder R¹ und R² zusammen mit dem N-Atom, an das sie gebunden sind, einen Pyrrolidin-2-on- oder einen Piperidin-2-on-Rest bilden;
R³ Wasserstoff, Halogen oder Niederalkyl ist;
R⁴ Wasserstoff oder Niederalkyl ist;
R⁵/R^{5'} unabhängig voneinander Wasserstoff, Niederalkyl, Niederalkyl, substituiert mit Halogen, Niederalkyl, substituiert mit Hydroxy, oder Cycloalkyl sind;
oder R⁵ und R^{5'} zusammen mit dem Kohlenstoffatom, an das sie gebunden sind, einen Cycloalkylrest bilden;
oder ein pharmazeutisch wirksames Salz davon.

2. Eine Verbindung der Formel I nach Anspruch 1, wobei Ar¹ und Ar² beide Phenyl sind.

3. Eine Verbindung der Formel I nach Anspruch 2, wobei R¹ Methyl ist und R² S(O)₂CH₃ ist.

4. Eine Verbindung der Formel I nach Anspruch 3, wobei die Verbindungen sind
2-(3,4-Dichlor-phenyl)-N-(3-fluor-4-trifluormethyl-benzyl)-4-[4-(methansulfonyl-methyl-amino)-piperidin-1-yl]-2,N-dimethyl-butyramid
N-(4-Chlor-3-trifluormethyl-benzyl)-2-(3,4-dichlor-phenyl)-4-[4-(methansulfonyl-methyl-amino)-piperidin-1-yl]-2,N-dimethyl-butyramid
2-(3,4-Dichlor-phenyl)-N-(4-fluor-3-trifluormethyl-benzyl)-4-[4-(methansulfonyl-methyl-amino)-piperidin-1-yl]-2,N-dimethyl-butyramid
N-(3,4-Dichlor-benzyl)-2-(3,4-dichlor-phenyl)-4-[4-(methansulfonyl-methyl-amino)-piperidin-1-yl]-2,N-dimethyl-butyramid oder
2-(3,4-Dichlor-phenyl)-N-[1-(3,4-dichlor-phenyl)-2-hydroxy-ethyl]-4-[4-(methansulfonyl-methyl-amino)-piperidin-1-yl]-2-methyl-butyramid; Diastereoisomer 1.

5. Eine Verbindung der Formel I nach Anspruch 2, wobei R¹ Cyclopropyl ist und R² S(O)₂CH₃ ist.

6. Eine Verbindung der Formel I nach Anspruch 5, wobei die Verbindungen sind
4-[4-(Cyclopropyl-methansulfonyl-amino)-piperidin-1-yl]-2-(3,4-dichlor-phenyl)-N-(3-fluor-4-trifluormethyl-benzyl)-2,N-dimethyl-butyramid
N-(4-Chlor-3-trifluormethyl-benzyl)-4-[4-(cyclopropyl-methansulfonyl-amino)-piperidin-1-yl]-2-(3,4-dichlor-phenyl)-2,N-dimethyl-butyramid
4-[4-(Cyclopropyl-methansulfonyl-amino)-piperidin-1-yl]-2-(3,4-dichlor-phenyl)-N-(4-fluor-3-trifluormethyl-benzyl)-2,N-dimethyl-butyramid oder
4-[4-(Cyclopropyl-methansulfonyl-amino)-piperidin-1-yl]-N-(3,4-dichlor-benzyl)-2-(3,4-dichlor-phenyl)-2,N-dimethyl-butyramid.

7. Eine Verbindung der Formel I nach Anspruch 2, wobei R¹ Ethyl ist und R² S(O)₂CH₃ ist.

8. Eine Verbindung der Formel I nach Anspruch 7, wobei die Verbindungen sind
2-(3,4-Dichlor-phenyl)-4-[4-(ethyl-methansulfonyl-amino)-piperidin-1-yl]-N-(3-fluor-4-trifluormethyl-benzyl)-2,N-dimethyl-butyramid
N-(3,4-Dichlor-benzyl)-2-(3,4-dichlor-phenyl)-4-[4-(ethyl-methansulfonyl-amino)-piperidin-1-yl]-2,N-dimethyl-butyramid
N-(3,4-Dichlor-benzyl)-2-(3,4-dichlor-phenyl)-4-[4-(ethyl-methansulfonyl-amino)-piperidin-1-yl]-2-methyl-butyramid
N-(4-Chlor-benzyl)-2-(3,4-dichlor-phenyl)-4-[4-(ethyl-methansulfonyl-amino)-piperidin-1-yl]-2,N-dimethyl-butyramid
N-(4-Chlor-3-trifluormethyl-benzyl)-2-(3,4-dichlor-phenyl)-4-[4-(ethyl-methansulfonyl-amino)-piperidin-1-yl]-2,N-dimethyl-butyramid
2-(3,4-Dichlor-phenyl)-4-[4-(ethyl-methansulfonyl-amino)-piperidin-1-yl]-N-(4-fluor-3-trifluormethyl-benzyl)-2,N-dimethyl-butyramid
N-(3-Chlor-benzyl)-2-(3,4-dichlor-phenyl)-4-[4-(ethyl-methansulfonyl-amino)-piperidin-1-yl]-2,N-dimethyl-butyramid
2-{3,4-Dichlor-phenyl)-4-[4-(ethyl-methansulfonyl-amino)-piperidin-1-yl]-2,N-dimethyl-N-(4-trifluormethyl-benzyl)-butyramid
2-(3,4-Dichlor-phenyl)-4-[4-(ethyl-methansulfonyl-amino)-piperidin-1-yl]-2,N-dimethyl-N-(4-methyl-benzyl)-butyramid
N-(2-Chlor-benzyl)-2-(3,4-dichlor-phenyl)-4-[4-(ethyl-methansulfonyl-amino)-piperidin-1-yl]-2,N-dimethyl-butyramid oder
2-(3,4-Dichlor-phenyl)-N-[1-(3,4-dichlor-phenyl)-2-hydroxy-ethyl]-4-[4-(ethyl-methansulfonyl-amino)-piperidin-1-yl]-2-methyl-butyramid; Diastereoisomer 1.

9. Eine Verbindung der Formel I nach Anspruch 2, wobei R¹ und R² zusammen mit dem N-Atom, an das sie gebunden sind, einen Pyrrolidin-2-on-Rest bilden.

10. Eine Verbindung der Formel I nach Anspruch 9, wobei die Verbindungen sind
2-(3,4-Dichlor-phenyl)-N-(3-fluor-4-trifluormethyl-benzyl)-2,N-dimethyl-4-[4-(2-oxo-pyrrolidin-1-yl)-piperidin-1-yl]-butyramid
N-(3,4-Dichlor-benzyl)-2-(3,4-dichlor-phenyl)-2,N-dimethyl-4-[4-(2-oxo-pyrrolidin-1-yl)-piperidin-1-yl]-butyramid
N-(3,4-Dichlor-benzyl)-2-(3,4-dichlor-phenyl)-2-methyl-4-[4-(2-oxo-pyrrolidin-1-yl)-piperidin-1-yl]-butyramid
N-(4-Chlor-benzyl)-2-(3,4-dichlor-phenyl)-2,N-dimethyl-4-[4-(2-oxo-pyrrolidin-1-yl)-piperidin-1-yl]-butyramid
N-(4-Chlor-3-trifluormethyl-benzyl)-2-(3,4-dichlor-phenyl)-2,N-dimethyl-4-[4-(2-oxo-pyrrolidin-1-yl)-piperidin-1-yl]-butyramid
2-(3,4-Dichlor-phenyl)-N-(4-fluor-3-trifluormethyl-benzyl)-2,N-dimethyl-4-[4-(2-oxo-pyrrolidin-1-yl)-piperidin-1-yl]-butyramid
N-(3-Chlor-benzyl)-2-(3,4-dichlor-phenyl)-2,N-dimethyl-4-[4-(2-oxo-pyrrolidin-1-yl)-piperidin-1-yl]-butyramid
2-(3,4-Dichlor-phenyl)-2,N-dimethyl-4-[4-(2-oxo-pyrrolidin-1-yl)-piperidin-1-yl]-N-(4-trifluormethyl-benzyl)-butyramid
2-(3,4-Dichlor-phenyl)-2,N-dimethyl-N-(4-methyl-benzyl)-4-[4-(2-oxo-pyrrolidin-1-yl)-piperidin-1-yl]-butyramid
N-(2-Chlor-benzyl)-2-(3,4-dichlor-phenyl)-2,N-dimethyl-4-[4-(2-oxo-pyrrolidin-1-yl)-piperidin-1-yl]-butyramid
2-(3,4-Dichlor-phenyl)-N-[1-(3,4-dichlor-phenyl)-2-hydroxy-ethyl]-2-methyl-4-[4-(2-oxo-pyrrolidin-1-yl)-piperidin-1-yl]-butyramid; Diastereoisomer 1
2-(3,4-Dichlor-phenyl)-2-methyl-4-[4-(2-oxo-pyrrolidin-1-yl)-piperidin-1-yl]-N-(2,2,2-trifluor-1-phenyl-ethyl)-butyramid
2-(3,4-Dichlor-phenyl)-2-methyl-4-[4-(2-oxo-pyrrolidin-1-yl)-piperidin-1-yl]-N-[2,2,2-trifluor-1-(4-fluor-phenyl)-ethyl]-butyramid
N-(4-Chlor-benzyl)-2-(3,4-dichlor-phenyl)-N-methyl-4-[4-(2-oxo-pyrrolidin-1-yl)-piperidin-1-yl]-butyramid
(R oder S)-2-(3,4-Dichlor-phenyl)-N-(4-fluor-3-trifluormethyl-benzyl)-2,N-dimethyl-4-[4-(2-oxo-pyrrolidin-1-yl)-piperidin-1-yl]-butyramid
(R oder S)-2-(3,4-Dichlor-phenyl)-N-[(S oder R)-1-(3,4-dichlor-phenyl)-2-hydroxy-ethyl]-2-methyl-4-[4-(2-oxo-pyrrolidin-1-yl)-piperidin-1-yl]-butyramid
2-(3,4-Dichlor-phenyl)-2-methyl-4-[4-(2-oxo-pyrrolidin-1-yl)-piperidin-1-yl]-N-((S)-1 - phenyl-propyl)-butyramid
2-(3,4-Dichlor-phenyl)-N-[(R)-1-(4-fluor-phenyl)-2-hydroxy-ethyl]-2-(R oder S)-methyl-4-[ 4-(2-oxo-pyrrolidin-1-yl)-piperidin-1-yl]-butyramid oder
2-(3,4-Dichlor-phenyl)-N-(4-fluor-3-trifluormethyl-benzyl)-2-methyl-4-[4-(2-oxopyrrolidin-1-yl)-piperidin-1-yl]-butyramid.

11. Eine Verbindung der Formel I nach Anspruch 2, wobei R¹ Niederalkyl ist und R² C(O)CH₃ ist.

12. Eine Verbindung der Formel I nach Anspruch 11, wobei die Verbindungen sind
4-[4-(Acetyl-methyl-amino)-piperidin-1-yl]-2-(3,4-dichlor-phenyl)-N-(3-fluor-4-trifluormethyl-benzyl)-2-methyl-butyramid
4-[4-(Acetyl-methyl-amino)-piperidin-1-yl]-N-(3,4-dichlor-benzyl)-2-(3,4-dichlorphenyl)-2,N-dimethyl-butyramid
4-[4-(Acetyl-methyl-amino)-piperidin-1-yl]-N-(3,4-dichlor-benzyl)-2-(3,4-dichlorphenyl)-2-methyl-butyramid
4-[4-(Acetyl-methyl-amino)-piperidin-1-yl]-N-(4-chlor-benzyl)-2-(3,4-dichlor-phenyl)-2,N-dimethyl-butyramid
4-[4-(Acetyl-methyl-amino)-piperidin-1-yl]-N-(4-chlor-3-trifluormethyl-benzyl)-2-(3,4-dichlor-phenyl)-2,N-dimethyl-butyramid
4-[4-(Acetyl-methyl-amino)-piperidin-1-yl]-2-(3,4-dichlor-phenyl)-N-(4-fluor-3-trifluormethyl-benzyl)-2,N-dimethyl-butyramid
4-[4-(Acetyl-methyl-amino)-piperidin-1-yl]-N-(3-chlor-benzyl)-2-(3,4-dichlor-phenyl)-2,N-dimethyl-butyramid
4-[4-(Acetyl-methyl-amino)-piperidin-1-yl]-2-(3,4-dichlor-phenyl)-2,N-dimethyl-N-(4-trifluormethyl-benzyl)-butyramid
4-[4-(Acetyl-methyl-amino)-piperidin-1-yl]-2-(3,4-dichlor-phenyl)-N-[1-(3,4-dichlorphenyl)-2-hydroxy-ethyl]-2-methyl-butyramid; Diastereoisomer 1
(R oder S)-4-[4-(Acetyl-methyl-amino)-piperidin-1-yl]-2-{3,4-dichlor-phenyl)-N-(4-fluor-3-trifluormethyl-benzyl)-2,N-dimethyl-butyramid
4-[4-(Acetyl-methyl-amino)-piperidin-1-yl]-N-[1-(4-chlor-phenyl)-2-hydroxy-ethyl]-2-(3,4-dichlor-phenyl)-2-methyl-butyramid (Diastereoisomer 1)
4-[4-(Acetyl-propyl-amino)-piperidin-1-yl]-2-(3,4-dichlor-phenyl)-N-[(R)-1-(4-fluorphenyl)-2-hydroxy-ethyl]-2-methyl-butyramid (Epimer 1)
4-[4-(Acetyl-propyl-amino)-piperidin-1-yl]-2-{3,4-dichlor-phenyl)-N-[(R)-1-(4-fluorphenyl)-2-hydroxy-ethyl]-2-methyl-butyramid (Epimer 2)
4-[4-(Acetyl-propyl-amino)-piperidin-1-yl]-2-(3,4-dichlor-phenyl)-2-methyl-N-((S)-1 - phenyl-propyl)-butyramid
4-[4-(Acetyl-ethyl-amino)-piperidin-1-yl]-2-(3,4-dichlor-phenyl)-N-[(R)-1-(4-fluorphenyl)-2-hydroxy-ethyl]-2-methyl-butyramid (Epimer 1) oder
4-[4-(Acetyl-ethyl-amino)-piperidin-1-yl]-2-(3,4-dichlor-phenyl)-2-methyl-N-((S )-1-phenyl-propyl)-butyramid.

13. Eine Verbindung der Formel I nach Anspruch 2, wobei R¹ -(CH₂)₂OCH₃ ist und R² C(O)CH₃ ist.

14. Eine Verbindung der Formel I nach Anspruch 13, wobei die Verbindungen sind
4- {4-[Acetyl-(2-methoxy-ethyl)-amino]-piperidin-1-yl}-2-(3,4-dichlor-phenyl)-N-[1-(4-fluor-phenyl)-2-hydroxy-ethyl]-2-methyl-butyramid (Diastereoisomer 1 )
4-{4-[Acetyl-(2-methoxy-ethyl)-amino]-piperidin-1-yl}-2-(3,4-dichlor-phenyl)-N-[1-(3,4-dichlor-phenyl)-2-hydroxy-ethyl]-2-methyl-butyramid (Diastereoisomer 1)
4-{4-[Acetyl-(2-methoxy-ethyl)-amino]-piperidin-1-yl}-2-(3,4-dichlor-phenyl)-N-[(R)-1-(4-fluor-phenyl)-2-hydroxy-ethyl]-2-(R oder S)-methyl-butyramid oder
4-{4-[Acetyl-(2-methoxy-ethyl)-amino]-piperidin-1-yl}-2-(3,4-dichlor-phenyl)-2-methyl-N-((S)-1-phenyl-propyl)-butyramid.

15. Ein Verfahren zur Herstellung der Verbindung nach einem der Ansprüche 1 bis 14, wobei das Verfahren umfasst
a) Abspalten einer O-Schutzgruppe unter wässrigen basischen Bedingungen von einer Verbindung der Formel IV 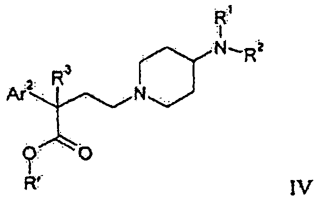
und Umsetzen einer Verbindung der Formel IV unter Kupplungsbedingungen mit einem Amin der Formel 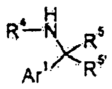 zu einer Verbindung der Formel 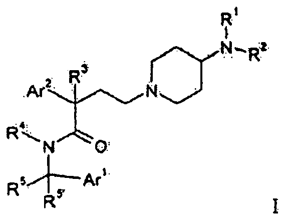 wobei die Definitionen die gleichen Bedeutungen wie in Anspruch 1 beschrieben haben, und
falls gewünscht, Umwandeln der erhaltenen Verbindungen in pharmazeutisch verträgliche Säureadditionssalze.

16. Eine Verbindung nach Anspruch 1, hergestellt durch ein Verfahren wie in Anspruch 15 beansprucht.

17. Ein Medikament, enthaltend eine oder mehrere Verbindungen, wie in einem der Ansprüche 1 bis 14 beansprucht, und einen pharmazeutisch verträglichen Exzipienten.

18. Ein Medikament nach Anspruch 17 zur Verwendung bei der Behandlung von Depression, Schmerz, Psychose, Parkinson-Krankheit, Schizophrenie, Angst oder Aufmerksamkeitsdefizits-Hyperaktivitätssyndrom (ADHS).

19. Die Verwendung einer Verbindung, wie in einem der Ansprüche 1 bis 14 beansprucht, zur Herstellung eines Medikaments zur Behandlung von Depression, Schmerz, Psychose, Parkinson-Krankheit, Schizophrenie, Angst oder Aufmerksamkeitsdefizits-Hyperaktivitätssyndrom (ADHS).

## Revendications

1. Composé de formule 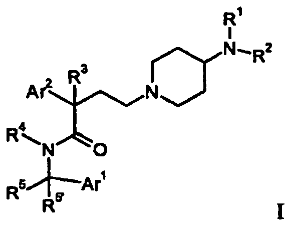 dans laquelle
Ar¹/Ar² sont indépendamment l'un de l'autre un phényle ou un pyridinyle, qui sont éventuellement substitués par un ou deux substituants choisis dans le groupe constitué par halogène, alkyle inférieur, alcoxy inférieur, alkyle inférieur substitué par halogène, alcoxy inférieur substitué par halogène, alkyle inférieur substitué par alcoxy, alkyle inférieur substitué par cyano, di(alkyl inférieur)amino, pyridinyle ou cyano;
R¹ est hydrogène, alkyle inférieur, -(CH₂)₂O-alkyle inférieur, ou cycloalkyle;
R² est -S(O)₂-alkyle inférieur ou -C(O)alkyle inférieur;
ou R¹ et R² forment ensemble, avec l'atome N auquel ils sont liés, un groupe pyrrolidin-2-one ou pipéridin-2-one;
R³ est hydrogène, halogène ou alkyle inférieur;
R⁴ est hydrogène ou alkyle inférieur;
R⁵/R^{5'} sont indépendamment l'un de l'autre hydrogène, alkyle inférieur, alkyle inférieur substitué par halogène, alkyle inférieur substitué par hydroxy ou cycloalkyle;
ou R⁵ et R^{5'} forment ensemble, avec l'atome de carbone auquel ils sont liés, un groupe cycloalkyle;
ou un de leurs sels pharmaceutiquement acceptables.

2. Composé de formule 1 selon la revendication 1, dans lequel Ar¹ et Ar² sont tous les deux un phényle.

3. Composé de formule 1 selon la revendication 2, dans lequel R¹ est un méthyle et R² est S(O)₂CH₃.

4. Composé de formule I selon la revendication 3, ce composé étant:
le 2-(3,4-dichlorophényl)-N-(3-fluoro-4-trifluorométhylbenzyl)-4-[4-(méthane-sulfonyl-méthylamino)pipéridin-1-yl]-2,N-diméthylbutyramide;
le N-(4-chloro-3-trifluorométhylbenzyl)-2-(3,4-dichlorophényl)-4-[4-(méthanesul-fonyl-méthylamino)pipéridin-1-yl]-2,N-diméthylbutyramide;
le 2-(3,4-dichlorophényl)-N-(4-fluoro-3-trifluorométhylbenzyl)-4-[4-(méthanesul-fonyl-méthylamino)pipéridin-1-yl]-2,N-diméthylbutyramide;
le N-(3,4-dichlorobenzyl)-2-(3,4-dichlorophényl)-4-[4-(méthanesulfonyl-méthyl-amino)pipéridin-1-yl]-2,N-diméthylbutyramide ou
le 2-(3,4-dichlorophényl)-N-[1-(3,4-dichlorophényl)-2-hydroxyéthyl]-4-[4-(méthane-sulfonyl-méthylamino)pipéridin-1-yl]-2-méthylbutyramide; diastéréoisomère 1.

5. Composé de formule I selon la revendication 2, dans lequel R¹ est un cyclopropyle et R² est S(O)₂CH₃.

6. Composé de formule I selon la revendication 5, ce composé étant:
le 4-[4-(cyclopropyl-méthanesulfonylamino)pipéridin-1-yl]-2-(3,4-dichlorophényl)-N-(3-fluoro-4-trifluorométhylbenzyl)-2,N-diméthylbutyramide;
le N-(4-chloro-3-trifluorométhylbenzyl)-4-[4-(cyclopropyl-méthanesulfonylamino)-pipéridin-1-yl]-2-(3,4-dichlorophényl)-2,N-diméthylbutyramide;
le 4-[4-(cyclopropyl-méthanesulfonylamino)pipéridin-1-yl]-2-(3,4-dichlorophényl)-N-(4-fluoro-3-trifluorométhylbenzyl)-2,N-diméthylbutyramide ou
le 4-[4-(cyclopropyl-méthanesulfonylamino)pipéridin-1-yl]-N-(3,4-dichlorobenzyl)-2-(3,4-dichlorophényl)-2,N-diméthylbutyramide.

7. Composé de formule I selon la revendication 2, dans lequel R¹ est un éthyle et R² est S(O)₂CH₃.

8. Composé de formule I selon la revendication 7, ce composé étant
le 2-(3,4-dichlorophényl)-4-[4-(éthyl-méthanesulfonylamino)pipéridin-1-yl]-N-(3-fluoro-4-trifluorométhylbenzyl)-2,N-diméthylbutyramide;
le N-(3,4-dichlorobenzyl)-2-(3,4-dichlorophényl)-4-[4-(éthyl-méthanesulfonyl-amino)pipéridin-1 -yl]-2,N-diméthylbutyramide;
le N-(3,4-dichlorobenzyl)-2-(3,4-dichlorophényl)-4-[4-(éthyl-méthanesulfonyl-amino)pipéridin-1-yl]-2-méthylbutyramide;
le N-(4-chlorobenzyl)-2-(3,4-dichlorophényl)-4-[4-(éthyl-méthanesulfonylamino)-pipéridin-1-yl]-2,N-diméthylbutyramide;
le N-(4-chloro-3-trifluorométhylbenzyl)-2-(3,4-dichlorophényl)-4-[4-(éthyl-méthanesulfonylamino)pipéridin-1-yl]-2,N-diméthylbutyramide;
le 2-(3,4-dichlorophényl)-4-[4-(éthyl-méthanesulfonylamino)pipéridin-1-yl]-N-(4-fluoro-3-trifluorométhylbenzyl)-2,N-diméthylbutyramide;
le N-(3-chlorobenzyl)-2-(3,4-dichlorophényl)-4-[4-(éthyl-méthanesulfonylamino)-pipéridin-1-yl]-2,N-diméthylbutyramide;
le 2-(3,4-dichlorophényl)-4-[4-(éthyl-méthanesulfonylamino)pipéridin-1-yl]-2,N-diméthyl-N-(4-trifluorométhylbenzyl)butyramide;
le 2-(3,4-dichlorophényl)-4-[4-(éthyl-méthanesulfonylamino)pipéridin-1-yl]-2,N-diméthyl-N-(4-méthylbenzyl)butyramide;
le N-(2-chlorobenzyl)-2-(3,4-dichlorophényl)-4-[4-(éthyl-méthanesulfonylamino)-pipéridin-1-yl]-2,N-diméthylbutyramide ou
le 2-(3,4-dichlorophényl)-N-[1-(3,4-dichlorophényl)-2-hydroxyéthyl]-4-[4-(éthyl-méthanesulfonylamino)pipéridin-1-yl]-2-méthylbutyramide; diastéréoisomère 1.

9. Composé de formule I selon la revendication 2, dans lequel R¹ et R² forment ensemble, avec l'atome N auquel ils sont liés, un groupe pyrrolidin-2-one.

10. Composé de formule I selon la revendication 9, ce composé étant:
le 2-(3,4-dichlorophényl)-N-(3-fluoro-4-trifluorométhylbenzyl)-2,N-diméthyl-4-[4-(2-oxopyrrolidin-1-yl)pipéridin-1-yl]butyramide;
le N-(3,4-dichlorobenzyl)-2-(3,4-dichlorophényl)-2,N-diméthyl-4-[4-(2-oxopyrrolidin-1-yl)pipéridin-1-yl]butyramide;
le N-(3,4-dichlorobenzyl)-2-(3,4-dichlorophényl)-2-méthyl-4-[4-(2-oxopyrrolidin-1-yl)pipéridin-1-yl]butyramide;
le N-(4-chlorobenzyl)-2-(3,4-dichlorophényl)-2,N-diméthyl-4-[4-(2-oxopyrrolidin-1-yl)pipéridin-1-yl]butyramide;
le N-(4-chloro-3-trifluorométhylbenzyl)-2-(3,4-dichlorophényl)-2,N-diméthyl-4-[4-(2-oxopyrrolidin-1-yl)pipéridin-1-yl]butyramide;
le 2-(3,4-dichlorophényl)-N-(4-fluoro-3-trifluorométhylbenzyl)-2,N-diméthyl-4-[4-(2-oxopyrrolidin-1-yl)pipéridin-1-yl]butyramide;
le N-(3-chlorobenzyl)-2-(3,4-dichlorophényl)-2,N-diméthyl-4-[4-(2-oxopyrrolidin-1-yl)pipéridin-1-yl]butyramide;
le 2-(3,4-dichlorophényl)-2,N-diméthyl-4-[4-(2-oxopyrrolidin-1-yl)pipéridin-1-yl]-N-(4-trifluorométhylbenzyl)butyramide;
le 2-(3,4-dichlorophényl)-2,N-diméthyl-N-(4-méthylbenzyl)-4-[4-(2-oxopyrrolidin-1-yl)pipéridin-1-yl]butyramide;
le N-(2-chlorobenzyl)-2-(3,4-dichlorophényl)-2,N-diméthyl-4-[4-(2-oxopyrrolidin-1-yl)pipéridin-1-yl]butyramide;
le 2-(3,4-dichlorophényl)-N-[1-(3,4-dichlorophényl)-2-hydroxyéthyl]-2-méthyl-4-[4-(2-oxopyrrolidin-1-yl)pipéridin-1-yl]butyramide; diastéréoisomère 1;
le 2-(3,4-dichlorophényl)-2-méthyl-4-[4-(2-oxopyrrolidin-1-yl)pipéridin-1-yl]-N-(2,2,2-trifluoro-1-phényléthyl)butyramide;
le 2-(3,4-dichlorophényl)-2-méthyl-4-[4-(2-oxopyrrolidin-1-yl)pipéridin-1-yl]-N-[2,2,2-trifluoro-1-(4-fluorophényl)-éthyl]butyramide;
le N-(4-chlorobenzyl)-2-(3,4-dichlorophényl)-N-méthyl-4-[4-(2-oxopyrrolidin-1-yl)pipéridin-1-yl]butyramide;
le (R ou S)-2-(3,4-dichlorophényl)-N-(4-fluoro-3-trifluorométhylbenzyl)-2,N-diméthyl-4-[4-(2-oxopyrrolidin-1 -yl)pipéridin-1 -yl]butyramide;
le (R ou S)-2-(3,4-dichlorophényl)-N-[(S ou R)-1-(3,4-dichlorophényl)-2-hydroxyéthyl]-2-méthyl-4-[4-(2-oxopyrrolidin-1-yl)pipéridin-1-yl]butyramide;
le 2-(3,4-dichlorophényl)-2-méthyl-4-[4-(2-oxopyrrolidin-1-yl)pipéridin-1-yl]-N-((S)-1-phénylpropyl)butyramide;
le 2-(3,4-dichlorophényl)-N-[(R)-1-(4-fluorophényl)-2-hydroxyéthyl]-2-(R ou S)-méthyl-4-[4-(2-oxopyrrolidin-1-yl)pipéridin-1-yl]butyramide ou
le 2-(3,4-dichlorophényl)-N-(4-fluoro-3-trifluorométhylbenzyl)-2-méthyl-4-[4-(2-oxopyrrolidin-1-yl)pipéridin-1-yl]butyramide.

11. Composé de formule I selon la revendication 2, dans lequel R¹ est un alkyle inférieur et R² est C(O)CH₃.

12. Composé de formule I selon la revendication 11, le composé étant:
le 4-[4-(acétyl-méthylamino)pipéridin-1-yl]-2-(3,4-dichlorophényl)-N-(3-fluoro-4-trifluorométhylbenzyl)-2-méthylbutyramide;
le 4-[4-(acétyl-méthylamino)pipéridin-1-yl]-N-(3,4-dichlorobenzyl)-2-(3,4-dichlorophényl)-2,N-diméthylbutyramide;
le 4-[4-(acétyl-méthylamino)pipéridin-1-yl]-N-(3,4-dichlorobenzyl)-2-(3,4-dichlorophényl)-2-méthylbutyramide;
le 4-[4-(acétyl-méthylamino)pipéridin-1-yl]-N-(4-chlorobenzyl)-2-(3,4-dichlorophényl)-2,N-diméthylbutyramide;
le 4- [4-(acétyl-méthylamino)pipéridin- 1-yl] -N-(4-chloro-3 -trifluorométhylbenzyl)-2-(3,4-dichlorophényl)-2,N-diméthylbutyramide;
le 4-[4-(acétyl-méthylamino)pipéridin-1-yl]-2-(3,4-dichlorophényl)-N-(4-fluoro-3-trifluorométhylbenzyl)-2,N-diméthylbutyramide;
le 4-[4-(acétyl-méthylamino)pipéridin-1-yl]-N-(3-chlorobenzyl)-2-(3,4-dichlorophényl)-2,N-diméthylbutyramide;
le 4-[4-(acétyl-méthylamino)pipéridin-1-yl]-2-(3,4-dichlorophényl)-2,N-diméthyl-N-(4-trifluorométhylbenzyl)butyramide;
le 4-[4-(acétyl-méthylamino)pipéridin- 1 -yl]-2-(3,4-dichlorophényl)-N-[ 1 -(3,4-dichlorophényl)-2-hydroxyéthyl]-2-méthylbutyramide; diastéréoisomère 1;
le (R ou S)-4-[4-(acétyl-méthylamino)pipéridin-1-yl]-2-(3,4-dichlorophényl)-N-(4-fluoro-3-trifluorométhylbenzyl)-2,N-diméthylbutyramide;
le 4-[4-(acétyl-méthylamino)pipéridin-1-yl]-N-[1-(4-chlorophényl)-2-hydroxyéthyl]-2-(3,4-dichlorophényl)-2-méthylbutyramide (diastéréoisomère 1);
le 4-[4-(acétyl-propylamino)pipéridin-1-yl]-2-(3,4-dichlorophényl)-N-[(R)-1-(4-fluorophényl)-2-hydroxyéthyl]-2-méthylbutyramide (épimère 1);
le 4-[4-(acétyl-propylamino)pipéridin-1-yl]-2-(3,4-dichlorophényl)-N-[(R)-1-(4-fluorophényl)-2-hydroxyéthyl]-2-méthylbutyramide (épimère 2);
le 4-[4-(acétyl-propylamino)pipéridin-1-yl]-2-(3,4-dichlorophényl)-2-méthyl-N-((S)-1-phénylpropyl)butyramide;
le 4-[4-(acétyl-éthylamino)pipéridin-1-yl]-2-(3,4-dichlorophényl)-N-[(R)-1-(4-fluorophényl)-2-hydroxyéthyl]-2-méthylbutyramide (épimère 1) ou
le 4-[4-(acétyl-éthylamino)pipéridin-1-yl]-2-(3,4-dichlorophényl)-2-méthyl-N-((S)-1-phénylpropyl)butyramide.

13. Composé de formule I selon la revendication 2, dans lequel R¹ est
- (CH₂)₂OCH₃ et R² est C(O)CH₃.

14. Composé de formule I selon la revendication 13, le composé étant:
le 4- (4-[acétyl-(2-méthoxyéthyl)amino]pipéridin- 1 -yl) -2-(3,4-dichlorophényl)-N-[ 1 - (4-fluorophényl)-2-hydroxyéthyl]-2-méthylbutyramide (diastéréoisomère 1);
le 4-{4-[acétyl-(2-méthoxyéthyl)amino]pipéridin-1-yl}-2-(3,4-dichlorophényl)-N-[1-(3,4-dichlorophényl)-2-hydroxyéthyl]-2-méthylbutyramide (diastéréoisomère 1);
le 4-{4-[acétyl-(2-méthoxyéthyl)amino]pipéridin-1-yl}-2-(3,4-dichlorophényl)-N-[(R)-1-(4-fluorophényl)-2-hydroxyéthyl]-2-(R ou S)-méthylbutyramide ou
le 4-{4-[acétyl-(2-méthoxyéthyl)amino]pipéridin-1-yl}-2-(3,4-dichlorophényl)-2-méthyl-N-((S)-1-phénylpropyl)butyramide.

15. Procédé de préparation du composé selon l'une quelconque des revendications 1-14, ce procédé comprenant
a) la séparation d'un groupe protecteur de O dans des conditions basiques aqueuses d'un composé de formule IV 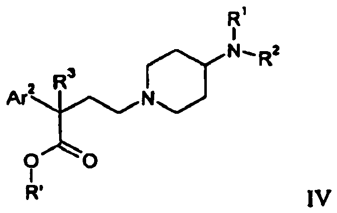
et la réaction d'un composé de formule IV dans des conditions de couplage avec une amine de formule 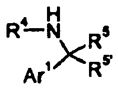 pour l'obtention d'un composé de formule 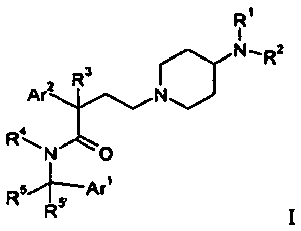 où les définitions ont la même signification que dans la revendication 1, et
si désiré, la conversion des composés obtenus en sels d'addition d'acides pharmaceutiquement acceptables.

16. Composé selon la revendication 1, préparé par un procédé selon la revendication 15.

17. Médicament contenant un ou plusieurs composés selon l'une quelconque des revendications 1-14 et un excipient pharmaceutiquement acceptable.

18. Médicament selon la revendication 17, pour utilisation dans le traitement de la dépression, de la douleur, de la psychose, de la maladie de Parkinson, de la schizophrénie, de l'anxiété ou du trouble d'hyperactivité avec déficit de l'attention (THDA).

19. Utilisation d'un composé selon l'une quelconque des revendications 1-14 pour la fabrication d'un médicament destiné au traitement de la dépression, de la douleur, de la psychose, de la maladie de Parkinson, de la schizophrénie, de l'anxiété ou du trouble d'hyperactivité avec déficit de l'attention (THDA).
